# EUROPEAN PATENT APPLICATION

(11) **EP 1 052 286 A2**
(43) Date of publication of application: **15.11.2000**
(21) Application number: 00302790.1
(22) Date of filing: 12.04.2000
(51) Int. Cl.: C12N 15/16, C12N 15/62, C12N 15/85, C12N 5/10, C07K 14/60, C07K 14/61, A61K 38/25, A61K 38/27, A61K 48/00

(54) **Growth hormone and growth hormone releasing hormone compositions**

(30) Priority: 12.04.1999 US 128830 P
(71) Applicant: Pfizer Products Inc., Groton, Connecticut 06340 (US)
(72) Inventor: Morsey, Mohamad Ali, Groton, Connecticut 06340 (US); Sheppard, Michael George, Groton, Connecticut 06340 (US)
(74) Representative: Simpson, Alison Elizabeth Fraser

(57) **Abstract**

The present invention relates to methods and compositions of growth hormone and/or growth hormone releasing hormone that promote of the release and the elevation of growth hormone when administered to animals. The present invention further relates to methods and compositions of growth hormone and/or growth hormone releasing hormone for treatment of diseases or disorders resulting from growth hormone related deficiencies. The invention also provides methods for producing novel growth hormone releasing hormone variants and their uses thereof.

## Description

### Introduction

The present invention relates to novel variants of growth hormone releasing hormone (GHRH) that have enhanced resistance to enzymatic degradation and polynucleotides encoding said GHRH variants. The present invention relates to methods and therapeutic compositions for the treatment of growth hormone related deficiencies comprising administrating to humans, companion animals, livestock or poultry, plasmid compositions comprising polynucleotides encoding GHRH or variants thereof, alone or in combination with polynucleotides encoding growth hormone or modified growth hormone. The present invention further relates to methods and compositions that promote the release and expression of growth hormone in order to enhance the growth and performance of companion animals, livestock or poultry comprising the administration plasmid compositions encoding GHRH variants, GHRH or modified GHRH, and/or GH or modified GH.

### Background of the Invention

Growth hormone-releasing hormone ("GHRH") is a peptide hormone secreted from the hypothalamus. Following secretion, GHRH enters the portal circulation connecting the hypothalamus to pituitary gland. GHRH then interacts with its receptors on the pituitary gland and induces the release of growth hormone ("GH"). GH secreted from the pituitary gland enters the general circulation and from there it reaches various organs and tissues of the body where it interacts with specific receptors and induces a wide range of developmental effects.

GHRH peptides have been isolated and characterized from several species including humans, porcine, ovine and bovine. In each of these species, GHRH is a small polypeptide consisting of 44 amino acids (GHRH(1-44)-NH₂). However, it has been also shown that smaller fragments, most notably those consisting of the first (amino terminal) 29 amino acids (referred to as GHRH1-29 fragment) retain the same intrinsic biological activity as the full length parent molecule.

GHRH is synthesized as a precursor polypeptide consisting of 107 or 108 amino acids depending on the species. Following synthesis, the precursor GHRH polypeptide undergoes sequential processing. First, the 31 amino acid signal peptide (Met⁻³⁰ to Arg⁰) of the GHRH precursor polypeptide is cleaved (Smith et al., 1992, *Biotechnology* 10:315-319). Subsequently, the GHRH precursor polypeptide is cleaved at position 46-47 and at position 45-46 by a trypsin-like endopeptidase and a carboxypeptidase, respectively, resulting in generation of GHRH(1-45)-OH and a 30 amino acid peptide (amino acids 77-107) designated GCTP (Brar, A.K. et al., 1991, Endocrinology 129: 3274-3280). The GHRH(1-45)-OH polypeptide is further processed by peptidyl glycine α-amidating monooxygenase ("PAM"), which transfers an amide group from Gly⁴⁵ to Leu⁴⁴ and results in the formation of GHRH(1-44)-NH₂, the full length form of GHRH (Brar, A.K. et al., 1991, Endocrinology 129: 3274-3280). The GCTP is also undergoes processing by PAM, which results in the transfer of an amide group from Gly⁷⁷ to Gln⁷⁶. Although the role of GHRH(1-44)-NH₂ in inducing the release of GH is well established, the role of the GCTP peptide is less clear. One report has implicated the GCTP peptide in the control of feeding behavior (Arase, K. et al., 1987, *Endocrinology* 121:1960-1965).

GH has been identified and its gene cloned from many species including human, porcine, bovine, and equine. Unlike GHRH, there exists natural variants of GH within a given species. For example, bovine GH is released from the pituitary gland in one of four variants which differ from one another by one or more amino acids and some studies suggest that these variants differ in their potency (*e.g*., in terms of their ability to increase milk yield). Several studies have also identified amino acid substitutions that lead to an increase in the affinity of GH to its receptors and/or enhanced stability to enzymatic degradation. Studies have also shown that immunization against specific peptides from GH (*e.g*., a peptide consisting of amino acids 35 to 53 of GH) leads to production of antibodies that bind growth hormone and increase the efficacy of GH treatment, presumably because the antibodies delay the clearance of GH from circulation, thus, increasing half-life of GH, and/or protect GH from proteolytic degradation (Bomford, F. and Aston, P., 1990, *Endocrinology* 125:31-38).

Significant research efforts have focused on the structural attributes of GH and GHRH, as well as their biological and developmental activities. A number of groups have attempted to exploit GH and GHRH in a manner that could provide important therapeutic and economic benefits as a result of their use in humans and animals. For example, the traditional treatment of GH-deficient children has been the administration of growth hormone isolated from human pituitary glands, however these preparations are no longer available in the United States due to virus-contaminated samples (Vance, 1990, *Clin. Chem* 36/3: 415-420). Recombinantly expressed and purified GH have been shown to have some benefits in treating GH-deficient children, however the combination of recombinantly expressed GH and GHRH in the treatment of GH-deficient children has provided conflicting results. (Vance, *supra*). Further, purified GH and GHRH must be administered at very high quantities to be effective as the exposure of these polypeptides to serum results in their rapid degradation to a polypeptide which exhibits considerably different biological and pharmacokinetic properties. (Fronman et al., 1989, *J. Clin. Invest.* 83:1533-1540).

Other studies have shown that GH or GHRH administered as purified polypeptides have significant impact on animal growth (muscle and bone growth), average daily gain, milk production, feed efficiency (the ratio of feed consumed to body weight gain), adipose tissue accretion and others. For example, it has been shown that daily administration of maximally effective doses of GH administered to growing pigs for 33-77 days can increase average daily gain -10-20%, improves feed efficiency 13-33%, decrease adipose tissue accretion by as much as 70%, and stimulates protein deposition (muscle growth) by as much as 62%. (Etherton et al.,1998, *Physiological Reviews* 78:745-761). Furthermore, when GH was administered to dairy cows, milk yields were increased by 10-15% (□4-6kg/day) (Etherton et al.,1998, *Physiological Reviews* 78:745-761).

A major impediment to fulfilling the therapeutic and economic potential of GHRH peptides is their susceptibility to cleavage (and subsequent conversion to inactive forms) by specific tissue and plasma proteolytic enzymes; most notably dipeptidylpeplidase IV ("DPPIV"). A number of researchers have focused on manipulating GHRH in order to develop compounds with significant therapeutic potential. Consequently, a wide variety of synthetic GHRH peptide analogues have been produced. They consist of GHRH polypeptides in which one or more amino acids have been chemically modified or replaced with other L- or D- amino acids. These modifications or substitutions are designed to yield analogues with biological properties superior to those of the parent molecule in terms of potency, stability and resistance to chemical and enzymatic degradation. However, these chemically modified polypeptides are not easily or efficiently produced in a suitable form to be administered to humans or animals.

In spite of the significant therapeutic and economic benefits of GH or GHRH alluded to above, exogenous supplementation of animals with GH or GHRH proteins have not been widely adopted as a component of routine management practices to enhance the quality of meat from animals and/or enhance the productivity of livestock. This is because in order to get these benefits, animals have to be repeatedly administered GH or GHRH polypeptides (often daily, but typically in a slow release formulation given every 7-10 days). (Etherton, T.D., 1997, *Nature Biotechnology* 15:1248) This situation is labor intensive, time consuming, expensive, and does not fit current management practices where animals are reared in large numbers and are handled very infrequently, it is apparent therefore that in order to realize the therapeutic and economic benefits of GH and/or GHRH administration, much improved formulations for delivery of these hormones must be developed to overcome the current limitations of their use; namely the need for repeated administration.

### Summary of the Invention

The present invention relates to novel variants of GHRH that have enhanced resistance to enzymatic degradation and polynucleotides encoding said variants. The present invention also relates to pharmaceutical formulations comprising polynucleotide sequences encoding GHRH variants alone or in combination with polynucleotide sequences encoding GHRH, modified GHRH, GH and/or modified GH. The present invention also relates to pharmaceutical formulations comprising GHRH variant peptides alone or in combination with GHRH polypeptides, modified GHRH polypeptides, GH polypeptides and/or modified GH polypeptides. The present invention further relates to pharmaceutical formulations comprising canine or feline GHRH peptides alone or in combination with GHRH variant polypeptides, modified GHRH polypeptides, GH polypeptides and/or modified GH polypeptides.

The present invention relates to therapeutic methods and compositions for the treatment of growth hormone related deficiencies comprising growth hormone ("GH") and/or growth hormone-releasing hormone ("GHRH") in human, companion animals, livestock and poultry. The invention also relates to methods for the improvement in the health of humans, companion animals, livestock and poultry. The invention also relates to methods for the treatment of obesity and frailty of companion animals. The invention further relates to methods for the enhancement of the growth and performance of companion livestock and poultry. The methods of the present invention comprise pharmaceutical compositions which enhance the expression of growth hormone or promote the release of growth hormone or both when administered to humans, companion animals, livestock or poultry. According to the present invention, the term "GHRH" relates to the full length wildtype form of GHRH which is 44 amino acids (aa) or a precursor form of GHRH. In accordance with the present invention, the term "modified GHRH" refers to any amino terminal polypeptide fragment of GHRH from 29 amino acids to 107 or 108 amino acids in length and any mutant of GHRH, including additions, deletions or substitutions at the nucleotide or amino acid level, which retains at least the level of activity of wildtype GHRH, that is, the ability to induce GH gene transcription at levels comparable to wildtype GHRH.

In accordance with the present invention, the term "GHRH variant" relates to a GHRH polypeptide to which one or more amino acids have been attached to the carboxy or amino terminus of the polypeptide, or a wildtype GHRH polypeptide that contains a substitution of one or more amino acids, so that the GHRH variant retains at least equal or enhanced wildtype GHRH activity and has enhanced resistance to enzymatic degradation relative to the wildtype GHRH. In accordance with the present invention, wildtype GHRH activity is measured by its ability to induce GH gene transcription. In accordance with the present invention, resistance to enzymatic degradation is determined by the ability of the polypeptide to resist degradation caused by dipeptidylpeptidase type IV.

According to the present invention, the term "GH" refers to the full length wildtype form of GH, which is 191 amino acids, and "modified GH" refers to any fragment of GH and any mutant including additions, deletions or substitutions at the nucleotide or amino acid level, which retains at least the level of wildtype activity of GH, that is, the ability to induce insulin growth factor (IGF) gene transcription at levels comparable to wildtype GH, or mimic the anti-adipogenic and lipolytic effects of GH.

### Description of the Figures

Figure 1 is a map of the pGHRH-4 construct (SEQ ID No. 47).
Figure 2 is a map of the pGHRH1-44SK construct (SEQ ID No. 48).
Figure 3 is a map of the pGHRH1-44WTSK685 construct (SEQ ID No. 49).
Figure 4 is a map of the pGHRH1-44WTSK2014 construct (SEQ ID No. 50).
Figure 5 is a graph depicting the GHRH expression levels detected in supernatants from pGHRH-4 transfected cells.

### Detailed description of the invention

The present invention relates to novel variants of GHRH that have enhanced resistance to enzymatic degradation and polynucleotides encoding said GHRH variants. The present invention relates to pharmaceutical compositions which promote the release and/or expression of GH. In particular, the pharmaceutical compositions of the present invention comprise polynucleotide sequences encoding GHRH variants alone or in combination with polynucleotide sequences encoding GHRH, modified GHRH, GH and/or modified GH or any combination thereof. In another embodiment, the pharmaceutical compositions of the present invention comprise GHRH variant polypeptides alone or in combination with GHRH polypeptides, GHRH modified polypeptides, GH polypeptides or GH modified polypeptides or any combination thereof.

The present invention relates to methods of treating disorders related to GH related deficiencies in humans, companion animals, livestock and poultry, comprising administering pharmaceutical formulations which enhance GH expression and/or release. The present invention further relates to methods of treating livestock and poultry in order to enhance growth and performance comprising administering pharmaceutical formulations which enhance GH expression and/or release.

The pharmaceutical formulations to be administered in accordance with the methods of the present invention encompass plasmid compositions comprising (a) polynucleotide sequences encoding GHRH variants; (b) polynucleotide sequences encoding GHRH or modified GHRH; (c) polynucleotide sequences encoding GH or modified GH; or any combination thereof, wherein the polynucleotide sequences are operably linked to a promoter or regulatory element, preferably one that is transcriptionally active in muscle tissue. The pharmaceutical formulations to be administered in accordance with the methods of the present invention also include: i) plasmid compositions comprising polynucleotides encoding for GH or modified or variant GHRH gene; ii) plasmid compositions comprising polynucleotides encoding for both GH and GHRH genes; iii) plasmid compositions comprising polynucleotides encoding for a GHRH gene, a GH gene or a gene encoding a fusion protein consisting of a peptide from GH and a carrier protein for induction of GH potency-enhancing antibodies; (iv) recombinant proteins, peptides, fragments or derivatives thereof comprising canine GHRH or feline GHRH; (v) recombinant proteins, peptides, fragments or derivatives thereof of the GHRH variants of the present invention; and (vi) recombinant fusion proteins, peptides, fragments or derivatives thereof comprising GH and GHRH.

In one embodiment, the pharmaceutical compositions of the present invention comprise polynucleotide sequences encoding canine or feline GHRH alone or in combination with polynucleotide sequences encoding GHRH variant, modified GHRH, GH and/or modified GH or any combination thereof. In another embodiment, the pharmaceutical compositions of the present invention comprise canine or feline GHRH polypeptides alone or in combination with GHRH variant polypeptides, modified GHRH, GH polypeptides or GH modified polypeptides or any combination thereof. The pharmaceutical compositions of the present invention are in suitable formulation to be administered to humans, companion animals, livestock or poultry for the treatment of growth hormone related deficiencies or the enhancement of growth and performance of livestock and poultry. The pharmaceutical compositions of the present invention are also in suitable formulation for the treatment of obesity and frailty of companion animals or the improvement in the health of humans, companion animals, livestock, and poultry.

The present invention relates to therapeutic methods and compositions for the treatment of growth hormone related deficiencies comprising growth hormone ("GH"); modified GH; growth hormone releasing hormone ("GHRH"); GHRH variants; modified GHRH or any combination thereof The therapeutic compositions of the invention are administered to animals, preferably to mammals, more preferably to companion animals (*e.g*., dogs, cats and horses), livestock (*e.g*., cows and pigs) and poultry (*e.g*., chickens and turkeys), and most preferably to humans. The invention also relates to methods and compositions for the enhancement of the growth and performance of animals, more preferably mammals, and most preferably livestock (*e.g*., cows and pigs) and poultry (*e.g*., chickens and turkeys) with the proviso that such compositions are not to be administered to mice, rats, rodents, guinea pigs, or rabbits. The invention also relates to methods and compositions for the treatment of obesity and frailty of animals, preferably to mammals, more preferably to companion animals (*e.g*., dogs, cats and horses). The invention further relates to methods and compositions for the improvement in the health of animals, preferably to mammals, more preferably to companion animals (*e.g*., dogs, cats and horses), livestock (*e.g*., cows and pigs) and poultry (*e.g*., chickens and turkeys), and most preferably to humans.

The present invention is based in part of the discovery of recombinantly engineered GHRH variants which retain at least the level of activity wildtype GHRH, that is the ability to induce GH gene transcription at levels comparable to wildtype GHRH, and which have enhanced resistance to enzymatic degradation relative to the wildtype GHRH. The GHRH variants of the present invention may be recombinantly expressed at high levels in host cells and easily isolated and purified in a form suitable for administration to humans and animals. Thus, the GHRH variants of the present invention may be efficiently produced and isolated at high levels as opposed to modified GHRH polypeptides in the art which are modified using traditional chemistry methods to introduce modifications in the native GHRH sequence.

In one embodiment, a GHRH variant of the present invention comprises the addition of one amino acid, preferably a hydrophobic residue and more preferably a tyrosine residue, to the amino terminus (position 1) of GHRH. In another embodiment, a GHRH variant comprises the addition of two amino acids, wherein the second amino acid is not proline or alanine, to the amino terminus (position 1) of GHRH. In another embodiment, a GHRH variant comprises the addition of three amino acids, wherein the second amino acid is proline or alanine, to the amino terminus (position 1) of GHRH. In another embodiment, a GHRH variant comprises the addition of more than three amino acids to the amino terminus (position 1) of GHRH, wherein the addition does not interfere with the functional activity of GHRH, that is, the ability of GHRH to induce GH gene transcription. In a preferred embodiment of the present invention, a GHRH variant comprises the addition of a tripeptide to the amino terminus, wherein the tripeptide is diprotin A or diprotin B or a peptide with a structure analogous to diprotin A or diprotin B. In yet another embodiment, a GHRH variant comprises the addition of glycine and arginine at the carboxy-terminus. This addition results in the amidation of GHRH; the glycine and arginine is cleaved off and the last amino acid before the added glycine is amidated.

In one embodiment, a GHRH variant comprises any of the amino acid additions described above and the substitution of glycine with alanine at residue 15. In another embodiment, a GHRH variant comprises any of the amino acid additions described above and the substitution of leucine with alanine at residue 22. In another embodiment, a GHRH variant comprises any of the amino acid additions described above and the substitutions of glycine with alanine at residue 15 and leucine with alanine at residue 22. In another embodiment, a GHRH variant comprises any of the amino acid additions at the amino terminus and the amino acid additions at the carboxy-terminus. In another embodiment, a GHRH variant comprises any of the amino acid additions at the amino terminus described above, the amino acid additions at the carboxy-terminus described above, and the substitution of glycine to alanine at residue 15. In another embodiment, a GHRH variant comprises any of the amino acid additions at the amino terminus described above, the amino acid additions at the carboxy-terminus described above, and the substitution of leucine to alanine at residue 22. In yet another embodiment, a GHRH variant comprises any of the amino acid additions at the amino terminus described above, the amino acid additions at the carboxy-terminus described above, and the substitutions of glycine to alanine at residue 15 and leucine to alanine at residue 22. The term "GHRH precursor variant" as used herein refers to a precursor form of the full length wildtype GHRH polypeptide to which one or more amino acids have been attached to the amino terminus of the polypeptide and/or contains a substitution of one or more amino acids, so that the GHRH precursor variant retains at least equal or enhanced wildtype GHRH activity and has enhanced resistance to enzymatic degradation relative to wildtype GHRH. In one embodiment of the present invention, a GHRH precursor variant comprises the amino acid additions and/or the amino acid substitutions described above. The present invention also encompasses a fusion variant comprising any of the GHRH variants described above and GH or modified GH. The term "fusion variant" as used herein refers to a fusion protein comprising GHRH variants or modified GHRH and GH or modified GH. In one embodiment of the present invention, a fusion variant comprises any of the GHRH variants described above, which consist of amino acid additions at the amino terminus and/or amino acid substitutions, and GH or modified GH.

The modifications and/or substitutions of GHRH described herein are made to GHRH polypeptides which retain the biological activity at least equal to the full length wildtype GHRH, preferably a precursor form of GHRH, more preferably the sequence of GHRH consisting of about 29 amino acids to about 44 amino acids. The polynucleotide sequences encoding the GHRH variants described herein are also within the scope of the present invention. The present invention provides that the polypeptides are encoded by the nucleic acid fragments of the present invention or by degenerate variants of the nucleic acid fragments of the present invention. By "degenerate variant" is intended nucleotide fragments which differ from a nucleic acid fragment of the present invention (*e.g*., an ORF) by nucleotide sequence but, due to the degeneracy of the genetic code, encode an identical polypeptide sequence. Preferred nucleic acid fragments of the present invention are the ORFs that encode proteins. The present invention encompasses GHRH variants encoded by the polynucleotide sequence any species.

The present invention encompasses polynucleotide sequences encoding precursor forms of GHRH, full length wildtype GHRH, modified GHRH, GHRH variants, and fragments of GHRH from 29 amino acids to 44 amino acids in length for any species, which retain at least the activity of wildtype GHRH. For example, the polynucleotide sequences encoding human, swine, and bovine growth hormone releasing hormone disclosed in Genbank accession number SEG_HSGHRH, accession number U90275, and accession number U29611, respectively, are incorporated herein by reference. The present invention also encompasses polynucleotide sequences encoding GHRH polypeptides disclosed for any species (*e.g*., the polynucleotide sequence encoding the human GHRH precursor polypeptide disclosed in Genbank accession number P01286 is incorporated herein by reference). The present invention further encompasses polynucleotide sequences encoding full length wildtype GH or modified GH for any species. For example, the polynucleotide sequences encoding human, swine, and bovine growth hormone disclosed in Genbank accession number J03071, accession number U19787, and accession number E00293, respectively, are incorporated herein by reference. The present invention also encompasses polynucleotide sequences encoding GH polypeptides disclosed for any species (*e.g*., the polynucleotide sequence encoding the bovine GH polypeptide disclosed in Genbank accession number STBO is incorporated herein by reference).

The polynucleotide sequence encoding GHRH, modified GHRH or GHRH variants can be inserted into an appropriate expression vector, *i.e*., a vector which contains the necessary elements for the transcription and translation of the inserted protein-coding sequence. The polynucleotide sequence encoding GH or modified GH can be inserted into an appropriate expression vector, *i.e*., a vector which contains the necessary elements for the transcription and translation of the inserted protein-coding sequence. The necessary transcriptional and translational signals can also be supplied by the native GH or native GHRH genes or its flanking regions. A variety of host-vector systems may be utilized to express the protein-coding sequence. These include but are not limited to mammalian cell systems infected with virus (*e.g*., vaccinia virus, adenovirus, etc.); insect cell systems infected with virus (*e.g*., baculovirus); microorganisms such as yeast containing yeast vectors, or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used. In one embodiment, the wildtype or modified human GH gene is expressed. In another embodiment, the wildtype, modified or variant human GHRH is expressed. In yet another embodiment, the wildtype or modified human GH and the wildtype, modified or variant human GHRH gene are expressed.

Any of the methods previously described for the insertion of DNA fragments into a vector may be used to construct expression vectors containing a chimeric gene, comprising GH or modified GH and GHRH, modified GHRH or GHRH variants, consisting of appropriate transcriptional and translational control signals and the protein coding sequences. These methods may include *in vitro* recombinant DNA and synthetic techniques and *in vivo* recombinants (genetic recombination). Expression of the nucleic acid sequence encoding GH or modified GH may be regulated by a second nucleic acid sequence so that the GH or modified GH is expressed in a host transformed with the recombinant DNA molecule. Expression of the nucleic acid sequence encoding GHRH, modified GHRH or GHRH variant may be regulated by a second nucleic acid sequence so that the GHRH modified GHRH or GHRH variant is expressed in a host transformed with the recombinant DNA molecule. For example, expression of GH or GHRH may be controlled by any promoter or enhancer element known in the art. Promoters which may be used to control GH and/or GHRH gene expression include, but are not limited to, the Cytomeglovirus (CMV) immediate early promoter region, the SV40 early promoter region (Bernoist and Chambon, 1981, *Nature* 290:304-310), the promoter contained in the 3' long terminal repeat of Rous sarcoma virus (Yamamoto, et al., 1980, *Cell* 22:787-797), the herpes thymidine kinase promoter (Wagner et al., 1981, *Proc. Natl. Acad. Sci. USA* 78:1441-1445), the regulatory sequences of the metallothionein gene (Brinster et al., 1982, *Nature* 296:39-42); prokaryotic expression vectors such as the β-lactamase promoter (Villa-Kamaroff et al., 1978, *Proc. Natl. Acad. Sci. USA* 75:3727-3731), or the *tac* promoter (DeBoer et al., 1983, *Proc. Natl. Acad. Sci. USA* 80:21-25); see also "Useful proteins from recombinant bacteria" in *Scientific American*, 1980, 242:74-94; plant expression vectors comprising the nopaline synthetase promoter region (Herrera-Estrella et al., *Nature* 303:209-213) or the cauliflower mosaic virus 35S RNA promoter (Gardner et al., 1981, *Nucl. Acids Res.* 9:2871), and the promoter of the photosynthetic enzyme ribulose biphosphate carboxylase (Herrera-Estrella et al., 1984, *Nature* 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, *Cell* 38:639-646; Ornitz et al., 1986, *Cold Spring Harbor Symp. Quant. Biol.* 50:399-409; MacDonald, 1987, *Hepatology* 7:425-515); insulin gene control region which is active in pancreatic beta cells (Hanahan, 1985, *Nature* 315:115-122), immunoglobulin gene control region which is active in lymphoid cells (Grosschedl et al., 1984, *Cell* 38:647-658; Adames et al., 1985, *Nature* 318:533-538; Alexander et al., 1987, *Mol. Cell. Biol.* 7:1436-1444), mouse mammary tumor virus control region which is active in testicular, breast, lymphoid and mast cells (Leder et al., 1986, *Cell* 45:485-495), albumin gene control region which is active in liver (Pinkert et al., 1987, *Genes and Devel.* 1:268-276), alpha-fetoprotein gene control region which is active in liver (Krumlauf et al., 1985, *Mol. Cell. Biol.* 5:1639-1648; Hammer et al., 1987, *Science* 235:53-58; alpha 1-antitrypsin gene control region which is active in the liver (Kelsey et al., 1987, *Genes and Devel.* 1:161-171), beta-globin gene control region which is active in myeloid cells (Mogram et al., 1985, *Nature* 315:338-340; Kollias et al., 1986, *Cell* 46:89-94; myelin basic protein gene control region which is active in oligodendrocyte cells in the brain (Readhead et al., 1987, *Cell* 48:703-712); myosin light chain-2 gene control region which is active in skeletal muscle (Sani, 1985, *Nature* 314:283-286), swine alpha-skeletal actin control region which is active in muscle (Reecy, M. et al., 1998, *Animal Biotechnology* 9:101-120) and gonadotropic releasing hormone gene control region which is active in the hypothalamus (Mason et al., 1986, *Science* 234:1372-1378).

In a specific embodiment, a vector is used that comprises a promoter operably linked to GH- or modified GH-encoding nucleic acid, one or more origins of replication, and, optionally, one or more selectable markers (*e.g*., an antibiotic resistance gene). In another embodiment, a vector is used that comprises a promoter operably linked to GHRH-, modified GHRH- or GHRH variant-encoding nucleic acid, one or more origins of replication, and, optionally, one or more selectable markers (*e.g*., an antibiotic resistance gene). In yet another embodiment, a vector is used that comprises a promoter operably linked to GH or modified GH and GHRH, modified GHRH or GHRH variant-encoding nucleic acids, one or more origins of replication, and, optionally, one or more selectable markers (*e.g*., an antibiotic resistance gene).

Expression vectors containing gene inserts can be identified by three general approaches: (a) nucleic acid hybridization, (b) presence or absence of "marker" gene functions, and (c) expression of inserted sequences. In the first approach, the presence of the GH- or modified GH-encoding polynucleotides and GHRH-, modified GHRH- or GHRH variant-encoding polynucleotides inserted in an expression vector(s) can be detected by nucleic acid hybridization using probes comprising sequences that are homologous to the inserted genes. In the second approach, the recombinant vector/host system can be identified and selected based upon the presence or absence of certain "marker" gene functions (*e.g*., thymidine kinase activity, resistance to antibiotics, transformation phenotype, occlusion body formation in baculovirus, etc.) caused by the insertion of the gene(s) in the vector(s). For example, if the GH gene is inserted within the marker gene sequence of the vector, recombinants containing the GH gene insert can be identified by the absence of the marker gene function. In the third approach, recombinant expression vectors can be identified by assaying the gene product expressed by the recombinant. Such assays can be based, for example, on the physical or functional properties of the GH and GHRH in *in vitro* assay systems, *e.g*., binding of GH with anti-GH antibody or binding of GHRH with anti-GHRH antibody.

Once a particular recombinant DNA molecule is identified and isolated, several methods known in the art may be used to propagate it. Once a suitable host system and growth conditions are established, recombinant expression vectors can be propagated and prepared in quantity. As previously explained, the expression vectors which can be used include, but are not limited to, the following vectors or their derivatives: human or animal viruses such as vaccinia virus or adenovirus; insect viruses such as baculovirus; yeast vectors; bacteriophage vectors (*e.g*., lambda), and plasmid and cosmid DNA vectors, to name but a few.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered may be controlled. Furthermore, different host cells have characteristic and specific mechanisms for the translational and post-translational processing and modification (*e.g*., glycosylation, phosphorylation of proteins). Appropriate cell lines or host systems can be chosen to ensure the desired modification and processing of the foreign protein expressed. For example, expression in a bacterial system can be used to produce an unglycosylated core protein product. Expression in yeast will produce a glycosylated product. Expression in mammalian cells can be used to ensure "native" glycosylation of a heterologous protein. Furthermore, different vector/host expression systems may effect processing reactions to different extents.

For long-term, high-yield production of recombinant proteins, stable expression is preferred. For example, cell lines which stably express the differentially expressed or pathway gene protein may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the differentially expressed or pathway gene protein. Such engineered cell lines may be particularly useful in screening and evaluation of compounds that affect the endogenous activity of the differentially expressed or pathway gene protein.

A number of selection systems may be used, including but not limited to the herpes simplex virus thymidine kinase (Wigler, et al., 1977, *Cell* 11:223), hypoxanthine-guanine phosphoribosyltransferase (Szybalska & Szybalski, 1962, *Proc. Natl. Acad. Sci. USA* 48:2026), and adenine phosphoribosyltransferase (Lowy et al., 1980, *Cell* 22:817) genes can be employed in tk⁻, hgprt⁻ or aprt⁻ cells, respectively. Also, antimetabolite resistance can be used as the basis of selection for dhfr, which confers resistance to methotrexate (Wigler et al., 1980, *Natl. Acad. Sci. USA* 77:3567; O'Hare et al., 1981, *Proc. Natl. Acad. Sci. USA* 78:1527); gpt, which confers resistance to mycophenolic acid (Mulligan & Berg, 1981, *Proc. Natl. Acad. Sci. USA* 78:2072); neo, which confers resistance to the aminoglycoside G-418 (Colberre-Garapin et al., 1981, *J. Mol. Biol.* 150:1); and hygro, which confers resistance to hygromycin (Santerre et al., 1984, *Gene* 30:147) genes.

The present invention provides for the treatment or prevention of GH associated diseases or disorders, including those disorders characterized by GH deficiency, comprising the administration of a pharmaceutical formulation to a human, companion animal, livestock or poultry which enhances GH expression and/or release. The present invention also provides for methods and protocols to enhance the growth and performance of livestock and poultry, comprising the administration of a pharmaceutical formulation to a companion animal, livestock or poultry which enhances GH expression and/or release. The present invention also provides for the treatment of obesity and frailty, comprising the administration of a pharmaceutical formulation to a companion animal which enhances or modulates GH expression and/or release. The present invention further provides for methods for the improvement in health, comprising the administration of a pharmaceutical formulation to a human, companion animal, livestock or poultry which enhances GH expression and/or release. In accordance with the invention, methods of the present invention encompass the administration of pharmaceutical formulations comprising: (a) polynucleotide sequences encoding GHRH variants alone or in combination with polynucleotide sequences encoding GHRH, modified GHRH, GH, modified GH or any combination thereof, wherein the polynucleotide sequences are operably linked to a promoter or regulatory element, preferably one that is transcriptionally active in muscle tissue; (b) polynucleotide sequences encoding canine or feline GHRH alone or in combination with polynucleotide sequences encoding GHRH variants, modified GHRH, GH, modified GH or any combination thereof, wherein the polynucleotide sequences are operably linked to a promoter or regulatory element, preferably one that is transcriptionally active in muscle tissue; (c) polynucleotide sequences encoding GHRH, modified GHRH, GH, modified GH or any combination thereof wherein the polynucleotide sequences are operably linked to a promoter or regulatory element, preferably one that is transcriptionally active in muscle tissue; (c) variant GHRH polypeptides alone, expressed as a fusion protein, or in combination with GHRH, modified GHRH, GH or modified GH polypeptides or any combination thereof; or (d) canine or feline GHRH polypeptides alone, expressed as a fusion protein, or in combination with GHRH variants, modified GHRH, GH or modified GH polypeptides or any combination thereof.

Generally, administration of products of a species origin or species reactivity (in the case of antibodies) that is the same species as that of the patient is preferred. Thus, in a preferred embodiment, human GH and/or GHRH genes, gene fragments or derivatives thereof are administered to a human patient for therapy or prophylaxis.

In a specific embodiment, nucleic acids comprising sequences encoding GH and/or GHRH or functional derivatives thereof, are administered to promote the release and/or elevation of growth hormone, by way of gene therapy. Gene therapy refers to therapy performed by the administration to a subject of an expressed or expressible nucleic acid. In this embodiment of the invention, the nucleic acids produce their encoded protein that mediate a therapeutic effect by promoting the function of GH.

Any of the methods for gene therapy available in the art can be used according to the present invention. Exemplary methods are described below.

For general reviews of the methods of gene therapy, see Goldspiel et al., 1993, *Clinical Pharmacy* 12:488-505; Wu and Wu, 1991, *Biotherapy* 3:87-95; Tolstoshev, 1993, *Ann. Rev. Pharmacol. Toxicol.* 32:573-596; Mulligan, 1993, *Science* 260:926-932; and Morgan and Anderson, 1993, *Ann. Rev. Biochem.* 62:191-217; May, 1993, TIBTECH 11(5):155-215). Methods commonly known in the ad of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

In a preferred aspect, the compound comprises nucleic acid sequences encoding GH or modified GH and GHRH, modified GHRH or GHRH variants, said nucleic acid sequences being part of expression vectors that express GH or modified GH and GHRH, modified GHRH or GHRH variants in a suitable host. In particular, such nucleic acid sequences have promoters operably linked to the GH or modified GH and GHRH, modified GHRH or GHRH variants coding regions, said promoters being inducible or constitutive, and, optionally, tissue-specific. In another particular embodiment, nucleic acid molecules are used in which the GH or modified GH and GHRH, modified GHRH or GHRH variants coding sequences and any other desired sequences are flanked by regions that promote homologous recombination at a desired site in the genome, thus providing for intrachromosomal expression of the GH or modified GH and GHRH, modified GHRH or GHRH variants nucleic acids (Koller and Smithies, 1989, *Proc. Natl. Acad. Sci. USA* 86:8932-8935; Zijlstra et al., 1989, *Nature* 342:435-438).

Delivery of the nucleic acids into a patient may be either direct, in which case the patient is directly exposed to the nucleic acid or nucleic acid-carrying vectors, or indirect, in which case, cells are first transformed with the nucleic acids *in vitro*, then transplanted into the patient. These two approaches are known, respectively, as *in vivo* or *ex vivo* gene therapy.

In a specific embodiment, the nucleic acid sequences are directly administered *in vivo*, where it is expressed to produce the encoded product. This can be accomplished by any of numerous methods known in the art, *e.g*., by constructing them as part of an appropriate nucleic acid expression vector and administering it so that they become intracellular, *e.g*., by infection using defective or attenuated retrovirals or other viral vectors (see U.S. Patent No. 4,980,286), or by direct injection of naked DNA, or by use of microparticle bombardment (*e.g*., a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, *e.g*., Wu and Wu, 1987, *J. Biol. Chem.* 262:4429-4432) (which can be used to target cell types specifically expressing the receptors), etc. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g*., PCT Publications WO 92/06180 dated April 16, 1992 (Wu et al.); WO 92/22635 dated December 23, 1992 (Wilson et al.); WO92/20316 dated November 26, 1992 (Findeis et al.); WO93/14188 dated July 22, 1993 (Clarke et al.), WO 93/20221 dated October 14, 1993 (Young)). Alternatively, the nucleic acid can be introduced intracellularly and incorporated within host cell DNA for expression, by homologous recombination (Koller and Smithies, 1989, *Proc. Natl. Acad. Sci. USA* 86:8932-8935; Zijlstra et al., 1989, *Nature* 342:435-438).

In a specific embodiment, viral vectors that contain nucleic acid sequences encoding GH or modified GH and/or GHRH, modified GHRH or GHRH variants are used. For example, a retroviral vector can be used (see Miller et al., 1993, *Meth. Enzymol*. 217:581-599). These retroviral vectors have been to delete retroviral sequences that are not necessary for packaging of the viral genome and integration into host cell DNA. The nucleic acid sequences encoding the GH or modified GH and GHRH, modified GHRH or GHRH variants to be used in gene therapy are cloned into one or more vectors, which facilitates delivery of the gene into a patient. More detail about retroviral vectors can be found in Boesen et al., 1994, *Biotherapy* 6:291-302, which describes the use of a retroviral vector to deliver the mdr1 gene to hematopoietic stem cells in order to make the stem cells more resistant to chemotherapy. Other references illustrating the use of retroviral vectors in gene therapy are: Clowes et al., 1994, *J. Clin. Invest.* 93:644-651; Kiem et al., 1994, *Blood* 83:1467-1473; Salmons and Gunzberg, 1993, *Human Gene Therapy* 4:129-141; and Grossman and Wilson, 1993, *Curr. Opin. in Genetics and Devel.* 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, *Current Opinion in Genetics and Development* 3:499-503 present a review of adenovirus-based gene therapy. Bout et al., 1994, *Human Gene Therapy* 5:3-10 demonstrated the use of adenovirus vectors to transfer genes to the respiratory epithelia of rhesus monkeys. Other instances of the use of adenoviruses in gene therapy can be found in Rosenfeld et al., 1991, *Science* 252:431-434; Rosenfeld at al., 1992, *Cell* 68:143-155; Mastrangeli et al., 1993, *J. Clin. Invest.* 91:225-234; PCT Publication WO94/12649; and Wang, at al., 1995, *Gene Therapy* 2:775-783. In a preferred embodiment, adenovirus vectors are used. Adeno-associated virus (AAV) has also been proposed for use in gene therapy (Walsh et al., 1993, *Proc. Soc. Exp. Biol. Med.* 204:289-300; U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, *e.g*., Loeffler and Behr, 1993, *Meth. Enzymol.* 217:599-618; Cohen et al., 1993, *Meth. Enzymol.* 217:618-644; Cline, 1985, *Pharmac. Ther.* 29:69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. Recombinant blood cells (*e.g*., hematopoietic stem or progenitor cells) are preferably administered intravenously. The amount of cells envisioned for use depends on the desired effect, subject=s state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, *e.g*., as obtained from bone marrow, umbilical cord blood, peripheral blood, fetal liver, etc.

In a preferred embodiment, the cell used for gene therapy is autologous to the subject.

In an embodiment in which recombinant cells are used in gene therapy, nucleic acid sequences encoding GH or modified GH and/or GHRH, modified GHRH or GHRH variants are introduced into the cells such that they are expressible by the cells or their progeny, and the recombinant cells are then administered *in vivo* for therapeutic effect. In a specific embodiment, stem or progenitor cells are used. Any stem and/or progenitor cells which can be isolated and maintained *in vitro* can potentially be used in accordance with this embodiment of the present invention (see e.g. PCT Publication WO 94/08598, dated April 28, 1994; Stemple and Anderson, 1992, *Cell* 71:973-985; Rheinwald, 1980, *Meth. Cell Bio*. 21A:229; and Pittelkow and Scott, 1986, *Mayo Clinic Proc*. 61:771).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

The polypeptides of the invention include polypeptides which comprise the amino acid sequence of canine or feline GHRH. The polypeptides of the invention also include polypeptides which comprise the amino acid sequence of a GHRH variant of the present invention. The polypeptides of the invention further include polypeptides which comprise the amino acid sequence of GH or modified and GHRH or modified GHRH. Protein compositions of the present invention may further comprise an acceptable carrier, such as a hydrophilic, *e.g*., pharmaceutically acceptable, carrier.

The invention also relates to methods for producing a polypeptide comprising growing a culture of the cells of the invention in a suitable culture medium, and purifying the protein from the culture. For example, the methods of the invention include a process for producing a polypeptide in which a host cell containing a suitable expression vector that includes a polynucleotide of the invention is cultured under conditions that allow expression of the encoded polypeptide. The polypeptide can be recovered from the culture, conveniently from the culture medium, and further purified.

The present invention further provides isolated polypeptides encoded by the nucleic acid fragments of the present invention or by degenerate variants of the nucleic acid fragments of the present invention. By "degenerate variant is intended nucleotide fragments which differ from a nucleic acid fragment of the present invention (e.g., an ORF) by nucleotide sequence but, due to the degeneracy of the genetic code, encode an identical polypeptide sequence. Preferred nucleic acid fragments of the present invention are the ORFs that encode proteins. A variety of methodologies known in the art can be utilized to obtain any one of the isolated polypeptides or proteins of the present invention. At the simplest level, the amino acid sequence can be synthesized using commercially available peptide synthesizers. This is particularly useful in producing small peptides and fragments of larger polypeptides. Fragments are useful, for example, in generating antibodies against the native polypeptide. In an alternative method, the polypeptide or protein is purified from bacterial cells which naturally produce the polypeptide or protein. One skilled in the art can readily follow known methods for isolating polypeptides and proteins in order to obtain one of the isolated polypeptides or proteins of the present invention. These include, but are not limited to, immunochromatography, HPLC, size-exclusion chromatography, ion-exchange chromatography, and immuno-affinity chromatography. *See, e.g*., Scopes, *Protein Purification:* *Principles and Practice*, Springer-Verlag (1994); Sambrook, *et al*., in *Molecular Cloning: A Laboratory Manual*; Ausubel *et al*., *Current Protocols in Molecular Biology*.

The polypeptides and proteins of the present invention can alternatively be purified from cells which have been altered to express the desired polypeptide or protein. As used herein, a cell is said to be altered to express a desired polypeptide or protein when the cell, through genetic manipulation, is made to produce a polypeptide or protein which it normally does not produce or which the cell normally produces at a lower level. One skilled in the art can readily adapt procedures for introducing and expressing either recombinant or synthetic sequences into eukaryotic or prokaryotic cells in order to generate a cell which produces one of the polypeptides or proteins of the present invention. The purified polypeptides can be used in *in vitro* binding assays which are well known in the art to identify molecules which bind to the polypeptides. These molecules include but are not limited to, for *e.g*., small molecules, molecules from combinatorial libraries, antibodies or other proteins.

The protein of the invention may also be expressed as a product of transgenic animals, e.g., as a component of the milk of transgenic cows, goats, pigs, or sheep which are characterized by somatic or germ cells containing a nucleotide sequence encoding the protein.

The protein may also be produced by known conventional chemical synthesis. Methods for constructing the proteins of the present invention by synthetic means are known to those skilled in the art. The synthetically-constructed protein sequences, by virtue of sharing primary, secondary or tertiary structural and/or conformational characteristics with proteins may possess biological properties in common therewith, including protein activity. Thus, they may be employed as biologically active or immunological substitutes for natural, purified proteins in screening of therapeutic compounds and in immunological processes for the development of antibodies.

The protein may also be produced by operably linking the isolated polynucleotide of the invention to suitable control sequences in one or more insect expression vectors, and employing an insect expression system. Materials and methods for baculovirus/insect cell expression systems are commercially available in kit form from, e.g., Invitrogen, San Diego, Calif., U.S.A. (the MaxBat.RTM. kit), and such methods are well known in the art, as described in Summers and Smith, Texas Agricultural Experiment Station Bulletin No. 1555 (1987), incorporated herein by reference. As used herein, an insect cell capable of expressing a polynucleotide of the present invention is "transformed."

The protein of the invention may be prepared by culturing transformed host cells under culture conditions suitable to express the recombinant protein. The resulting expressed protein may then be purified from such culture (i.e., from culture medium or cell extracts) using known purification processes, such as gel filtration and ion exchange chromatography. The purification of the protein may also include an affinity column containing agents which will bind to the protein; one or more column steps over such affinity resins as concanavalin A-agarose, heparin-toyopearl.RTM. or Cibacrom blue 3GA Sepharose.RTM.; one or more steps involving hydrophobic interaction chromatography using such resins as phenyl ether, butyl ether, or propyl ether; or immunoaffinity chromatography.

Alternatively, the protein of the invention may also be expressed in a form which will facilitate purification. For example, it may be expressed as a fusion protein, such as those of maltose binding protein (MBP), glutathione-S-transferase (GST) or thioredoxin (TRX). Kits for expression and purification of such fusion proteins are commercially available from New England BioLab (Beverly, Mass.), Pharmacia (Piscataway, N.J.) and In Vitrogen, respectively. The protein can also be tagged with an epitope and subsequently purified by using a specific antibody directed to such epitope. One such epitope ("Flag") is commercially available from Kodak (New Haven, Conn.).

Finally, one or more reverse-phase high performance liquid chromatography (RP-HPLC) steps employing hydrophobic RP-HPLC media, e.g., silica gel having pendant methyl or other aliphatic groups, can be employed to further purify the protein. Some or all of the foregoing purification steps, in various combinations, can also be employed to provide a substantially homogeneous isolated recombinant protein. The protein thus purified is substantially free of other mammalian proteins and is defined in accordance with the present invention as an "isolated protein."

The compounds of the invention are preferably tested *in vitro*, and then *in vivo* for the desired therapeutic or prophylactic activity, prior to use in humans. For example, *in vitro* assays which can be used to determine whether administration of a specific compound is indicated, include *in vitro* cell culture assays in which a patient tissue sample is grown in culture, and exposed to or otherwise administered a compound, and the effect of such compound upon the tissue sample is observed.

The expression of GH or modified GH and GHRH, modified GHRH or GHRH variants can be assayed by the immunoassays, gel electrophoresis followed by visualization, or any other method known to those skilled in the art.

In various specific embodiments, *in vitro* assays can be carried out with representative cells of cell types involved in a patient's disorder, to determine if a compound has a desired effect upon such cell types. In accordance with the present invention, the functional activity of GHRH can be measured by its ability to induce GH gene transcription *in vitro*. In accordance with the present invention, the functional activity of GHRH can be measured by its ability to induce IGF gene transcription *in vitro*.

Compounds for use in therapy can be tested in suitable animal model systems prior to testing in humans, including but not limited to pigs, chicken, cows or monkeys.

The invention provides methods of treatment (and prophylaxis) by administration to a subject of an effective amount of a compound of the invention. In a preferred aspect, the compound is substantially purified (*e.g*., substantially free from substances that limit its effect or produce undesired side-effects). The subject is preferably an animal, including but not limited to animals such as cows, pigs, horses, chickens, cats, dogs, etc., and is preferably a mammal, and most preferably human. In a specific embodiment, a non-human mammal is the subject.

Formulations and methods of administration that can be employed when the compound comprises a nucleic acid are described above; additional appropriate formulations and routes of administration can be selected from among those described herein below.

Various delivery systems are known and can be used to administer a compound of the invention, *e.g*., encapsulation in liposomes, microparticles, microcapsules, recombinant cells capable of expressing the compound, receptor-mediated endocytosis (see, *e.g*., Wu and Wu, 1987, *J. Biol. Chem.* 262:4429-4432), construction of a nucleic acid as part of a retroviral or other vector, etc. Methods of introduction include but are not limited to intratumoral, intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (*e.g*., oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g*., by use of an inhaler or nebulizer, and formulation with an aerosolizing agent.

In a specific embodiment, it may be desirable to administer the pharmaceutical compositions of the invention locally to the area in need of treatment; this may be achieved by, for example, and not by way of limitation, local infusion during surgery, topical application, *e.g*., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the compound can be delivered in a vesicle, in particular a liposome (see Langer, *Science* 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, *ibid*., pp. 317-327; see generally *ibid*.)

In yet another embodiment, the compound can be delivered in a controlled release system. In one embodiment, a pump may be used (*see* Langer, *supra*; Sefton, *CRC Crit. Ref. Biomed. Eng.* 14:201 (1987); Buchwald et al., *Surgery* 88:507 (1980); Saudek et al., *N. Engl. J. Med.* 321:574 (1989)). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, *J. Macromol. Sci. Rev. Macromol. Chem*. 23:61 (1983); *see also* Levy et al., *Science* 228:190 (1985); During et al., *Ann. Neurol.* 25:351 (1989); Howard et al., *J. Neurosurg.* 71:105 (1989)). In yet another embodiment, a controlled release system can be placed in proximity of the therapeutic target, *i.e*., the brain, thus requiring only a fraction of the systemic dose (*see, e.g*., Goodson, in Medical Applications of Controlled Release, *supra*, vol. 2, pp. 115-138 (1984)).

Other controlled release systems are discussed in the review by Langer (*Science* 249:1527-1533 (1990)).

In a specific embodiment where the compound of the invention is a nucleic acid encoding a protein, the nucleic acid can be administered *in vivo* to promote expression of its encoded protein, by constructing it as part of an appropriate nucleic acid expression vector and administering it so that it becomes intracellular, *e.g*., by use of a retroviral vector (*see* U.S. Patent No. 4,980,286), or by direct injection, or by use of microparticle bombardment (*e.g*., a gene gun; Biolistic, Dupont), or coating with lipids or cell-surface receptors or transfecting agents, or by administering it in linkage to a homeobox-like peptide which is known to enter the nucleus (see *e.g*., Joliot et al., 1991, *Proc. Natl. Acad. Sci. USA* 88:1864-1868), etc. Alternatively, a nucleic acid can be introduced intracellularly and incorporated within host cell DNA for expression, by homologous recombination.

The present invention also provides pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the compound of the invention which will be effective in the treatment of cancer can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### EXAMPLES

### CLONING OF CANINE AND FELINE GHRH

### 1. CLONING OF CANINE GHRH

In order to clone the canine GHRH, the canine genomic library (Clonetech Lab) is screened with the radioactively labeled fragment encoding the porcine GHRH (SEQ ID No. 1) following protocols known to one of ordinary skill in the art. The fragment encoding the porcine GHRH (SEQ ID No. 1) is labeled using commercially available DNA labeling kits as recommended by the manufacturer. Clones identified that contain the gene coding for the canine precursor GHRH are isolated and sequenced by methods known to one of ordinary skill in the art. The sequence results obtained from sequencing both DNA strands of a clone is compared with sequences of known GHRH species and the canine GHRH is subcloned into appropriate plasmid vectors (*e.g*., pVR1012; Vical, San Diego, CA) according to protocols known to one of ordinary skill in the art.

### 2. CLONING OF FELINE GHRH

In order to clone the feline GHRH, the feline genomic library (Clonetech Lab) is screened with the radioactively labeled fragment encoding the porcine GHRH (SEQ ID No. 1) following protocols known to one of ordinary skill in the art. The fragment encoding the porcine GHRH (SEQ ID No. 1) is labeled using commercially available DNA labeling kits as recommended by the manufacturer. Clones identified that contain the gene coding for the feline precursor GHRH are isolated and sequenced by methods known to one of ordinary skill in the art. The sequence results obtained from sequencing both DNA strands of a clone is compared with sequences of known GHRH species and the feline GHRH is subcloned into appropriate plasmid vectors (*e.g*., pVR1012; Vical, San Diego, CA) according to protocols known to one of ordinary skill in the art.

### SYNTHESIS OF GHRH CONSTRUCTS

### 3. pGHRH-4 (pGHRH1-44WTCMV)

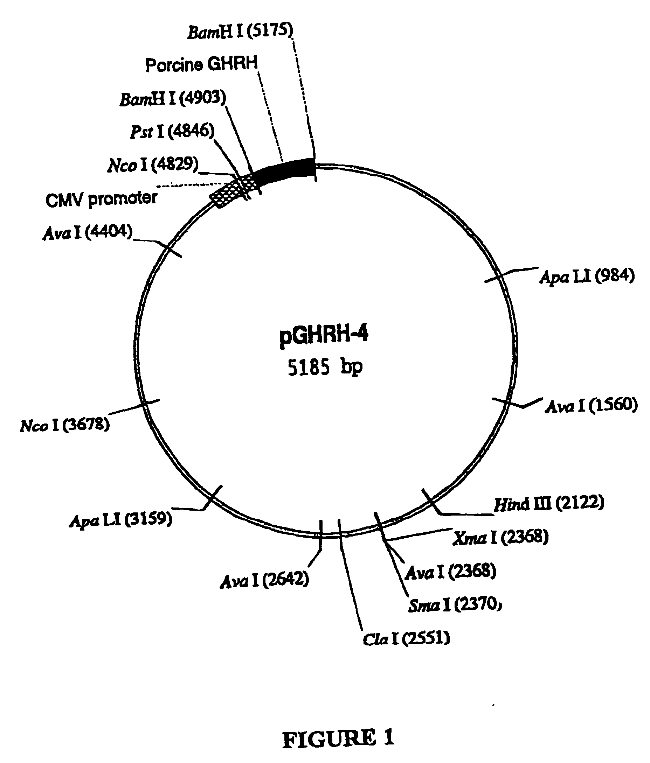

In order to construct a plasmid containing the gene that codes for the natural porcine GHRH polypeptide and to have the latter secreted into the blood circulation when such plasmids are injected into animals, primers designated GHRH-1 (SEQ ID No. 61), GHRH-2 (SEQ ID No. 62), GHRH-4 (SEQ ID No. 66), and GHRH-7 (SEQ ID No. 67) were synthesized. The primers were used in reverse transcription polymerase chain reactions (RT-PCRs) to amplify the human GHRH signal sequence from human mRNA and the porcine GHRH protein sequence from porcine mRNA. The resulting PCR-amplified human GHRH signal sequence and porcine GHRH protein sequence were digested with Bgl II and Bam HI, respectively. Then the fragments were ligated together and cloned into the Bam HI site of the plasmid pVR1012 (Vical, San Diego, CA) to produce a plasmid designated pGHRH-4 (Figure 1). The expression of the GHRH oligonucleotide (SEQ ID Nos. 1 and 2), which encodes a 75 amino acid polypeptide comprising the porcine GHRH protein sequence (44 amino acids) preceded by the signal sequence from human GHRH protein (31 amino acids), is driven by Cytomegalo-virus immediate early (CMV IE) promoter/enhancer element.

### 4. pGHRH1-44WTSK685:

A plasmid containing the polynucleotide sequences encoding the 75-amino acid GHRH protein described above driven by a 685 bp fragment derived from the swine α-skeletal actin promoter (SEQ ID No. 3) was constructed as described below.

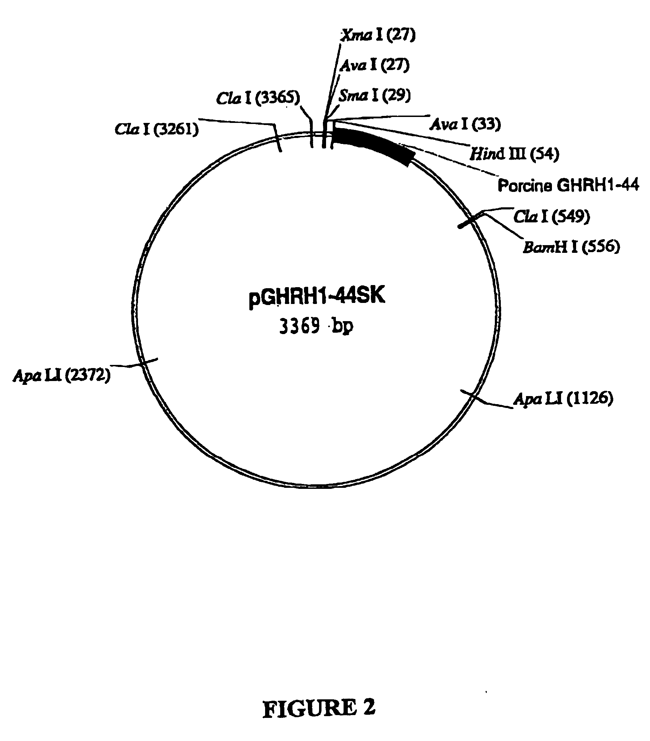

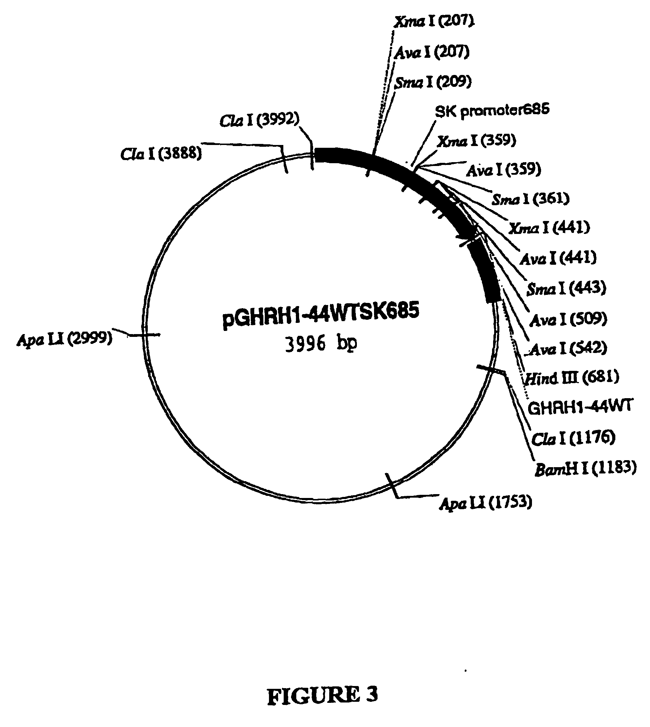

The oligonucleotide fragment encoding the 75 amino acid GHRH protein (SEQ ID No. 1) was PCR-amplified from plasmid pGHRH-4 using a primer designated p97-S1 containing a Hind III site SEQ ID No. 4) and a primer designated p97-A258, containing an Xba I site (SEQ ID No. 5). The PCR-amplified sequence was then cloned into the Hind III-Xba I site of plasmid pGL3 basic (Promega) to produce a plasmid designated GHRH1-44 SK (Figure 2). A 685 bp fragment corresponding to a portion of the porcine α-skeletal actin promoter designated SK685 was PCR-amplified from swine genomic DNA using primer designated SK-3, containing a Kpn I site (SEQ ID No. 6) and primer designated SK-4, containing a Hind III site; (SEQ ID No. 7). The PCR-amplified SK685 promoter fragment was then cloned into plasmid GHRH1-44SK digested with Kpn I and HindIII enzymes to produce a plasmid designated GHRH1-44WTSK685 (Figure 3).

### 5. pGHRH1-44WTSK2014

A plasmid containing the polynucleotide sequences encoding the 75-amino acid GHRH protein described above operatively linked to a fragment derived from the swine α-skeletal actin promoter approximately 2014 bp (SEQ ID No. 8) was constructed as described below.

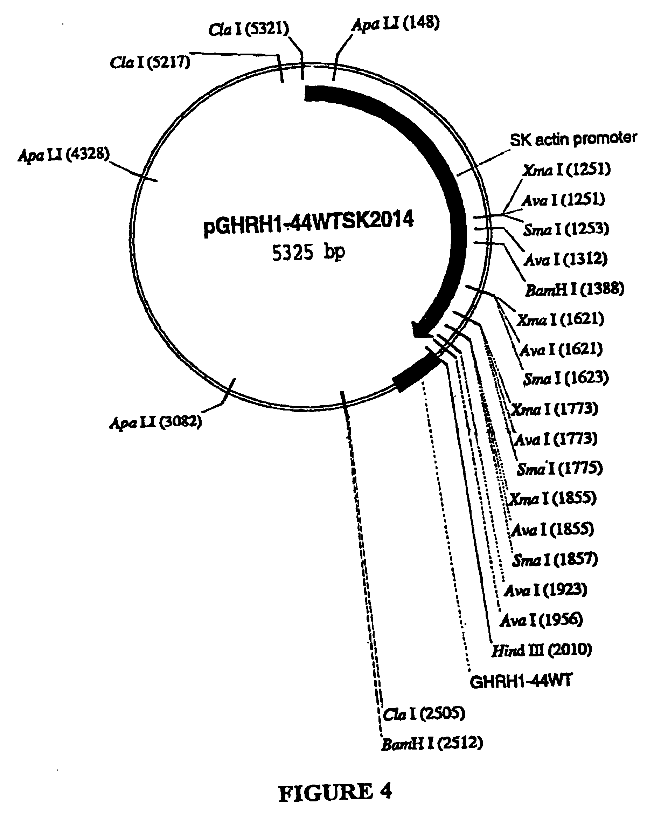

An approximately 2014 bp fragment designated SK 2014 corresponding to a portion of the porcine α-skeletal actin promoter was PCR-amplified from swine genomic DNA using primer designated SK-7; containing a Kpn I site (SEQ ID No. 9) and primer designated SK-8; containing a Hind III site; (SEQ ID No. 10). The PCR-amplified SK2014 promoter fragment was then cloned into plasmid GHRH1-44SK which was digested with Kpn I and Hind III enzymes to produce a plasmid designated GHRH1-44WTSK2014 (Figure 4).

### 6. pGHRH1-29WTCMV

A plasmid containing the polynucleotide sequences encoding the signal sequence derived from human GHRH polynucleotides and encoding amino acids 1-29 of swine GHRH protein was produced as described below.

An approximately 189 bp DNA fragment was PCR-amplified from plasmid pGHRH-4 using primer designated GHRH-5, containing a Bam HI site; (SEQ ID No. 11) and primer designated GHRH-6, containing a Bgl II site; (SEQ ID No. 12). The PCR-amplified fragment was digested with Bam HI and Bgl II enzymes and cloned into plasmid pVR1012 (Vical, San Diego, CA) which was digested with Bam HI and Bgl II enzymes to produce a plasmid designated GHRH1 -29WTCMV (SEQ ID No. 51) in which expression of the GHRH 1-29 protein is driven by CMV IE promoter/enhancer sequences.

### 7. pGHRH1-29YWTCMV

In order to produce novel variants of GHRH protein with enhanced stability to enzymatic degradation (*e.g*. DPPIV enzyme degradation) a plasmid containing polynucleotide sequences encoding the signal sequence of human GHRH and an altered version of the 1-29 porcine GHRH protein was produced. The alteration consisted of the addition of an extra tyrosine residue just preceding the first tyrosine residue of the natural porcine GHRH 1-29 sequence. This modification alters the amino terminal in such away that it is no longer recognized or cleaved by DPPIV enzyme. A plasmid containing the gene for this variant GHRH protein was produced as described below.

A set of overlapping oligonucleotides (SEQ ID Nos. 13-25) were synthesized, mixed, and GHRH was amplified using the PCR method. The PCR reaction resulted in the formation of a fragment of approximately 192 bp encoding amino acids 1-29 of GHRH in which nucleotides encoding a tyrosine residue were inserted immediately 5' to the coding sequence of GHR(1-29) and further containing a Bam HI site (5' end) and an Bgl II site (3' end). The 192 bp fragment was then digested with Bam HI and Bgl II enzymes and cloned into plasmid pVR1012 which was digested with Bam HI and Bgl II enzymes to produce plasmid designated GHRH1-29YWTCMV (SEQ ID No. 52) in which expression of GHRH1-29 (now 30) is driven by CMV IE promoter enhancer elements.

### 8. pGHRH1-29YWTSK685

A plasmid containing the 192 bp fragment described above in Section 5 under the control of the SK685 promoter fragment was produced as described below.

The 192 bp fragment was amplified with two primers designated p99-S1, containing a 5' end Hind III site; (SEQ ID No. 26) and p99-A214, containing a 3' end Xba I site; (SEQ ID No. 27). The PCR-amplified fragment was then digested with Hind III and Xba I enzymes and cloned into plasmid GHRH1-29 Yala1522SK685 (see below) also digested with Hind III and Xba I enzymes to produce plasmid GHRH1-29YWTSK685 (SEQ ID No. 53).

### 9. pGHRH1-29YWTSK2014

A plasmid containing the 192 bp fragment described above in Section 5 under the control of the SK2014 promoter fragment was produced as described below.

The 192 bp fragment was amplified with two primers designated p99-S1 containing a 5' end Hind III site; (SEQ ID No. 28) and p99-A214 containing a 3' end Xba I site; (SEQ ID No. 29). The PCR-amplified fragment was then digested with Hind III and Xba I enzymes and cloned into plasmid GHRH1-29 YAla 1522SK2014 (see below) also digested with Hind III and Xba I enzymes to produce plasmid GHRH1-29YWTSK2014 (SEQ ID No. 54).

### 10. pGHRH1-29YAla1522CMV

In order to produce novel variants of GHRH protein with enhanced stability to enzymatic degradation (*e.g*.. due to DPPIV enzyme) and enhanced potency, a plasmid containing the signal sequence of human GHRH and an altered version of the 1-29 porcine GHRH protein was produced. The alteration consisted of the addition of an extra tyrosine residue just preceding the first tyrosine residue of the natural porcine GHRH 1-29 sequence and replacement of glycine 15 and leucine 22 with alanine. These modifications alter the amino terminal end of GHRH1-29 in such away that it is no longer by recognized or cleaved by the DPP IV enzyme and the modified protein has enhanced potency relative to the 29 or the 44 amino acid GHRH protein. A plasmid containing the gene for this variant protein was produced as described below.

A set of overlapping oligonucleotides were synthesized (SEQ ID Nos. 30-42), mixed and amplified using the PCR method. The PCR reaction resulted in the formation of a fragment of approximately 192 bp containing a Bam HI site (5' end) and an Bgl II site (3' end) and in which an extra three nucleotides encoding for tyrosine is immediately 5' to the nucleotide sequence encoding the natural tyrosine at position 1 of the GHRH1-29 sequence. Furthermore, the alteration included replacement of the 3 nucleotides encoding glycine 15 with three nucleotides encoding alanine and replacement of three nucleotides encoding leucine 22 with three nucleotides encoding alanine. The 192 bp fragment was then digested with Bam HI and Bgl II enzymes and cloned into plasmid pVR1012 which was digested with Bam HI and Bgl II enzymes to produce plasmid designated GHRH1-29YAla 1522CMV in which expression of the variant GHRH1-29 (now 30) is driven by CMV IE promoter enhancer elements (SEQ ID No. 55).

### 11. pGHRH1-29YAla15225K685

A plasmid containing the GHRH1-29YAla1522 fragment described above under the control of the SK685 promoter fragment was produced as described below.

An approximately 192 bp fragment as described above in Section 8 was PCR-amplified with primers designated p99-S1, containing a Hind III at 5' end (SEQ ID No. 26) and p99-A214 containing a Xba I site at 3' end; (SEQ ID No. 27). The PCR-amplified fragment was digested with Hind III and Xba I enzymes and cloned into plasmid *pGL3* (Promega) also digested with Hind III and Xba I to produce plasmid GHRH1-29YAla 1522SK (SEQ ID No. 56). A 685 bp fragment corresponding to a portion of the porcine α-skeletal actin promoter was PCR-amplified from swine genomic DNA using primers SK-3 (Kpn I site) and SK-4 (Hind III site). This fragment was designated SK685. The PCR-amplified SK685 promoter fragment was then cloned into plasmid GHRH1-29YAla1522SK digested with KpnI and HindIII enzymes to produce a plasmid designated GHRH1-29YA1a1522SK685 (SEQ ID No. 57).

### 12. pGHRH1-29Yala1522SK2014

A plasmid containing the GHRH1-29YAla1522 fragment described above under the control of the SK2014 promoter fragment was produced as described below.

An approximately 192 bp fragment as described above in Section 8 was PCR amplified with primers designated p99-S1 containing a Hind III at 5' end; (SEQ ID No. 26) and p99-A214 containing a Xba I site at 3' end; (SEQ ID No. 27). The PCR-amplified fragment was digested with Hind III and Xba I enzymes and cloned into plasmid *pGL3* (Promega) also digested with HindIII and Xba I to produce plasmid GHRH1-29YAla1522SK. An 2014 bp fragment corresponding to a portion of the porcine α-skeletal actin promoter was PCR-amplified from swine genomic DNA using primers SK-7 containing a Kpn I site and SK-8 containing a Hind III site. This fragment was designated SK2014. The PCR-amplified SK2014 promoter fragment was then cloned into plasmid GHRH1-29YAla1522SK digested with Kpn I and Hind III enzymes to produce a plasmid designated GHRH1-29YAla1522SK2014 (SEQ ID No. 58).

### 13. pGHRH 1-44YWTCMV

In order to produce novel variants of GHRH protein with enhanced stability to enzymatic degradation (*e.g*., due to DPPIV enzyme) a plasmid containing the signal sequence of human GHRH and an altered version of the 1-44 porcine GHRH protein was produced. The alteration consisting of the addition of an extra tyrosine residue immediately 5' to the nucleotide sequence encoding the first tyrosine residue of the natural porcine GHRH 1-44 sequence. This modification will alter the amino terminal end in such away that it is no longer by recognized or cleaved by DPPIV enzyme. A plasmid containing the gene for this modified GHRH protein is produced as described below.

A set primers designated GHRH-1 (SEQ ID No. 61) and GHRH-3 (SEQ ID No. 65) were used in a PCR reaction amplify the human GHRH signal sequence and porcine GHRH from pGHRH1-29YWTCMV. The resulting GHRH fragment was digested with Bam HI and Bgl II, and cloned into the Bam HI site of plasmid pVR1012 (Vical, San Diego, CA) to produce the plasmid designated GHRH1-44YWTCMV (SEQ ID No. 59).

### 14. SYNTHESIS OF GH CONSTRUCTS

In order to provide plasmid constructs containing GH genes suitable for treatment of growth hormone maladies or to enhance animal health and productivity, the construction of plasmids of the invention and their methods of use are described below in detail.

The canine GH gene was cloned into plasmids vectors suitable for the various aspects of the present invention using the following procedures. Total RNA was prepared from the pituitary gland of a dog using the RNAzol B method using reagents and procedures from Biotecx Laboratories, Houston TX. Briefly, about 0.15 mg of the tissue was homogenized in 2 ml of RNAzol B solution in a RNase-free glass homogenizer. The material was then divided into two equal halves, and RNA extracted by chloroform and ethanol precipitation. The nucleic acid pellet was dried and taken up in RNase-free water. Reverse transcription (RT) of total RNA was done in a 20 ml volume using 0.02 mg of RNA, 138 pmol of oligo #2 (SEQ ID No. 43), 1 mM MnCl₂, and the recommended amounts of dNTPs and rTth enzyme from the RT-PCR kit purchased from Perkin-Elmer, Norwalk, CT. The reaction was incubated at 70□ for 11 min in a Perkin-Elmer Thermal cycler. The completed (RT) reaction was then subjected to PCR following addition of 66 pmol of oligo #1 (SEQ ID No. 44), 2.5 mM MgCl₂, and chelating buffer from the RT-PCR reaction kit. The PCR conditions were as follows: 94 C, 1 min; 55 C, 1 min; and 72 C, 2 min; for 32 cycles. The □0.7 kb PCR-amplified DNA fragment obtained was cloned into plasmid pCRScript purchased from Stratagene, La Jolla, CA and used according to manufacturer's recommendations. The recombinant plasmid thus generated was termed cCG-SP. The insert fragment was partially sequenced and confirmed to contain the growth hormone ("GH") DNA sequences. cGH-SP plasmid DNA was then used as a template to PCR-amplify using oligonucleotides oligo #3 (SEQ ID No. 45) and oligo #4 (SEQ ID No. 46) using reagents from the PCR system kit from Perkin-Elmer using standard procedures and following cycling conditions: 94 C, 1 min 1 cycle; 94 C, 30 sec; 55 C, 30 sec; 72 C, 1 min; 30 cycles. The □0.7 kb PCR-amplified DNA fragment obtained was subjected to column purification (Qiagen, Chatsworth, CA), and digested with restriction enzymes EcoRV and BglII by standard protocols (Sambrook et al., 1989). The digested PCR fragment was ligated to EcoRV-BglII digested pCMV-MCS, a plasmid derivative of pCMVb (Clontech, Palo Alto, CA), engineered to contain multiple cloning sites in place of lacZ gene. The ligation product was used to transform E. coli, and transformants were selected for resistance to ampicillin (pCMV-MCS-enooded marker). Transformants were analyzed by plasmid DNA preparation and restriction site analysis, and a clone of the GH DNA sequences in pCMV-MCS was isolated (termed pCGH#9). The insert sequences were completely sequenced by standard procedures to confirm presence of GH DNA sequences. The EcoRV-BglII GH fragment from pCGH#9 has also been sub-cloned into gene therapy plasmid VR1012 (obtained from Vical, San Diego, CA). This clone referred to as pC51.

### 15. SYNTHESIS OF GH-GHRH CONSTRUCTS

A GH-GHRH fusion protein, comprising the carboxy terminal 20 amino acids of GH
and full length wildtype GHRH is produced. The full length GHRH gene is PCR amplified from plasmid pGHRH-4 using two primers designated GHRH-1 containing a Bgl II site (SEQ ID No. 61) and GHRH-2 containing a Bam HI site (SEQ ID No. 62). The PCR-amplified fragment is then cloned into the Bam HI site of the pVR1012 plasmid. Two complementary oligonucleotides encoding the carboxy terminal amino acids 172-191 of GH (GH-1 oligo.; SEQ ID No. 62 and GH-2 oligo.; SEQ ID No. 63) are synthesized. The GH-1 and GH-2 oligonucleotides are annealed and cloned into the Bam HI site of the pVR1012 plasmid containing the full length GHRH gene to produce pGHRH1-44WTGHpep (SEQ ID No. 60).

### 16. IN VITRO STUDIES ASSESSING GHRH EXPRESSION LEVELS

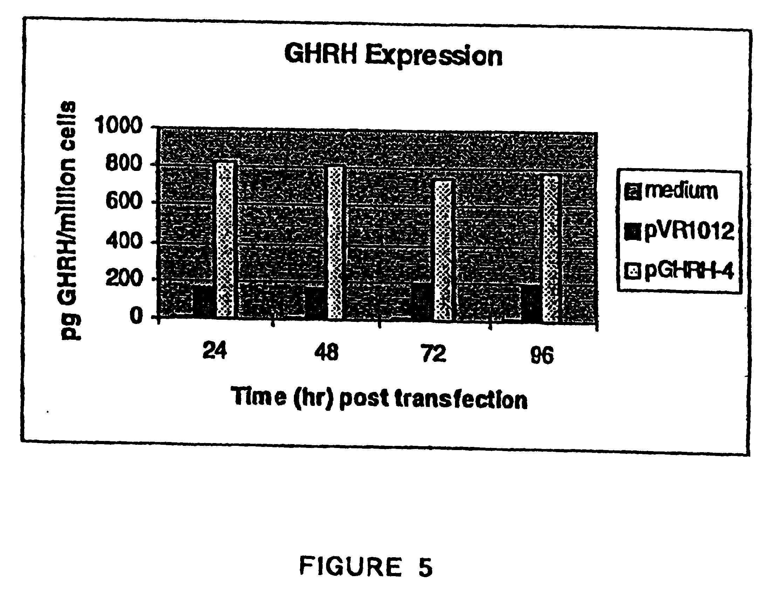

In order to assess the expression level of GHRH from pGHRH-4, this plasmid or pVR1012 was transfected into C2C12 mouse myoblasts using the fugene reagent according to the manufacturer=s recommendations (Boehringer Ingelheim). Supernatant harvested from transfected and non-transfected cells at various time points were assayed for the presence of GHRH using a commerically available radioimmune assay kit (Pennisula Laboratories). The results depicted in Figure 5 indicate that GHRH production could be detected 24 hours post-transfection.

### 17. THE EFFECT OF GH PLASMID INJECTION ON SWINE GROWTH

In order to evaluate the effect of a single injection of plasmids containing GH gene on swine growth, experiments addressing the effect GH treatment on swine of different ages were carried out according to the experimental design described below.

### MATERIALS & METHODS

Thirty six 3-week old (weaned) cross-bred (Yorkshire X landrace) pigs of mixed sex were brought into experimental barns and maintained for an acclimation period of 3 weeks. Animals were kept in pens (2/pen) according to treatment group. Animals were fed daily a non-medicated commercial pig diet ad libitum (16% protein pellet until approximately 45 Kg body weight and then a 14% protein pellet until slaughter) and fresh water was available ad libitum. Animals were randomized into one of four treatment groups (A-D; table 1) according to weight, sex and litter. Eight animals in group A were injected once with plasmids containing GH gene at 6 weeks of age. Controls (10 animals; group C) were injected with blank plasmid vector when they were also 6 weeks of age. Eight animals in group B were injected once with plasmids containing GH gene at 13 weeks of age. Controls (group D; 10 animals) were injected with blank plasmid vector when they were also 13 weeks of age. Food consumption was recorded daily for each pen and animals were weighed on two consecutive days each week until slaughter. Loin eye area and back fat were quantified at slaughter.

**Table 1**

| **Experimental Design** | | | | |
|---|---|---|---|---|
| Group | Number of Animals | Plasmid dose | Age at time of injection | Weight at time of injection |
| A | 8 | 4.6 mg | 6 weeks | 12.4 |
| B | 8 | 4.6 mg | 13 weeks | 4.2 |
| C | 10 | Placebo | 6 weeks | 13.8 |
| D | 10 | Placebo | 13 weeks | 41.9 |

### RESULTS

As shown in table 2, treatment of 6 week-old pigs with plasmids encoding GH gene results in enhanced performance. This is evident by the 5% increase in weight gain and 5.1% increase in Average daily gain (ADG) achieved in GH injected animals versus placebo injected pigs. Moreover, the data shows that GH plasmid injection resulted in an improvement of 3.3 % in feed efficiency (ratio of feed consumed relative to weight gain) as well as an increase of 7.2% in loin eye area of GH plasmid injected animals.

The data in table 3 shows that treatment of 13 week-old pigs with plasmids encoding GH gene results in an even higher magnitude of increase in animal performance relative to age placebo injected controls than treatment of 6 week-old pigs. This is evident by the 9.5% increase in weight gain and 9.3% increase in Average daily gain (ADG) achieved in GH injected animals versus placebo injected pigs. Moreover, the data shows that GH plasmid injection resulted in an improvement of 6.7 % in feed efficiency (ratio of feed consumed relative to weight gain) as well as an increase of 6.8% in loin eye area of GH plasmid injected animals. Thus treatment of animals with plasmids containing GH gene results in enhanced animal growth and performance.

**Table 2**

| **The effect of GH plasmid administration of weight gain** | | | |
|---|---|---|---|
| Group | A | C | % Improvement |
| Weight at injection time (Kg) | 12.4 | 13.8 | |
| Weight gain^{a} | 77.7 | 73.8 | 5.0% |
| ADG^{b} | .79 | .75 | 5.1% |
| total feed intake/pen^{c} | 473.3 | 457.6 | 3.4% |
| Feed intake/pen/day^{d} | 4.8 | 4.7 | 2.1% |
| feed to gain^{e} | 3.03 | 3.13 | 3.3% |
| Loin eye area at Slaughter (Cm²) | 39.51 | 36.83 | 7.2% |

| | | | |
|---|---|---|---|
| a→e = from time of injection to time of slaughter | | | |

**Table 3**

| **The effect of GH plasmid administration of weight gain** | | | |
|---|---|---|---|
| Group | B | D | % Improvement |
| Weight at injection time (Kg) | 42.0 | 41.9 | N/A |
| Weight gain^{a} | 52.1 | 47.6 | 9.5% |
| ADG^{b} | 1.06 | .97 | 9.3% |
| total feed intake/pen^{c} | 309.6 | 300.7 | 3.0% |
| Feed intake/pen/day^{d} | 6.3 | 6.1 | 3.3% |
| feed to gain^{e} | 3.0 | 3.2 | 6.7% |
| Loin eye area at Slaughter (Cm²) | 41.61 | 38.96 | 6.8% |

| | | | |
|---|---|---|---|
| a→e = from time of injection to time of slaughter | | | |

A number of references have been cited and the entire disclosures of which are incorporated herein by reference.

The present invention is not to be limited in scope by the specific embodiments described which are intended as single illustrations of individual aspects of the invention, and functionally equivalent methods and components are within the scope of the invention. Indeed various modifications of the invention, in addition to those shown and described herein will become apparent to those skilled in the art from the foregoing description and accompanying drawings. Such modifications are intended to fall within the scope of the appended claims.

SEQUENCE LISTING

<110> PFIZER PRODUCTS INC.

<120> GROWTH HORMONE AND GROWTH HORMONE RELEASING HORMONE
      COMPOSITIONS

<130> PC10525

<140>
<141>

<160> 67

<170> PatentIn Ver. 2.1

<210> 1
<211> 246
<212> DNA
<213> Artificial Sequence

<220>
<223> Description of Artificial Sequence:
      oligonucleotide

<400> 1
ggatccgcca ccatgccact ctgggtgttc ttctttgtga tcctcaccct cagcaacagc 60
tcccactgct ccccacctcc ccctttgacc ctcaggatgc ggcggtatgc agatgccatc 120
ttcaccaaca gctaccggaa ggtgctgggc cagctgtccg cccgcaagct gctccaggac 180
atcatgagca ggcagcaggg agagagaaac caagagcaag gagcaagggt gcggctttga 240
agatct                                                            246


<210> 2
<211> 75
<212> PRT
<213> Artificial Sequence

<220>
<223> Description of Artificial Sequence:  GHRH protein

<400> 2
Met Pro Leu Trp Val Phe Phe Phe Val Ile Leu Thr Leu Ser Asn Ser
  1               5                  10                  15

Ser His Cys Ser Pro Pro Pro Pro Leu Thr Leu Arg Met Arg Arg Tyr
             20                  25                  30

Ala Asp Ala Ile Phe Thr Asn Ser Tyr Arg Lys Val Leu Gly Gln Leu
        35                  40                  45

Ser Ala Arg Lys Leu Leu Gln Asp Ile Met Ser Arg Gln Gln Gly Glu
    50                  55                  60

Arg Asn Gln Glu Gln Gly Ala Arg Val Arg Leu
65                  70                  75

<210> 3
<211> 685
<212> DNA
<213> Artificial Sequence

<220>
<223> Description of Artificial Sequence:
      obligonucleotide

<400> 3
ggtaccatcg ctggggagct gggggagggg tcgccttcct gccctaccca ggactccggg 60
tgcgaccgct cctctatctc tccagcccac caccactcca ccacttggac acgtctccct 120
cctccctgga gtcgctctag agggtttggg ggtctgagta aagaacccga agtagggata 180
cagtgtggcg gcaccttcca gaggccccgg gcgcagggta gaccggggcg gggcggcccg 240
cggacaggtg cagccccagg cgcaggcgca ctcgcgcctc ccggcgcagg cggtgaacct 300
cgccccaccc cagcccctcc ggggggcagc tgggccgggt cgggaggggc ccaccagccc 360
gggagacact ccatatacgg ccaggcccgc tttacctggg ctccggccag gccgctcctt 420
ctttggtcag cacaggggac ccgggcgggg gcccaggccg ctaacccgcc gggggagggg 480
gctccagtgc ccaacaccca aatatggctc gagaagggga gcgacattcc agtgaggcgg 540
ctcgggggga gaacccgcgg gctatataaa acctgagcgt ggggaccagc ggccaccgca 600
gcggacagcg ccgagagaag cctcgcttcc ctcccgcggc gaccagggcc ccagccggag 660
agcagcaggt gtagccacca agctt                                       685

<210> 4
<211> 30
<212> DNA
<213> Artificial Sequence

<220>
<223> Description of Artificial Sequence: primer

<400> 4
aatcccaagc ttgccaccat gccactctgg                                  30

<210> 5
<211> 30

<212> DNA
<213> Artificial Sequence

<220>
<223> Description of Artificial Sequence:  Primer

<400> 5
tattgctcta gatcaaagcc gcacccttgc                                        30


<210> 6
<211> 39
<212> DNA
<213> Artificial Sequence

<220>
<223> Description of Artificial Sequence:  Primer

<400> 6
cgggtaccat cgctggggga gctggggcag gggtcgcct                              39


<210> 7
<211> 40
<212> DNA
<213> Artificial Sequence

<220>
<223> Description of Artificial Sequence:  Primer

<400> 7
cccgcttggt ggctacacct gctgctctcc ggctggggcc                             40


<210> 8
<211> 2014
<212> DNA
<213> Artificial Sequence

<220>
<223> Description of Artificial Sequence:  SK 2014

<400> 8
ggtaccgcta taggagagaa aagagctgca ctgagcaccc tccttcccct ttaaatgtca 60
acagattagg agtcagtgaa tgacagcaca cctcttgcta ccttagagac caaaatttaa 120
gctactcccc ttaagctata gctagagtgc acctgccagt gtctttagtc cccactgatg 180
gaacaggacc caaggtattg aagatggaac atagttattc attcatcctc taatttaaaa 240
agctggatat gctgtacagc agaaattgac ggaacaatgt aaatcaacta taacagaaga 300

aataaaaacc tggggggaaa gaagctgact atgaaacccc aggagctttc tacatgggcc 360
tggactcacc aaactcttta ttttgtaatg gacttctgac atttttagga agggctgtcc 420
tgatgtgggc tatagaagag ggtttcacat gcttcttcaa gaggacccac actgtcccag 480
ttgctgagtc ccaccaccag atgctagtgg cagctatttg gggaacactt aggcactaca 540
aaaaaatgag tgattccatt ctggctcaca ccatatccct gatgtacccc ttaaagcatg 600
tcactgagtt catcacagaa aattgtttcc cctgtgcctt ccacaacaag gttagagctg 660
tccttggggc caggggaagg gggcagggag tgagaagcac caactggata acctcctctg 720
acccccactc caccttacca taagtagatc caaatccttc tagaaaatta ggaaggcata 780
tccccatata tcagcgatat aaatagaact gcttcagcgc tctggtagac ggtgactctc 840
caaggtggac tgggaggcag cctggccttg gctgggcatc gtcctctaaa tagaaagatg 900
aacttgttca gcctttccag aaggaaaact gctgcccagc ctacagtgca acgtccttgt 960
cttccatctg gaggaagcac gggtgacata tcatctagta agggcacctc tctgtttcca 1020
cctccaggtc gaggggtgtg acccttactt ctcagcctca agggagggac actcaacycc 1080
ccaaaaagac atgagggcgc tcagctcggc ccaccgcacc ccggaccgga gccgtcaccc 1140
cccgaaattc actcccttca caagccccca agcgcgttct ctggtgcgga ctgctccggg 1200
gccctggctt tgtgcccagc gttgtcagag ccaccgccct gagcctgtcc ccgggagccc 1260
cgcgcctcct cccaccgctc crctctcgcg ccccgcggcc agttgtctgc cccgagacag 1320
ctgcgcgccc tcccgctgcc ggtggccctc tccggtgggg gtggggaccg acagggtcag 1380
ccctccggat ccggggcgct ccgggtagcg gggagaagtg atcgctgggg agctggggga 1440
ggggtcgcct tcctgcccta cccaggactc cgggtgcgac cgctcctcta tctctccagc 1500
ccaccaccac tccaccactt ggacacgtct ccctcctccc tggagtcgct ctagagggtt 1560
tgggggtctg agtaaagaac ccgaagtagg gatacagtgt ggcggcacct tccagaggcc 1620
ccgggcgcag ggtagaccgg ggcggggcgg cccgcggaca ggtgcagccc caggcgcagg 1680
cgcactcgcg cctcccggcg caggcggtga acctcgcccc accccagccc ctccgggggg 1740
cagctgggcc gggtcgggag ggcccaccca gcccgggaga cactccatat acggccaggc 1800
ccgctttacc tgggctccgg ccaggccgct ccttctttgg tcagcacagg ggacccgggc 1860
gggggcccag gccgctaacc cgccggggga gggggctcca gtgcccaaca cccaaatatg 1920
gctcgagaag gggagcgaca ttccagtgag gcggctcggg gggagaaccc gcgggctata 1980
taaaacctga gcgtggggac cagcggccaa gctt 2014

<210> 9
<211> 30
<212> DNA
<213> Artificial Sequence

<220>
<223> Description of Artificial Sequence: Primer

<400> 9
cccaagcttg gccgctggtc cccacgctca 30

<210> 10
<211> 38
<212> DNA
<213> Artificial Sequence

<220>
<223> Description of Artificial Sequence: Primer

<400> 10
caggtaccgc tataggagac aaaagagtgc actgagca 38

<210> 11
<211> 46
<212> DNA
<213> Artificial Sequence

<220>
<223> Description of Artificial Sequence: Primer

<400> 11
agatatcccg gccgctctag accaggcccc tggatccgcc accatg 46

<210> 12
<211> 44
<212> DNA
<213> Artificial Sequence

<220>
<223> Description of Artificial Sequence: Primer

<400> 12
gaagatctct acctgctcat gatgtcctgg agcagcttgc gggc 44

<210> 13
<211> 20
<212> DNA
<213> Artificial Sequence

<220>
<223> Description of Artificial Sequence: Primer

<400> 13
gtcattccga gattcggata 20

<210> 14
<211> 40
<212> DNA
<213> Artificial Sequence

<220>
<223> Description of Artificial Sequence: Primer

<400> 14
gtcattccga gattcggata cacaggatcc gccaccatcc 40


<210> 15
<211> 40
<212> DNA
<213> Artificial Sequence

<220>
<223> Description of Artificial Sequence: Primer

<400> 15
cactctgggt gttcttcttt gtgatcctca ccctcagcaa 40


<210> 16
<211> 40
<212> DNA
<213> Artificial Sequence

<220>
<223> Description of Artificial Sequence: Primer

<400> 16
cagctcccac tgctccccac ctcccccttt gaccctcagg 40


<210> 17
<211> 40
<212> DNA
<213> Artificial Sequence

<220>
<223> Description of Artificial Sequence: Primer

<400> 17
atgcggcggt attatgcaga tgccatcttc accaacagct 40


<210> 18
<211> 40
<212> DNA
<213> Artificial Sequence

<220>
<223> Description of Artificial Sequence:  Primer

<400> 18
accggaaggt gctgggccag ctgtccgccc gcaagctcct                              40


<210> 19
<211> 40
<212> DNA
<213> Artificial Sequence

<220>
<223> Description of Artificial Sequence:  Primer

<400> 19
ccaggacatc atgagcaggt agagatctga taagcgttat                              40


<210> 20
<211> 20
<212> DNA
<213> Artificial Sequence

<220>
<223> Description of Artificial Sequence:  Primer

<400> 20
ataacgctta tcagatctct                                                    20


<210> 21
<211> 40
<212> DNA
<213> Artificial Sequence

<220>
<223> Description of Artificial Sequence:  Primer

<400> 21
acctgctcat gatgtcctgg accagcttgc gggcggacag                              40


<210> 22
<211> 40
<212> DNA
<213> Artificial Sequence

<220>
<223> Description of Artificial Sequence: Primer

<400> 22
ctggcccagc accttccggt agctgttggt gaagatggca 40

<210> 23
<211> 40
<212> DNA
<213> Artificial Sequence

<220>
<223> Description of Artificial Sequence: Primer

<400> 23
tctgcataat accgccgcat cctgagggtc aaacccccag 40

<210> 24
<211> 40
<212> DNA
<213> Artificial Sequence

<220>
<223> Description of Artificial Sequence: Primer

<400> 24
gtggggagca gtgggagctg ttgctgaggg tgaggatcac 40

<210> 25
<211> 40
<212> DNA
<213> Artificial Sequence

<220>
<223> Description of Artificial Sequence: Primer

<400> 25
gtggggagca gtgggagctg ttgctgaggg tcaggatcac 40

<210> 26
<211> 30
<212> DNA
<213> Artificial Sequence

<220>
<223> Description of Artificial Sequence:  Primer

<400> 26
gagctcaagc ttgccaccat gccactctgg                                        30


<210> 27
<211> 28
<212> DNA
<213> Artificial Sequence

<220>
<223> Description of Artificial Sequence:  Primer

<400> 27
aagatctaga ctacctgctc atgatgtc                                          28


<210> 28
<211> 30
<212> DNA
<213> Artificial Sequence

<220>
<223> Description of Artificial Sequence:  Primer

<400> 28
gagctcaagc ttgccaccat gccactctgg                                        30


<210> 29
<211> 28
<212> DNA
<213> Artificial Sequence

<220>
<223> Description of Artificial Sequence:  Primer

<400> 29
aagatctaga ctacctgctc atgatgtc                                          28


<210> 30
<211> 20
<212> DNA
<213> Artificial Sequence

<220>
<223> Description of Artificial Sequence:  Primer

<400> 30
gtcattccga gattcggata                                                    20


<210> 31
<211> 40
<212> DNA
<213> Artificial Sequence

<220>
<223> Description of Artificial Sequence:  Primer

<400> 31
gtcattccga gattcggata cacaggatcc gccaccatcc                              40


<210> 32
<211> 40
<212> DNA
<213> Artificial Sequence

<220>
<223> Description of Artificial Sequence:  Primer

<400> 32
cactctgggt gttcttcttt gtgatcctca ccctcagcaa                              40


<210> 33
<211> 39
<212> DNA
<213> Artificial Sequence

<220>
<223> Description of Artificial Sequence:  Primer

<400> 33
cagctccact gctccccacc tccccctttg accctcagg                               39


<210> 34
<211> 40
<212> DNA
<213> Artificial Sequence

<220>
<223> Description of Artificial Sequence: Primer

<400> 34
atgcggcggt attatgcaga tcccatcttc accaacagct 40

<210> 35
<211> 40
<212> DNA
<213> Artificial Sequence

<220>
<223> Description of Artificial Sequence: Primer

<400> 35
accggaaggt gctggcccag ctgtccgccc gcaaggccct 40

<210> 36
<211> 40
<212> DNA
<213> Artificial Sequence

<220>
<223> Description of Artificial Sequence: Primer

<400> 36
ccaggacatc atgagcaggt agagatctga taagcgttat 40

<210> 37
<211> 20
<212> DNA
<213> Artificial Sequence

<220>
<223> Description of Artificial Sequence: Primer

<400> 37
ataacgctta tcagatctct 20

<210> 38
<211> 40
<212> DNA
<213> Artificial Sequence

<220>
<223> Description of Artificial Sequence: Primer

<400> 38
acctgctcat gatgtcctgg agggccttgc gggcccacag 40

<210> 39
<211> 40
<212> DNA
<213> Artificial Sequence

<220>
<223> Description of Artificial Sequence: Primer

<400> 39
ctgggccagc accttccggt acctgttggt gaagatggca 40

<210> 40
<211> 40
<212> DNA
<213> Artificial Sequence

<220>
<223> Description of Artificial Sequence: Primer

<400> 40
   tctccataat accgccgcat cctgagggtc aaagggggag 40

<210> 41
<211> 40
<212> DNA
<213> Artificial Sequence

<220>
<223> Description of Artificial Sequence: Primer

<400> 41
gtggggagca gtgggagctg ttgctgaggg tgaggatcac 40

<210> 42
<211> 40
<212> DNA
<213> Artificial Sequence

<220>
<223> Description of Artificial Sequence: Primer

<400> 42
gtggggagca gtgggagctc ttgctgaggg tgaggatcac 40

<210> 43
<211> 25
<212> DNA
<213> Artificial Sequence

<220>
<223> Description of Artificial Sequence: Primer

<400> 43
ctagaaggca cagctgcttt ccacg 25

<210> 44
<211> 25
<212> DNA
<213> Artificial Sequence

<220>
<223> Description of Artificial Sequence: Primer

<400> 44
atggctgcag gcccccggac ctctg 25

<210> 45
<211> 27
<212> DNA
<213> Artificial Sequence

<220>
<223> Description of Artificial Sequence: Primer

<400> 45
aaagatatca tggctgcagg cccccgg 27

<210> 46
<211> 27
<212> DNA
<213> Artificial Sequence

<220>
<223> Description of Artificial Sequence:  Primer

<400> 46
aaaagatctc tagaaggcac agctgct                                          27


<210> 47
<211> 5185
<212> DNA
<213> Artificial Sequence

<220>
<223> Description of Artificial Sequence:  pGHRH-4
      construct

<400> 47
gctgtgcctt ctagttgcca gccatctgtt gtttgcccct cccccgtgcc ttccttgacc 60
ctggaaggtg ccactcccac tgtcctttcc taataaaatg aggaaattgc atcgcattgt 120
ctgagtaggt gtcattctat tctggggggt ggggtggggc aggacagcaa gggggaggat 180
tgggaagaca atagcaggca tgctggggat gcggtgggct ctatgggtac ccaggtgctg 240
aagaattgac ccggttcctc ctgggccaga aagaagcagg cacatcccct tctctgtgac 300
acaccctgtc cacgcccctg gttcttagtt ccagccccac tcataggaca ctcatagctc 360
aggagggctc cgccttcaat cccacccgct aaagtacttg gagcggtctc tccctccctc 420
atcagcccac caaaccaaac ctagcctcca agagtgggaa gaaattaaag caagataggc 480
tattaagtgc agagggagag aaaatgcctc caacatgtga ggaagtaatg agagaaatca 540
tagaatttct tccgcttcct cgctcactga ctcgctgcgc tcggtcgttc ggctgcggcg 600
agcggtatca gctcactcaa aggcggtaat acggttatcc acagaatcag gggataacgc 660
aggaaagaac atgtgagcaa aaggccagca aaaggccagg aaccgtaaaa aggccgcgtt 720
gctggcgttt ttccataggc tccgcccccc tgacgagcat cacaaaaatc gacgctcaag 780
tcagaggtgg cgaaacccga caggactata aagataccag gcgtttcccc ctggaagctc 840
cctcgtgcgc tctcctgttc cgaccctgcc gcttaccgga tacctgtccg cctttctccc 900
ttcgggaagc gtggcgcttt ctcatagctc acgctgtagg tatctcagtt cggtgtaggt 960
cgttcgctcc aagctgggct gtgtgcacga accccccgtt cagcccgacc gctgcgcctt 1020
atccggtaac tatcgtcttg agtccaaccc ggtaagacac gacttatcgc cactggcagc 1080
agccactggt aacaggatta gcagagcgag gtatgtaggc ggtgctacag agttcttgaa 1140
gtggtggcct aactacggct acactagaag aacagtattt ggtatctgcg ctctgctgaa 1200
gccagttacc ttcggaaaaa gagttggtag ctcttgatcc ggcaaacaaa ccaccgctgg 1260
tagcggtggt ttttttgttt gcaagcagca gattacgcgc agaaaaaaag gatctcaaga 1320
agatcctttg atcttttcta cggggtctga cgctcagtgg aacgaaaact cacgttaagg 1380
gattttggtc atgagattat caaaaaggat cttcacctag atccttttaa attaaaaatg 1440
aagttttaaa tcaatctaaa gtatatatga gtaaacttgg tctgacagtt accaatgctt 1500
aatcagtgag gcacctatct cagcgatctg tctatttcgt tcatccatag ttgcctgact 1560
cggggggggg gggcgctgag gtctgcctcg tgaagaaggt gttgctgact cataccaggc 1620
ctgaatcgcc ccatcatcca gccagaaagt gagggagcca cggttgatga gagctttgtt 1680
gtaggtggac cagttggtga ttttgaactt ttgctttgcc acggaacggt ctgcgttgtc 1740
gggaagatgc gtgatctgat ccttcaactc agcaaaagtt cgatttattc aacaaagccg 1800
ccgtcccgtc aagtcagcgt aatgctctgc cagtgttaca accaattaac caattctgat 1860

tagaaaaact catcgagcat caaatgaaac tgcaatttat tcatatcagg attatcaata 1920
ccatattttt gaaaaagccg tttctgtaat gaaggagaaa actcaccgag gcagttccat 1980
aggatggcaa gatcctggta tcggtctgcg attccgactc gtccaacatc aatacaacct 2040
attaatttcc cctcgtcaaa aataaggtta tcaagtgaga aatcaccatg agtgacgact 2100
gaatccggtg agaatggcaa aagcttatgc atttctttcc agacttgttc aacaggccag 2160
ccattacgct cgtcatcaaa atcactcgca tcaaccaaac cgttattcat tcgtgattgc 2220
gcctgagcga gacgaaatac gcgatcgctg ttaaaaggac aattacaaac aggaatcgaa 2280
tgcaaccggc gcaggaacac tgccagcgca tcaacaatat tttcacctga atcaggatat 2340
tcttctaata cctggaatgc tgttttcccg gggatcgcag tggtgagtaa ccatgcatca 2400
tcaggagtac ggataaaatg cttgatggtc ggaagaggca taaattccgt cagccagttt 2460
agtctgacca tctcatctgt aacatcattg gcaacgctac ctttgccatg tttcagaaac 2520
aactctggcg catcgggctt cccatacaat cgatagattg tcgcacctga ttgcccgaca 2580
ttatcgcgag cccatttata cccatataaa tcagcatcca tgttggaatt taatcgcggc 2640
ctcgagcaag acgtttcccg ttgaatatgg ctcataacac cccttgtatt actgtttatg 2700
taagcagaca gttttattgt tcatgatgat atatttttat cttgtgcaat gtaacatcag 2760
agattttgag acacaacgtg gctttccccc cccccccatt attgaagcat ttatcagggt 2820
tattgtctca tgagcggata catatttgaa tgtatttaga aaaataaaca aataggggtt 2880
ccgcgcacat ttccccgaaa agtgccacct gacgtctaag aaaccattat tatcatgaca 2940
ttaacctata aaaataggcg tatcacgagg ccctttcgtc ctcgcgcgtt tcggtgatga 3000
cggtgaaaac ctctgacaca tgcagctccc ggagacggtc acagcttgtc tgtaagcgga 3060
tgccgggagc agacaagccc gtcagggcgc gtcagcgggt gttggcgggt gtcggggctg 3120
gcttaactat gcggcatcag agcagattgt actgagagtg caccatatgc ggtgtgaaat 3180
accgcacaga tgcgtaagga gaaaataccg catcagattg gctattggcc attgcatacg 3240
ttgtatccat atcataatat gtacatttat attggctcat gtccaacatt accgccatgt 3300
tgacattgat tattgactag ttattaatag taatcaatta cggggtcatt agttcatagc 3360
ccatatatgg agttccgcgt tacataactt acggtaaatg gcccgcctgg ctgaccgccc 3420
aacgaccccc gcccattgac gtcaataatg acgtatgttc ccatagtaac gccaataggg 3480
actttccatt gacgtcaatg ggtggagtat ttacggtaaa ctgcccactt ggcagtacat 3540
caagtgtatc atatgccaag tacgccccct attgacgtca atgacggtaa atggcccgcc 3600
tggcattatg cccagtacat gaccttatgg gactttccta cttggcagta catctacgta 3660
ttagtcatcg ctattaccat ggtgatgcgg ttttggcagt acatcaatgg gcgtggatag 3720
cggtttgact cacggggatt tccaagtctc caccccattg acgtcaatgg gagtttgttt 3780
tggcaccaaa atcaacggga ctttccaaaa tgtcgtaaca actccgcccc attgacgcaa 3840
atgggcggta ggcgtgtacg gtgggaggtc tatataagca gagctcgttt agtgaaccgt 3900
cagatcgcct ggagacgcca tccacgctgt tttgacctcc atagaagaca ccgggaccga 3960
tccagcctcc gcggccggga acggtgcatt ggaacgcgga ttccccgtgc caagagtgac 4020
gtaagtaccg cctatagact ctataggcac acccctttgg ctcttatgca tgctatactg 4080
tttttggctt ggggcctata cacccccgct tccttatgct ataggtgatg gtatagctta 4140
gcctataggt gtgggttatt gaccattatt gaccactccc ctattggtga cgatactttc 4200
cattactaat ccataacatg gctctttgcc acaactatct ctattggcta tatgccaata 4260
ctctgtcctt cagagactga cacggactct gtatttttac aggatggggt cccatttatt 4320
atttacaaat tcacatatac aacaacgccg tcccccgtgc ccgcagtttt tattaaacat 4380
agcgtgggat ctccacgcga atctcgggta cgtgttccgg acatgggctc ttctccggta 4440
gcggcggagc ttccacatcc gagccctggt cccatgcctc cagcggctca tggtcgctcg 4500
gcagctcctt gctcctaaca gtggaggcca gacttaggca cagcacaatg cccaccacca 4560
ccagtgtgcc gcacaaggcc gtggcggtag ggtatgtgtc tgaaaatgag cgtggagatt 4620
gggctcgcac ggctgacgca gatggaagac ttaaggcagc ggcagaagaa gatgcaggca 4680
gctgagttgt tgtattctga taagagtcag aggtaactcc cgttgcggtg ctgttaacgg 4740

tggagggcag tgtagtctga gcagtactcg ttgctgccgc gcgcgccacc agacataata 4800
gctgacagac taacagactg ttcctttcca tgggtctttt ctgcagtcac cgtcgtcgac 4860
acgtgtgatc agatatcgcg gccgctctag accaggcgcc tggatccgcc accatgccac 4920
tctgggtgtt cttctttgtg atcctcaccc tcagcaacag ctcccactgc tccccacctc 4980
cccctttgac cctcaggatg cggcggtatg cagatgccat cttcaccaac agctaccgga 5040
aggtgctggg ccagctgtcc gcccgcaagc tgctccagga catcatgagc aggcagcagg 5100
gagagagaaa ccaagagcaa ggagcaaggg tgcggctttg aagatcttag tagtagtagg 5160
cggccgctct agaggatcca gatct 5185

<210> 48
<211> 3369
<212> DNA
<213> Artificial Sequence

<220>
<223> Description of Artificial Sequence: pGHRH1-44SK
      construct

<400> 48
ggtaccgagc tcttacgcgt gctagcccgg gctcgagatc tgcgatctaa gtaagcttgc 60
caccatgcca ctctgggtgt tcttctttgt gatcctcacc ctcagcaaca gctcccactg 120
ctccccacct ccccctttga ccctcaggat gcggcggtat gcagatgcca tcttcaccaa 180
cagctaccgg aaggtgctgg gccagctgtc cgcccgcaag ctgctccagg acatcatgag 240
caggcagcag ggagagagaa accaagagca aggagcaagg gtgcggcttt gatctagagt 300
cggggcggcc ggccgcttcg agcagacatg ataagataca ttgatgagtt tggacaaacc 360
acaactagaa tgcagtgaaa aaaatgcttt atttgtgaaa tttgtgatgc tattgcttta 420
tttgtaacca ttataagctg caataaacaa gttaacaaca acaattgcat tcattttatg 480
tttcaggttc agggggaggt gtgggaggtt ttttaaagca agtaaaacct ctacaaatgt 540
ggtaaaatcg ataaggatcc gtcgaccgat gcccttgaga gccttcaacc cagtcagctc 600
cttccggtgg gcgcggggca tgactatcgt cgccgcactt atgactgtct tctttatcat 660
gcaactcgta ggacaggtgc cggcagcgct cttccgcttc ctcgctcact gactcgctgc 720
gctcggtcgt tcggctgcgg cgagcggtat cagctcactc aaaggcggta atacggttat 780
ccacagaatc aggggataac gcaggaaaga acatgtgagc aaaaggccag caaaaggcca 840
ggaaccgtaa aaaggccgcg ttgctggcgt ttttccatag gctccgcccc cctgacgagc 900
atcacaaaaa tcgacgctca agtcagaggt ggcgaaaccc gacaggacta taaagatacc 960
aggcgtttcc ccctggaagc tccctcgtgc gctctcctgt tccgaccctg ccgcttaccg 1020
gatacctgtc cgcctttctc ccttcgggaa gcgtggcgct ttctcaatgc tcacgctgta 1080
ggtatctcag ttcggtgtag gtcgttcgct ccaagctggg ctgtgtgcac gaaccccccg 1140
ttcagcccga ccgctgcgcc ttatccggta actatcgtct tgagtccaac ccggtaagac 1200
acgacttatc gccactggca gcagccactg gtaacaggat tagcagagcg aggtatgtag 1260
gcggtgctac agagttcttg aagtggtggc ctaactacgg ctacactaga aggacagtat 1320
ttggtatctg cgctctgctg aagccagtta ccttcggaaa aagagttggt agctcttgat 1380
ccggcaaaca aaccaccgct ggtagcggtg gtttttttgt ttgcaagcag cagattacgc 1440
gcagaaaaaa aggatctcaa gaagatcctt tgatcttttc tacggggtct gacgctcagt 1500
ggaacgaaaa ctcacgttaa gggattttgg tcatgagatt atcaaaaagg atcttcacct 1560
agatcctttt aaattaaaaa tgaagtttta aatcaatcta aagtatatat gagtaaactt 1620
ggtctgacag ttaccaatgc ttaatcagtg aggcacctat ctcagcgatc tgtctatttc 1680

gttcatccat agttgcctga ctccccgtcg tgtagataac tacgatacgg gagggcttac 1740
catctggccc cagtgctgca atgataccgc gagacccacg ctcaccggct ccagatttat 1800
cagcaataaa ccagccagcc ggaagggccg agcgcagaag tggtcctgca actttatccg 1860
cctccatcca gtctattaat tgttgccggg aagctagagt aagtagttcg ccagttaata 1920
gtttgcgcaa cgttgttgcc attgctacag gcatcgtggt gtcacgctcg tcgtttggta 1980
tggcttcatt cagctccggt tcccaacgat caaggcgagt tacatgatcc cccatgttgt 2040
gcaaaaaagc ggttagctcc ttcggtcctc cgatcgttgt cagaagtaag ttggccgcag 2100
tgttatcact catggttatg gcagcactgc ataattctct tactgtcatg ccatccgtaa 2160
gatgcttttc tgtgactggt gagtactcaa ccaagtcatt ctgagaatag tgtatgcggc 2220
gaccgagttg ctcttgcccg gcgtcaatac gggataatac cgcgccacat agcagaactt 2280
taaaagtgct catcattgga aaacgttctt cggggcgaaa actctcaagg atcttaccgc 2340
tgttgagatc cagttcgatg taacccactc gtgcacccaa ctgatcttca gcatctttta 2400
ctttcaccag cgtttctggg tgagcaaaaa caggaaggca aaatgccgca aaaaagggaa 2460
taagggcgac acggaaatgt tgaatactca tactcttcct ttttcaatat tattgaagca 2520
tttatcaggg ttattgtctc atgagcggat acatatttga atgtatttag aaaaataaac 2580
aaataggggt tccgcgcaca tttccccgaa aagtgccacc tgacgcgccc tgtagcggcg 2640
cattaagcgc ggcgggtgtg gtggttacgc gcagcgtgac cgctacactt gccagcgccc 2700
tagcgcccgc tcctttcgct ttcttccctt cctttctcgc cacgttcgcc ggctttcccc 2760
gtcaagctct aaatcggggg ctccctttag ggttccgatt tagtgcttta cggcacctcg 2820
accccaaaaa acttgattag ggtgatggtt cacgtagtgg gccatcgccc tgatagacgg 2880
tttttcgccc tttgacgttg gagtccacgt tctttaatag tggactcttg ttccaaactg 2940
gaacaacact caaccctatc tcggtctatt cttttgattt ataagggatt ttgccgattt 3000
cggcctattg gttaaaaaat gagctgattt aacaaaaatt taacgcgaat tttaacaaaa 3060
tattaacgtt tacaatttcc cattcgccat tcaggctgcg caactgttgg gaagggcgat 3120
cggtgcgggc ctcttcgcta ttacgccagc ccaagctacc atgataagta agtaatatta 3180
aggtacggga ggtacttgga gcggccgcaa taaaatatct ttattttcat tacatctgtg 3240
tgttggtttt ttgtgtgaat cgatagtact aacatacgct ctccatcaaa acaaaacgaa 3300
acaaaacaaa ctagcaaaat aggctgtccc cagtgcaagt gcaggtgcca gaacatttct 3360
ctatcgata 3369

<210> 49
<211> 3976
<212> DNA
<213> Artificial Sequence

<220>
<223> Description of Artificial Sequence:
      pGHRH1-44WTSK685 construct

<400> 49
ggtaccatcg ctggggagct gggggagggg tcgccttcct gccctaccca ggactccggg 60
tgcgaccgct cctctatctc tccagcccac caccactcca ccacttggac acgtctccct 120
cctccctgga gtcgctctag agggtttggg ggtctgagta aagaacccga agtagggata 180
cagtgtggcg gcaccttcca gaggccccgg gcgcagggta gaccgggcgg gggcggcccg 240
cggacaggtg cagccccagg cgcaggcgca ctcgcgcctc ccggcgcagg cggtgaacct 300
cgccccaccc cagcccctcc ggggggcagc tgggccgggt cgggaggggc ccaccagccc 360
gggagacact ccatatacgg ccaggcccgc tttacctggg ctccggccag gccgctcctt 420

ctttggtcag cacaggggac ccgggcgggg gcccaggccg ctaacccgcc gggggagggg 480
gctccagtgc ccaacaccca aatatggctc gagaagggga gcgacattcc agtgaggcgg 540
ctcgggggga gaacccgcgg gctatataaa acctgagcgt ggggaccagc ggccaccgca 600
gcggacagcg ccgagagaag cctcgcttcc ctcccgcggc gaccagggcc ccagccggag 660
agcagcaggt gtagccacca agcttgccac catgccactc tgggtgttct tctttgtgat 720
cctcaccctc agcaacagct cccactgctc cccacctccc cctttgaccc tcaggatgcg 780
gcggtatgca gatgccatct tcaccaacag ctaccggaag gtgctgggcc agctgtccgc 840
ccgcaagctg ctccaggaca tcatgagcag gcagcaggga gagagaaacc aagagcaagg 900
agcaagggtg cggctttgat ctagagtcgg ggcggccggc cgcttcgagc agacatgata 960
agatacattg atgagtttgg acaaaccaca actagaatgc agtgaaaaaa atgctttatt 1020
tgtgaaattt gtgatgctat tgctttattt gtaaccatta taagctgcaa taaacaagtt 1080
aacaacaaca attgcattca ttttatgttt caggttcagg gggaggtgtg ggaggttttt 1140
taaagcaagt aaaacctcta caaatgtggt aaaatcgata aggatccgtc gaccgatgcc 1200
cttgagagcc ttcaacccag tcagctcctt ccggtgggcg cggggcatga ctatcgtcgc 1260
cgcacttatg actgtcttct ttatcatgca actcgtagga caggtgccgg cagcgctctt 1320
ccgcttcctc gctcactgac tcgctgcgct cggtcgttcg gctgcggcga gcggtatcag 1380
ctcactcaaa ggcggtaata cggttatcca cagaatcagg ggataacgca ggaaagaaca 1440
tgtgagcaaa aggccagcaa aaggccagga accgtaaaaa ggccgcgttg ctggcgtttt 1500
tccataggct ccgcccccct gacgagcatc acaaaaatcg acgctcaagt cagaggtggc 1560
gaaacccgac aggactataa agataccagg cgtttccccc tggaagctcc ctcgtgcgct 1620
ctcctgttcc gaccctgccg cttaccggat acctgtccgc ctttctccct tcgggaagcg 1680
tggcgctttc tcaatgctca cgctgtaggt atctcagttc ggtgtaggtc gttcgctcca 1740
agctgggctg tgtgcacgaa ccccccgttc agcccgaccg ctgcgcctta tccggtaact 1800
atcgtcttga gtccaacccg gtaagacacg acttatcgcc actggcagca gccactggta 1860
acaggattag cagagcgagg tatgtaggcg gtgctacaga gttcttgaag tggtggccta 1920
actacggcta cactagaagg acagtatttg gtatctgcgc tctgctgaag ccagttacct 1980
tcggaaaaag agttggtagc tcttgatccg gcaaacaaac caccgctggt agcggtggtt 2040
tttttgtttg caagcagcag attacgcgca gaaaaaaagg atctcaagaa gatcctttga 2100
tcttttctac ggggtctgac gctcagtgga acgaaaactc acgttaaggg attttggtca 2160
tgagattatc aaaaaggatc ttcacctaga tccttttaaa ttaaaaatga agttttaaat 2220
caatctaaag tatatatgag taaacttggt ctgacagtta ccaatgctta atcagtgagg 2280
cacctatctc agcgatctgt ctatttcgtt catccatagt tgcctgactc cccgtcgtgt 2340
agataactac gatacgggag ggcttaccat ctggccccag tgctgcaatg ataccgcgag 2400
acccacgctc accggctcca gatttatcag caataaacca gccagccgga agggccgagc 2460
gcagaagtgg tcctgcaact ttatccgcct ccatccagtc tattaattgt tgccgggaag 2520
ctagagtaag tagttcgcca gttaatagtt tgcgcaacgt tgttgccatt gctacaggca 2580
tcgtggtgtc acgctcgtcg tttggtatgg cttcattcag ctccggttcc caacgatcaa 2640
ggcgagttac atgatccccc atgttgtgca aaaaagcggt tagctccttc ggtcctccga 2700
tcgttgtcag aagtaagttg gccgcagtgt tatcactcat ggttatggca gcactgcata 2760
attctcttac tgtcatgcca tccgtaagat gcttttctgt gactggtgag tactcaacca 2820
agtcattctg agaatagtgt atgcggcgac cgagttgctc ttgcccggcg tcaatacggg 2880
ataataccgc gccacatagc agaactttaa aagtgctcat cattggaaaa cgttcttcgg 2940
ggcgaaaact ctcaaggatc ttaccgctgt tgagatccag ttcgatgtaa cccactcgtg 3000
cacccaactg atcttcagca tcttttactt tcaccagcgt ttctgggtga gcaaaaacag 3060
gaaggcaaaa tgccgcaaaa aagggaataa gggcgacacg gaaatgttga atactcatac 3120
tcttcctttt tcaatattat tgaagcattt atcagggtta ttgtctcatg agcggataca 3180
tatttgaatg tatttagaaa aataaacaaa taggggttcc gcgcacattt ccccgaaaag 3240
tgccacctga cgcgccctgt agcggcgcat taagcgcggc gggtgtggtg gttacgcgca 3300

gcgtgaccgc tacacttgcc agcgccctag cgcccgctcc tttcgctttc ttcccttcct 3360
ttctcgccac gttcgccggc tttccccgtc aagctctaaa tcgggggctc cctttagggt 3420
tccgatttag tgctttacgg cacctcgacc ccaaaaaact tgattagggt gatggttcac 3480
gtagtgggcc atcgccctga tagacggttt ttcgcccttt gacgttggag tccacgttct 3540
ttaatagtgg actcttgttc caaactggaa caacactcaa ccctatctcg gtctattctt 3600
ttgatttata agggattttg ccgatttcgg cctattggtt aaaaaatgag ctgatttaac 3660
aaaaatttaa cgcgaatttt aacaaaatat taacgtttac aatttcccat tcgccattca 3720
ggctgcgcaa ctgttgggaa gggcgatcgg tgcgggcctc ttcgctatta cgccagccca 3780
agctaccatg ataagtaagt aatattaagg tacgggaggt acttggagcg gccgcaataa 3840
aatatcttta ttttcattac atctgtgtgt tggttttttg tgtgaatcga tagtactaac 3900
atacgctctc catcaaaaca aaacgaaaca aaacaaacta gcaaaatagg ctgtccccag 3960
tgcaagtgca ggtgcc 3976

<210> 50
<211> 5325
<212> DNA
<213> Artificial Sequence

<220>
<223> Description of Artificial Sequence:
      pGHRH1-44WTSK2014 construct

<400> 50
ggtaccgcta taggagagaa aagagctgca ctgagcaccc tccttcccct ttaaatgtca 60
acagattagg agtcagtgaa tgacagcaca cctcttgcta ccttagagac caaaatttaa 120
gctactcccc ttaagctata gctagagtgc acctgccagt gtctttagtc cccactgatg 180
gaacaggacc caaggtattg aagatggaac atagttattc attcatcctc taatttaaaa 240
agctggatat gctgtacagc agaaattgac ggaacaatgt aaatcaacta taacagaaga 300
aataaaaacc tggggggaaa gaagctgact atgaaacccc aggagctttc tacatgggcc 360
tggactcacc aaactcttta ttttgtaatg gacttctgac atttttagga agggctgtcc 420
tgatgtgggc tatagaagag ggtttcacat gcttcttcaa gaggacccac actgtcccag 480
ttgctgagtc ccaccaccag atgctagtgg cagctatttg gggaacactt aggcactaca 540
aaaaaatgag tgattccatt ctggctcaca ccatatccct gatgtacccc ttaaagcatg 600
tcactgagtt catcacagaa aattgtttcc cctgtgcctt ccacaacaag gttagagctg 660
tccttggggc caggggaagg gggcagggag tgagaagcac caactggata acctcctctg 720
acccccactc caccttacca taagtagatc caaatccttc tagaaaatta ggaaggcata 780
tccccatata tcagcgatat aaatagaact gcttcagcgc tctggtagac ggtgactctc 840
caaggtggac tgggaggcag cctggccttg gctgggcatc gtcctctaaa tagaaagatg 900
aacttgttca gcctttccag aaggaaaact gctgcccagc ctacagtgca acgtccttgt 960
cttccatctg gaggaagcac gggtgacata tcatctagta agggcacctc tctgtttcca 1020
cctccaggtc gaggggtgtg acccttactt ctcagcctca agggagggac actcaacccc 1080
ccaaaaagac atgagggcgc tcagctcggc ccaccgcacc ccggaccgga gccgtcaccc 1140
cccgaaattc actcccttca caagccccca agcgcgttct ctggtgcgga ctgctccggg 1200
gccctggctt tgtgcccagc gttgtcagag ccaccgccct gagcctgtcc ccgggagccc 1260
cgcgcctcct cccaccgctc cgctctcgcg ccccgcggcc agttgtctgc cccgagacag 1320
ctgcgcgccc tcccgctgcc ggtggccctc tccggtgggg gtggggaccg acagggtcag 1380
ccctccggat ccggggcgct ccgggtagcg gggagaagtg atcgctgggg agctggggga 1440

ggggtcgcct tcctgcccta cccaggactc cgggtgcgac cgctcctcta tctctccagc 1500
ccaccaccac tccaccactt ggacacgtct ccctcctccc tggagtcgct ctagagggtt 1560
tgggggtctg agtaaagaac ccgaagtagg gatacagtgt ggcggcacct tccagaggcc 1620
ccgggcgcag ggtagaccgg ggcggggcgg cccgcggaca ggtgcagccc caggcgcagg 1680
cgcactcgcg cctcccggcg caggcggtga acctcgcccc accccagccc ctccgggggg 1740
cagctgggcc gggtcgggag gggcccacca gcccgggaga cactccatat acggccaggc 1800
ccgctttacc tgggctccgg ccaggccgct ccttctttgg tcagcacagg ggacccgggc 1860
gggggcccag gccgctaacc cgccggggga gggggctcca gtgcccaaca cccaaatatg 1920
gctcgagaag gggagcgaca ttccagtgag gcggctcggg gggagaaccc gcgggctata 1980
taaaacctga gcgtggggac cagcggccaa gcttgccacc atgccactct gggtgttctt 2040
ctttgtgatc ctcaccctca gcaacagctc ccactgctcc ccacctcccc ctttgaccct 2100
caggatgcgg cggtatgcag atgccatctt caccaacagc taccggaagg tgctgggcca 2160
gctgtccgcc cgcaagctgc tccaggacat catgagcagg cagcagggag agagaaacca 2220
agagcaagga gcaagggtgc ggctttgatc tagagtcggg gcggccggcc gcttcgagca 2280
gacatgataa gatacattga tgagtttgga caaaccacaa ctagaatgca gtgaaaaaaa 2340
tgctttattt gtgaaatttg tgatgctatt gctttatttg taaccattat aagctgcaat 2400
aaacaagtta acaacaacaa ttgcattcat tttatgtttc aggttcaggg ggaggtgtgg 2460
gaggtttttt aaagcaagta aaacctctac aaatgtggta aaatcgataa ggatccgtcg 2520
accgatgccc ttgagagcct tcaacccagt cagctccttc cggtgggcgc ggggcatgac 2580
tatcgtcgcc gcacttatga ctgtcttctt tatcatgcaa ctcgtaggac aggtgccggc 2640
agcgctcttc cgcttcctcg ctcactgact cgctgcgctc ggtcgttcgg ctgcggcgag 2700
cggtatcagc tcactcaaag gcggtaatac ggttatccac agaatcaggg gataacgcag 2760
gaaagaacat gtgagcaaaa ggccagcaaa aggccaggaa ccgtaaaaag gccgcgttgc 2820
tggcgttttt ccataggctc cgcccccctg acgagcatca caaaaatcga cgctcaagtc 2880
agaggtggcg aaacccgaca ggactataaa gataccaggc gtttccccct ggaagctccc 2940
tcgtgcgctc tcctgttccg accctgccgc ttaccggata cctgtccgcc tttctccctt 3000
cgggaagcgt ggcgctttct caatgctcac gctgtaggta tctcagttcg gtgtaggtcg 3060
ttcgctccaa gctgggctgt gtgcacgaac cccccgttca gcccgaccgc tgcgccttat 3120
ccggtaacta tcgtcttgag tccaacccgg taagacacga cttatcgcca ctggcagcag 3180
ccactggtaa caggattagc agagcgaggt atgtaggcgg tgctacagag ttcttgaagt 3240
ggtggcctaa ctacggctac actagaagga cagtatttgg tatctgcgct ctgctgaagc 3300
cagttacctt cggaaaaaga gttggtagct cttgatccgg caaacaaacc accgctggta 3360
gcggtggttt ttttgtttgc aagcagcaga ttacgcgcag aaaaaaagga tctcaagaag 3420
atcctttgat cttttctacg gggtctgacg ctcagtggaa cgaaaactca cgttaaggga 3480
ttttggtcat gagattatca aaaaggatct tcacctagat ccttttaaat taaaaatgaa 3540
gttttaaatc aatctaaagt atatatgagt aaacttggtc tgacagttac caatgcttaa 3600
tcagtgaggc acctatctca gcgatctgtc tatttcgttc atccatagtt gcctgactcc 3660
ccgtcgtgta gataactacg atacgggagg gcttaccatc tggccccagt gctgcaatga 3720
taccgcgaga cccacgctca ccggctccag atttatcagc aataaaccag ccagccggaa 3780
gggccgagcg cagaagtggt cctgcaactt tatccgcctc catccagtct attaattgtt 3840
gccgggaagc tagagtaagt agttcgccag ttaatagttt gcgcaacgtt gttgccattg 3900
ctacaggcat cgtggtgtca cgctcgtcgt ttggtatggc ttcattcagc tccggttccc 3960
aacgatcaag gcgagttaca tgatccccca tgttgtgcaa aaaagcggtt agctccttcg 4020
gtcctccgat cgttgtcaga agtaagttgg ccgcagtgtt atcactcatg gttatggcag 4080
cactgcataa ttctcttact gtcatgccat ccgtaagatg cttttctgtg actggtgagt 4140
actcaaccaa gtcattctga gaatagtgta tgcggcgacc gagttgctct tgcccggcgt 4200
caatacggga taataccgcg ccacatagca gaactttaaa agtgctcatc attggaaaac 4260
gttcttcggg gcgaaaactc tcaaggatct taccgctgtt gagatccagt tcgatgtaac 4320

ccactcgtgc acccaactga tcttcagcat cttttacttt caccagcgtt tctgggtgag 4380
caaaaacagg aaggcaaaat gccgcaaaaa agggaataag ggcgacacgg aaatgttgaa 4440
tactcatact cttccttttt caatattatt gaagcattta tcagggttat tgtctcatga 4500
gcggatacat atttgaatgt atttagaaaa ataaacaaat aggggttccg cgcacatttc 4560
cccgaaaagt gccacctgac gcgccctgta gcggcgcatt aagcgcggcg ggtgtggtgg 4620
ttacgcgcag cgtgaccgct acacttgcca gcgccctagc gcccgctcct ttcgctttct 4680
tcccttcctt tctcgccacg ttcgccggct ttccccgtca agctctaaat cgggggctcc 4740
ctttagggtt ccgatttagt gctttacggc acctcgaccc caaaaaactt gattagggtg 4800
atggttcacg tagtgggcca tcgccctgat agacggtttt tcgccctttg acgttggagt 4860
ccacgttctt taatagtgga ctcttgttcc aaactggaac aacactcaac cctatctcgg 4920
tctattcttt tgatttataa gggattttgc cgatttcggc ctattggtta aaaaatgagc 4980
tgatttaaca aaaatttaac gcgaatttta acaaaatatt aacgtttaca atttcccatt 5040
cgccattcag gctgcgcaac tgttgggaag ggcgatcggt gcgggcctct tcgctattac 5100
gccagcccaa gctaccatga taagtaagta atattaaggt acgggaggta cttggagcgg 5160
ccgcaataaa atatctttat tttcattaca tctgtgtgtt ggttttttgt gtgaatcgat 5220
agtactaaca tacgctctcc atcaaaacaa aacgaaacaa aacaaactag caaaataggc 5280
tgtccccagt gcaagtgcag gtgccagaac atttctctat cgata 5325

<210> 51
<211> 5108
<212> DNA
<213> Artificial Sequence

<220>
<223> Description of Artificial Sequence:
      pGHRH1-29WTCMV construct

<400> 51
gctgtgcctt ctagttgcca gccatctgtt gtttgcccct cccccgtgcc ttccttgacc 60
ctggaaggtg ccactcccac tgtcctttcc taataaaatg aggaaattgc atcgcattgt 120
ctgagtaggt gtcattctat tctggggggt ggggtggggc aggacagcaa gggggaggat 180
tgggaagaca atagcaggca tgctggggat gcggtgggct ctatgggtac ccaggtgctg 240
aagaattgac ccggttcctc ctgggccaga aagaagcagg cacatcccct tctctgtgac 300
acaccctgtc cacgcccctg gttcttagtt ccagccccac tcataggaca ctcatagctc 360
aggagggctc cgccttcaat cccacccgct aaagtacttg gagcggtctc tccctccctc 420
atcagcccac caaaccaaac ctagcctcca agagtgggaa gaaattaaag caagataggc 480
tattaagtgc agagggagag aaaatgcctc caacatgtga ggaagtaatg agagaaatca 540
tagaatttct tccgcttcct cgctcactga ctcgctgcgc tcggtcgttc ggctgcggcg 600
agcggtatca gctcactcaa aggcggtaat acggttatcc acagaatcag gggataacgc 660
aggaaagaac atgtgagcaa aaggccagca aaaggccagg aaccgtaaaa aggccgcgtt 720
gctggcgttt ttccataggc tccgcccccc tgacgagcat cacaaaaatc gacgctcaag 780
tcagaggtgg cgaaacccga caggactata aagataccag gcgtttcccc ctggaagctc 840
cctcgtgcgc tctcctgttc cgaccctgcc gcttaccgga tacctgtccg cctttctccc 900
ttcgggaagc gtggcgcttt ctcatagctc acgctgtagg tatctcagtt cggtgtaggt 960
cgttcgctcc aagctgggct gtgtgcacga accccccgtt cagcccgacc gctgcgcctt 1020
atccggtaac tatcgtcttg agtccaaccc ggtaagacac gacttatcgc cactggcagc 1080
agccactggt aacaggatta gcagagcgag gtatgtaggc ggtgctacag agttcttgaa 1140

gtggtggcct aactacggct acactagaag aacagtattt ggtatctgcg ctctgctgaa 1200
gccagttacc ttcggaaaaa gagttggtag ctcttgatcc ggcaaacaaa ccaccgctgg 1260
tagcggtggt ttttttgttt gcaagcagca gattacgcgc agaaaaaaag gatctcaaga 1320
agatcctttg atcttttcta cggggtctga cgctcagtgg aacgaaaact cacgttaagg 1380
gattttggtc atgagattat caaaaaggat cttcacctag atccttttaa attaaaaatg 1440
aagttttaaa tcaatctaaa gtatatatga gtaaacttgg tctgacagtt accaatgctt 1500
aatcagtgag gcacctatct cagcgatctg tctatttcgt tcatccatag ttgcctgact 1560
cggggggggg gggcgctgag gtctgcctcg tgaagaaggt gttgctgact cataccaggc 1620
ctgaatcgcc ccatcatcca gccagaaagt gagggagcca cggttgatga gagctttgtt 1680
gtaggtggac cagttggtga ttttgaactt ttgctttgcc acggaacggt ctgcgttgtc 1740
gggaagatgc gtgatctgat ccttcaactc agcaaaagtt cgatttattc aacaaagccg 1800
ccgtcccgtc aagtcagcgt aatgctctgc cagtgttaca accaattaac caattctgat 1860
tagaaaaact catcgagcat caaatgaaac tgcaatttat tcatatcagg attatcaata 1920
ccatattttt gaaaaagccg tttctgtaat gaaggagaaa actcaccgag gcagttccat 1980
aggatggcaa gatcctggta tcggtctgcg attccgactc gtccaacatc aatacaacct 2040
attaatttcc cctcgtcaaa aataaggtta tcaagtgaga aatcaccatg agtgacgact 2100
gaatccggtg agaatggcaa aagcttatgc atttctttcc agacttgttc aacaggccag 2160
ccattacgct cgtcatcaaa atcactcgca tcaaccaaac cgttattcat tcgtgattgc 2220
gcctgagcga gacgaaatac gcgatcgctg ttaaaaggac aattacaaac aggaatcgaa 2280
tgcaaccggc gcaggaacac tgccagcgca tcaacaatat tttcacctga atcaggatat 2340
tcttctaata cctggaatgc tgttttcccg gggatcgcag tggtgagtaa ccatgcatca 2400
tcaggagtac ggataaaatg cttgatggtc ggaagaggca taaattccgt cagccagttt 2460
agtctgacca tctcatctgt aacatcattg gcaacgctac ctttgccatg tttcagaaac 2520
aactctggcg catcgggctt cccatacaat cgatagattg tcgcacctga ttgcccgaca 2580
ttatcgcgag cccatttata cccatataaa tcagcatcca tgttggaatt taatcgcggc 2640
ctcgagcaag acgtttcccg ttgaatatgg ctcataacac cccttgtatt actgtttatg 2700
taagcagaca gttttattgt tcatgatgat atatttttat cttgtgcaat gtaacatcag 2760
agattttgag acacaacgtg gctttccccc cccccccatt attgaagcat ttatcagggt 2820
tattgtctca tgagcggata catatttgaa tgtatttaga aaaataaaca aataggggtt 2880
ccgcgcacat ttccccgaaa agtgccacct gacgtctaag aaaccattat tatcatgaca 2940
ttaacctata aaaataggcg tatcacgagg ccctttcgtc ctcgcgcgtt tcggtgatga 3000
cggtgaaaac ctctgacaca tgcagctccc ggagacggtc acagcttgtc tgtaagcgga 3060
tgccgggagc agacaagccc gtcagggcgc gtcagcgggt gttggcgggt gtcggggctg 3120
gcttaactat gcggcatcag agcagattgt actgagagtg caccatatgc ggtgtgaaat 3180
accgcacaga tgcgtaagga gaaaataccg catcagattg gctattggcc attgcatacg 3240
ttgtatccat atcataatat gtacatttat attggctcat gtccaacatt accgccatgt 3300
tgacattgat tattgactag ttattaatag taatcaatta cggggtcatt agttcatagc 3360
ccatatatgg agttccgcgt tacataactt acggtaaatg gcccgcctgg ctgaccgccc 3420
aacgaccccc gcccattgac gtcaataatg acgtatgttc ccatagtaac gccaataggg 3480
actttccatt gacgtcaatg ggtggagtat ttacggtaaa ctgcccactt ggcagtacat 3540
caagtgtatc atatgccaag tacgccccct attgacgtca atgacggtaa atggcccgcc 3600
tggcattatg cccagtacat gaccttatgg gactttccta cttggcagta catctacgta 3660
ttagtcatcg ctattaccat ggtgatgcgg ttttggcagt acatcaatgg gcgtggatag 3720
cggtttgact cacggggatt tccaagtctc caccccattg acgtcaatgg gagtttgttt 3780
tggcaccaaa atcaacggga ctttccaaaa tgtcgtaaca actccgcccc attgacgcaa 3840
atgggcggta ggcgtgtacg gtgggaggtc tatataagca gagctcgttt agtgaaccgt 3900
cagatcgcct ggagacgcca tccacgctgt tttgacctcc atagaagaca ccgggaccga 3960
tccagcctcc gcggccggga acggtgcatt ggaacgcgga ttccccgtgc caagagtgac 4020

gtaagtaccg cctatagact ctataggcac acccctttgg ctcttatgca tgctatactg 4080
tttttggctt ggggcctata cacccccgct tccttatgct ataggtgatg gtatagctta 4140
gcctataggt gtgggttatt gaccattatt gaccactccc ctattggtga cgatactttc 4200
cattactaat ccataacatg gctctttgcc acaactatct ctattggcta tatgccaata 4260
ctctgtcctt cagagactga cacggactct gtatttttac aggatggggt cccatttatt 4320
atttacaaat tcacatatac aacaacgccg tcccccgtgc ccgcagtttt tattaaacat 4380
agcgtgggat ctccacgcga atctcgggta cgtgttccgg acatgggctc ttctccggta 4440
gcggcggagc ttccacatcc gagccctggt cccatgcctc cagcggctca tggtcgctcg 4500
gcagctcctt gctcctaaca gtggaggcca gacttaggca cagcacaatg cccaccacca 4560
ccagtgtgcc gcacaaggcc gtggcggtag ggtatgtgtc tgaaaatgag cgtggagatt 4620
gggctcgcac ggctgacgca gatggaagac ttaaggcagc ggcagaagaa gatgcaggca 4680
gctgagttgt tgtattctga taagagtcag aggtaactcc cgttgcggtg ctgttaacgg 4740
tggagggcag tgtagtctga gcagtactcg ttgctgccgc gcgcgccacc agacataata 4800
gctgacagac taacagactg ttcctttcca tgggtctttt ctgcagtcac cgtcgtcgac 4860
acgtgtgatc agatatcgcg gccgctctag accaggcgcc tggatccgcc accatgccac 4920
tctgggtgtt cttctttgtg atcctcaccc tcagcaacag ctcccactgc tccccacctc 4980
cccctttgac cctcaggatg cggcggtatg cagatgccat cttcaccaac agctaccgga 5040
aggtgctggg ccagctgtcc gcccgcaagc tgctccagga catcatgagc aggtagagat 5100
ccagatct 5108

<210> 52
<211> 5108
<212> DNA
<213> Artificial Sequence

<220>
<223> Description of Artificial Sequence:
      pGHRH1-29YWTCMV construct

<400> 52
gctgtgcctt ctagttgcca gccatctgtt gtttgcccct cccccgtgcc ttccttgacc 60
ctggaaggtg ccactcccac tgtcctttcc taataaaatg aggaaattgc atcgcattgt 120
ctgagtaggt gtcattctat tctggggggt ggggtggggc aggacagcaa gggggaggat 180
tgggaagaca atagcaggca tgctggggat gcggtgggct ctatgggtac ccaggtgctg 240
aagaattgac ccggttcctc ctgggccaga aagaagcagg cacatcccct tctctgtgac 300
acaccctgtc cacgcccctg gttcttagtt ccagccccac tcataggaca ctcatagctc 360
aggagggctc cgccttcaat cccacccgct aaagtacttg gagcggtctc tccctccctc 420
atcagcccac caaaccaaac ctagcctcca agagtgggaa gaaattaaag caagataggc 480
tattaagtgc agagggagag aaaatgcctc caacatgtga ggaagtaatg agagaaatca 540
tagaatttct tccgcttcct cgctcactga ctcgctgcgc tcggtcgttc ggctgcggcg 600
agcggtatca gctcactcaa aggcggtaat acggttatcc acagaatcag gggataacgc 660
aggaaagaac atgtgagcaa aaggccagca aaaggccagg aaccgtaaaa aggccgcgtt 720
gctggcgttt ttccataggc tccgcccccc tgacgagcat cacaaaaatc gacgctcaag 780
tcagaggtgg cgaaacccga caggactata aagataccag gcgtttcccc ctggaagctc 840
cctcgtgcgc tctcctgttc cgaccctgcc gcttaccgga tacctgtccg cctttctccc 900
ttcgggaagc gtggcgcttt ctcatagctc acgctgtagg tatctcagtt cggtgtaggt 960
cgttcgctcc aagctgggct gtgtgcacga accccccgtt cagcccgacc gctgcgcctt 1020

atccggtaac tatcgtcttg agtccaaccc ggtaagacac gacttatcgc cactggcagc 1080
agccactggt aacaggatta gcagagcgag gtatgtaggc ggtgctacag agttcttgaa 1140
gtggtggcct aactacggct acactagaag aacagtattt ggtatctgcg ctctgctgaa 1200
gccagttacc ttcggaaaaa gagttggtag ctcttgatcc ggcaaacaaa ccaccgctgg 1260
tagcggtggt ttttttgttt gcaagcagca gattacgcgc agaaaaaaag gatctcaaga 1320
agatcctttg atcttttcta cggggtctga cgctcagtgg aacgaaaact cacgttaagg 1380
gattttggtc atgagattat caaaaaggat cttcacctag atccttttaa attaaaaatg 1440
aagttttaaa tcaatctaaa gtatatatga gtaaacttgg tctgacagtt accaatgctt 1500
aatcagtgag gcacctatct cagcgatctg tctatttcgt tcatccatag ttgcctgact 1560
cggggggggg gggcgctgag gtctgcctcg tgaagaaggt gttgctgact cataccaggc 1620
ctgaatcgcc ccatcatcca gccagaaagt gagggagcca cggttgatga gagctttgtt 1680
gtaggtggac cagttggtga ttttgaactt ttgctttgcc acggaacggt ctgcgttgtc 1740
gggaagatgc gtgatctgat ccttcaactc agcaaaagtt cgatttattc aacaaagccg 1800
ccgtcccgtc aagtcagcgt aatgctctgc cagtgttaca accaattaac caattctgat 1860
tagaaaaact catcgagcat caaatgaaac tgcaatttat tcatatcagg attatcaata 1920
ccatattttt gaaaaagccg tttctgtaat gaaggagaaa actcaccgag gcagttccat 1980
aggatggcaa gatcctggta tcggtctgcg attccgactc gtccaacatc aatacaacct 2040
attaatttcc cctcgtcaaa aataaggtta tcaagtgaga aatcaccatg agtgacgact 2100
gaatccggtg agaatggcaa aagcttatgc atttctttcc agacttgttc aacaggccag 2160
ccattacgct cgtcatcaaa atcactcgca tcaaccaaac cgttattcat tcgtgattgc 2220
gcctgagcga gacgaaatac gcgatcgctg ttaaaaggac aattacaaac aggaatcgaa 2280
tgcaaccggc gcaggaacac tgccagcgca tcaacaatat tttcacctga atcaggatat 2340
tcttctaata cctggaatgc tgttttcccg gggatcgcag tggtgagtaa ccatgcatca 2400
tcaggagtac ggataaaatg cttgatggtc ggaagaggca taaattccgt cagccagttt 2460
agtctgacca tctcatctgt aacatcattg gcaacgctac ctttgccatg tttcagaaac 2520
aactctggcg catcgggctt cccatacaat cgatagattg tcgcacctga ttgcccgaca 2580
ttatcgcgag cccatttata cccatataaa tcagcatcca tgttggaatt taatcgcggc 2640
ctcgagcaag acgtttcccg ttgaatatgg ctcataacac cccttgtatt actgtttatg 2700
taagcagaca gttttattgt tcatgatgat atatttttat cttgtgcaat gtaacatcag 2760
agattttgag acacaacgtg gctttccccc cccccccatt attgaagcat ttatcagggt 2820
tattgtctca tgagcggata catatttgaa tgtatttaga aaaataaaca aataggggtt 2880
ccgcgcacat ttccccgaaa agtgccacct gacgtctaag aaaccattat tatcatgaca 2940
ttaacctata aaaataggcg tatcacgagg ccctttcgtc ctcgcgcgtt tcggtgatga 3000
cggtgaaaac ctctgacaca tgcagctccc ggagacggtc acagcttgtc tgtaagcgga 3060
tgccgggagc agacaagccc gtcagggcgc gtcagcgggt gttggcgggt gtcggggctg 3120
gcttaactat gcggcatcag agcagattgt actgagagtg caccatatgc ggtgtgaaat 3180
accgcacaga tgcgtaagga gaaaataccg catcagattg gctattggcc attgcatacg 3240
ttgtatccat atcataatat gtacatttat attggctcat gtccaacatt accgccatgt 3300
tgacattgat tattgactag ttattaatag taatcaatta cggggtcatt agttcatagc 3360
ccatatatgg agttccgcgt tacataactt acggtaaatg gcccgcctgg ctgaccgccc 3420
aacgaccccc gcccattgac gtcaataatg acgtatgttc ccatagtaac gccaataggg 3480
actttccatt gacgtcaatg ggtggagtat ttacggtaaa ctgcccactt ggcagtacat 3540
caagtgtatc atatgccaag tacgccccct attgacgtca atgacggtaa atggcccgcc 3600
tggcattatg cccagtacat gaccttatgg gactttccta cttggcagta catctacgta 3660
ttagtcatcg ctattaccat ggtgatgcgg ttttggcagt acatcaatgg gcgtggatag 3720
cggtttgact cacggggatt tccaagtctc caccccattg acgtcaatgg gagtttgttt 3780
tggcaccaaa atcaacggga ctttccaaaa tgtcgtaaca actccgcccc attgacgcaa 3840
atgggcggta ggcgtgtacg gtgggaggtc tatataagca gagctcgttt agtgaaccgt 3900

cagatcgcct ggagacgcca tccacgctgt tttgacctcc atagaagaca ccgggaccga 3960
tccagcctcc gcggccggga acggtgcatt ggaacgcgga ttccccgtgc caagagtgac 4020
gtaagtaccg cctatagact ctataggcac acccctttgg ctcttatgca tgctatactg 4080
tttttggctt ggggcctata cacccccgct tccttatgct ataggtgatg gtatagctta 4140
gcctataggt gtgggttatt gaccattatt gaccactccc ctattggtga cgatactttc 4200
cattactaat ccataacatg gctctttgcc acaactatct ctattggcta tatgccaata 4260
ctctgtcctt cagagactga cacggactct gtatttttac aggatggggt cccatttatt 4320
atttacaaat tcacatatac aacaacgccg tcccccgtgc ccgcagtttt tattaaacat 4380
agcgtgggat ctccacgcga atctcgggta cgtgttccgg acatgggctc ttctccggta 4440
gcggcggagc ttccacatcc gagccctggt cccatgcctc cagcggctca tggtcgctcg 4500
gcagctcctt gctcctaaca gtggaggcca gacttaggca cagcacaatg cccaccacca 4560
ccagtgtgcc gcacaaggcc gtggcggtag ggtatgtgtc tgaaaatgag cgtggagatt 4620
gggctcgcac ggctgacgca gatggaagac ttaaggcagc ggcagaagaa gatgcaggca 4680
gctgagttgt tgtattctga taagagtcag aggtaactcc cgttgcggtg ctgttaacgg 4740
tggagggcag tgtagtctga gcagtactcg ttgctgccgc gcgcgccacc agacataata 4800
gctgacagac taacagactg ttcctttcca tgggtctttt ctgcagtcac cgtcgtcgac 4860
acgtgtgatc agatatcgcg gccgctctag accaggcgcc tggatccgcc accatgccac 4920
tctgggtgtt cttctttgtg atcctcaccc tcagcaacag ctcccactgc tccccacctc 4980
ccctttgacc cctcaggatg cggcggtatg cagatgccat cttcaccaac agctaccgga 5040
aggtgctggg ccagctgtcc gcccgcaagc tcctccagga catcatgagc aggtagagat 5100
ccagatct 5108

<210> 53
<211> 3954
<212> DNA
<213> Artificial Sequence

<220>
<223> Description of Artificial Sequence:
      pGHRH1-29YWTSK685 construct

<400> 53
ggtaccatcg ctggggagct gggggagggg tcgccttcct gccctaccca ggactccggg 60
tgcgaccgct cctctatctc tccagcccac caccactcca ccacttggac acgtctccct 120
cctccctgga gtcgctctag agggtttggg ggtctgagta aagaacccga agtagggata 180
cagtgtggcg gcaccttcca gaggccccgg gcgcagggta gaccggggcg gggcggcccg 240
cggacaggtg cagccccagg cgcaggcgca ctcgcgcctc ccggcgcagg cggtgaacct 300
cgccccaccc cagcccctcc ggggggcagc tgggccgggt cgggaggggc ccaccagccc 360
gggagacact ccatatacgg ccaggcccgc tttacctggg ctccggccag gccgctcctt 420
ctttggtcag cacaggggac ccgggcgggg gcccaggccg ctaacccgcc gggggagggg 480
gctccagtgc ccaacaccca aatatggctc gagaagggga gcgacattcc agtgaggcgg 540
ctcgggggga gaacccgcgg gctatataaa acctgagcgt ggggaccagc ggccaccgca 600
gcggacagcg ccgagagaag cctcgcttcc ctcccgcggc gaccagggcc ccagccggag 660
agcagcaggt gtagccacca agcttgccac catgccactc tgggtgttct tctttgtgat 720
cctcaccctc agcaacagct cccactgctc cccacctccc cctttgaccc tcaggatgcg 780
gcggtattat gcagatgcca tcttcaccaa cagctaccgg aaggtgctgg gccagctgtc 840
cgcccgcaag ctcctccagg acatcatgag caggtagtct agagtcgggg cggccggccg 900

cttcgagcag acatgataag atacattgat gagtttggac aaaccacaac tagaatgcag 960
tgaaaaaaat gctttatttg tgaaatttgt gatgctattg ctttatttgt aaccattata 1020
agctgcaata aacaagttaa caacaacaat tgcattcatt ttatgtttca ggttcagggg 1080
gaggtgtggg aggtttttta aagcaagtaa aacctctaca aatgtggtaa aatcgataag 1140
gatccgtcga ccgatgccct tgagagcctt caacccagtc agctccttcc ggtgggcgcg 1200
gggcatgact atcgtcgccg cacttatgac tgtcttcttt atcatgcaac tcgtaggaca 1260
ggtgccggca gcgctcttcc gcttcctcgc tcactgactc gctgcgctcg gtcgttcggc 1320
tgcggcgagc ggtatcagct cactcaaagg cggtaatacg gttatccaca gaatcagggg 1380
ataacgcagg aaagaacatg tgagcaaaag gccagcaaaa ggccaggaac cgtaaaaagg 1440
ccgcgttgct ggcgtttttc cataggctcc gcccccctga cgagcatcac aaaaatcgac 1500
gctcaagtca gaggtggcga aacccgacag gactataaag ataccaggcg tttccccctg 1560
gaagctccct cgtgcgctct cctgttccga ccctgccgct taccggatac ctgtccgcct 1620
ttctcccttc gggaagcgtg gcgctttctc aatgctcacg ctgtaggtat ctcagttcgg 1680
tgtaggtcgt tcgctccaag ctgggctgtg tgcacgaacc ccccgttcag cccgaccgct 1740
gcgccttatc cggtaactat cgtcttgagt ccaacccggt aagacacgac ttatcgccac 1800
tggcagcagc cactggtaac aggattagca gagcgaggta tgtaggcggt gctacagagt 1860
tcttgaagtg gtggcctaac tacggctaca ctagaaggac agtatttggt atctgcgctc 1920
tgctgaagcc agttaccttc ggaaaaagag ttggtagctc ttgatccggc aaacaaacca 1980
ccgctggtag cggtggtttt tttgtttgca agcagcagat tacgcgcaga aaaaaaggat 2040
ctcaagaaga tcctttgatc ttttctacgg ggtctgacgc tcagtggaac gaaaactcac 2100
gttaagggat tttggtcatg agattatcaa aaaggatctt cacctagatc cttttaaatt 2160
aaaaatgaag ttttaaatca atctaaagta tatatgagta aacttggtct gacagttacc 2220
aatgcttaat cagtgaggca cctatctcag cgatctgtct atttcgttca tccatagttg 2280
cctgactccc cgtcgtgtag ataactacga tacgggaggg cttaccatct ggccccagtg 2340
ctgcaatgat accgcgagac ccacgctcac cggctccaga tttatcagca ataaaccagc 2400
cagccggaag ggccgagcgc agaagtggtc ctgcaacttt atccgcctcc atccagtcta 2460
ttaattgttg ccgggaagct agagtaagta gttcgccagt taatagtttg cgcaacgttg 2520
ttgccattgc tacaggcatc gtggtgtcac gctcgtcgtt tggtatggct tcattcagct 2580
ccggttccca acgatcaagg cgagttacat gatcccccat gttgtgcaaa aaagcggtta 2640
gctccttcgg tcctccgatc gttgtcagaa gtaagttggc cgcagtgtta tcactcatgg 2700
ttatggcagc actgcataat tctcttactg tcatgccatc cgtaagatgc ttttctgtga 2760
ctggtgagta ctcaaccaag tcattctgag aatagtgtat gcggcgaccg agttgctctt 2820
gcccggcgtc aatacgggat aataccgcgc cacatagcag aactttaaaa gtgctcatca 2880
ttggaaaacg ttcttcgggg cgaaaactct caaggatctt accgctgttg agatccagtt 2940
cgatgtaacc cactcgtgca cccaactgat cttcagcatc ttttactttc accagcgttt 3000
ctgggtgagc aaaaacagga aggcaaaatg ccgcaaaaaa gggaataagg gcgacacgga 3060
aatgttgaat actcatactc ttcctttttc aatattattg aagcatttat cagggttatt 3120
gtctcatgag cggatacata tttgaatgta tttagaaaaa taaacaaata ggggttccgc 3180
gcacatttcc ccgaaaagtg ccacctgacg cgccctgtag cggcgcatta agcgcggcgg 3240
gtgtggtggt tacgcgcagc gtgaccgcta cacttgccag cgccctagcg cccgctcctt 3300
tcgctttctt cccttccttt ctcgccacgt tcgccggctt tccccgtcaa gctctaaatc 3360
gggggctccc tttagggttc cgatttagtg ctttacggca cctcgacccc aaaaaacttg 3420
attagggtga tggttcacgt agtgggccat cgccctgata gacggttttt cgccctttga 3480
cgttggagtc cacgttcttt aatagtggac tcttgttcca aactggaaca acactcaacc 3540
ctatctcggt ctattctttt gatttataag ggattttgcc gatttcggcc tattggttaa 3600
aaaatgagct gatttaacaa aaatttaacg cgaattttaa caaaatatta acgtttacaa 3660
tttcccattc gccattcagg ctgcgcaact gttgggaagg gcgatcggtg cgggcctctt 3720
cgctattacg ccagcccaag ctaccatgat aagtaagtaa tattaaggta cgggaggtac 3780

ttggagcggc cgcaataaaa tatctttatt ttcattacat ctgtgtgttg gttttttgtg 3840
tgaatcgata gtactaacat acgctctcca tcaaaacaaa acgaaacaaa acaaactagc 3900
aaaataggct gtccccagtg caagtgcagg tgccagaaca tttctctatc gata 3954

<210> 54
<211> 5163
<212> DNA
<213> Artificial Sequence

<220>
<223> Description of Artificial Sequence:
      pGHRH1-29YWTSK2014 construct

<400> 54
ggtaccgcta taggagagaa aagagctgca ctgagcaccc tccttcccct ttaaatgtca 60
acagattagg agtcagtgaa tgacagcaca cctcttgcta ccttagagac caaaatttaa 120
gctactcccc ttaagctata gctagagtgc acctgccagt gtctttagtc cccactgatg 180
gaacaggacc caaggtattg aagatggaac atagttattc attcatcctc taatttaaaa 240
agctggatat gctgtacagc agaaattgac ggaacaatgt aaatcaacta taacagaaga 300
aataaaaacc tgggggaaaa gaagctgact atgaaacccc aggagctttc tacatgggcc 360
tggactcacc aaactcttta ttttgtaatg gacttctgac atttttagga agggctgtcc 420
tgatgtgggc tatagaagag ggtttcacat gcttcttcaa gaggacccac actgtcccag 480
ttgctgagtc ccaccaccag atgctagtgg cagctatttg gggaacactt aggcactaca 540
aaaaaatgag tgattccatt ctggctcaca ccatatccct gatgtacccc ttaaagcatg 600
tcactgagtt catcacagaa aattgtttcc cctgtgcctt ccacaacaag gttagagctg 660
tccttggggc caggggaagg gggcagggag tgagaagcac caactggata acctcctctg 720
acccccactc caccttacca taagtagatc caaatccttc tagaaaatta ggaaggcata 780
tccccatata tcagcgatat aaatagaact gcttcagcgc tctggtagac ggtgactctc 840
caaggtggac tgggaggcag cctggccttg gctgggcatc gtcctctaaa tagaaagatg 900
aacttgttca gcctttccag aaggaaaact gctgcccagc ctacagtgca acgtccttgt 960
cttccatctg gaggaagcac gggtgacata tcatctagta agggcacctc tctgtttcca 1020
cctccaggtc gaggggtgtg acccttactt ctcagcctca agggagggac actcaacccc 1080
ccaaaaagac atgagggcgc tcagctcggc ccaccgcacc ccggaccgga gccgtcaccc 1140
cccgaaattc actcccttca caagccccca agcgcgttct ctggtgcgga ctgctccggg 1200
gccctggctt tgtgcccagc gttgtcagag ccaccgccct gagcctgtcc ccgggagccc 1260
cgcgcctcct cccaccgctc cgctctcgcg ccccgcggcc agttgtctgc cccgagacag 1320
ctgcgcgccc tcccgctgcc ggtggccctc tccggtgggg gtggggaccg acagggtcag 1380
ccctccggat ccggggcgct ccgggtagcg gggagaagtg atcgctgggg agctggggga 1440
ggggtcgcct tcctgcccta cccaggactc cgggtgcgac cgctcctcta tctctccagc 1500
ccgggcgcag ggtagaccgg ggcggggcgg cccgcggaca ggtgcagccc caggcgcagg 1560
cgcactcgcg cctcccggcg caggcggtga acctcgcccc accccagccc ctccgggggg 1620
cagctgggcc gggtcgggag ggcccaccca gcccgggaga cactccatat acggccaggc 1680
ccgctttacc tgggctccgg ccaggccgct ccttctttgg tcagcacagg ggacccgggc 1740
gggggcccag gccgctaacc cgccggggga gggggctcca gtgcccaaca cccaaatatg 1800
gctcgagaag gggagcgaca ttccagtgag gcggctcggg gggagaaccc gcgggctata 1860
taaaacctga gcgtggggac cagcggccaa gcttgccacc atgccactct gggtgttctt 1920
ctttgtgatc ctcaccctca gcaacagctc ccactgctcc ccacctcccc ctttgaccct 1980

caggatgcgg cggtattatg cagatgccat cttcaccaac agctaccgga aggtgctggg 2040
ccagctgtcc gcccgcaagc tcctccagga catcatgagc aggtagtcta gagtcggggc 2100
ggccggccgc ttcgagcaga catgataaga tacattgatg agtttggaca aaccacaact 2160
agaatgcagt gaaaaaaatg ctttatttgt gaaatttgtg atgctattgc tttatttgta 2220
accattataa gctgcaataa acaagttaac aacaacaatt gcattcattt tatgtttcag 2280
gttcaggggg aggtgtggga ggttttttaa agcaagtaaa acctctacaa atgtggtaaa 2340
atcgataagg atccgtcgac cgatgccctt gagagccttc aacccagtca gctccttccg 2400
gtgggcgcgg ggcatgacta tcgtcgccgc acttatgact gtcttcttta tcatgcaact 2460
cgtaggacag gtgccggcag cgctcttccg cttcctcgct cactgactcg ctgcgctcgg 2520
tcgttcggct gcggcgagcg gtatcagctc actcaaaggc ggtaatacgg ttatccacag 2580
aatcagggga taacgcagga aagaacatgt gagcaaaagg ccagcaaaag gccaggaacc 2640
gtaaaaaggc cgcgttgctg gcgtttttcc ataggctccg cccccctgac gagcatcaca 2700
aaaatcgacg ctcaagtcag aggtggcgaa acccgacagg actataaaga taccaggcgt 2760
ttccccctgg aagctccctc gtgcgctctc ctgttccgac cctgccgctt accggatacc 2820
tgtccgcctt tctcccttcg ggaagcgtgg cgctttctca atgctcacgc tgtaggtatc 2880
tcagttcggt gtaggtcgtt cgctccaagc tgggctgtgt gcacgaaccc cccgttcagc 2940
ccgaccgctg cgccttatcc ggtaactatc gtcttgagtc caacccggta agacacgact 3000
tatcgccact ggcagcagcc actggtaaca ggattagcag agcgaggtat gtaggcggtg 3060
ctacagagtt cttgaagtgg tggcctaact acggctacac tagaaggaca gtatttggta 3120
tctgcgctct gctgaagcca gttaccttcg gaaaaagagt tggtagctct tgatccggca 3180
aacaaaccac cgctggtagc ggtggttttt ttgtttgcaa gcagcagatt acgcgcagaa 3240
aaaaaggatc tcaagaagat cctttgatct tttctacggg gtctgacgct cagtggaacg 3300
aaaactcacg ttaagggatt ttggtcatga gattatcaaa aaggatcttc acctagatcc 3360
ttttaaatta aaaatgaagt tttaaatcaa tctaaagtat atatgagtaa acttggtctg 3420
acagttacca atgcttaatc agtgaggcac ctatctcagc gatctgtcta tttcgttcat 3480
ccatagttgc ctgactcccc gtcgtgtaga taactacgat acgggagggc ttaccatctg 3540
gccccagtgc tgcaatgata ccgcgagacc cacgctcacc ggctccagat ttatcagcaa 3600
taaaccagcc agccggaagg gccgagcgca gaagtggtcc tgcaacttta tccgcctcca 3660
tccagtctat taattgttgc cgggaagcta gagtaagtag ttcgccagtt aatagtttgc 3720
gcaacgttgt tgccattgct acaggcatcg tggtgtcacg ctcgtcgttt ggtatggctt 3780
cattcagctc cggttcccaa cgatcaaggc gagttacatg atcccccatg ttgtgcaaaa 3840
aagcggttag ctccttcggt cctccgatcg ttgtcagaag taagttggcc gcagtgttat 3900
cactcatggt tatggcagca ctgcataatt ctcttactgt catgccatcc gtaagatgct 3960
tttctgtgac tggtgagtac tcaaccaagt cattctgaga atagtgtatg cggcgaccga 4020
gttgctcttg cccggcgtca atacgggata ataccgcgcc acatagcaga actttaaaag 4080
tgctcatcat tggaaaacgt tcttcggggc gaaaactctc aaggatctta ccgctgttga 4140
gatccagttc gatgtaaccc actcgtgcac ccaactgatc ttcagcatct tttactttca 4200
ccagcgtttc tgggtgagca aaaacaggaa ggcaaaatgc cgcaaaaaag ggaataaggg 4260
cgacacggaa atgttgaata ctcatactct tcctttttca atattattga agcatttatc 4320
agggttattg tctcatgagc ggatacatat ttgaatgtat ttagaaaaat aaacaaatag 4380
gggttccgcg cacatttccc cgaaaagtgc cacctgacgc gccctgtagc ggcgcattaa 4440
gcgcggcggg tgtggtggtt acgcgcagcg tgaccgctac acttgccagc gccctagcgc 4500
ccgctccttt cgctttcttc ccttcctttc tcgccacgtt cgccggcttt ccccgtcaag 4560
ctctaaatcg ggggctccct ttagggttcc gatttagtgc tttacggcac ctcgacccca 4620
aaaaacttga ttagggtgat ggttcacgta gtgggccatc gccctgatag acggtttttc 4680
gccctttgac gttggagtcc acgttcttta atagtggact cttgttccaa actggaacaa 4740
cactcaaccc tatctcggtc tattcttttg atttataagg gattttgccg atttcggcct 4800
attggttaaa aaatgagctg atttaacaaa aatttaacgc gaattttaac aaaatattaa 4860

cgtttacaat ttcccattcg ccattcaggc tgcgcaactg ttgggaaggg cgatcggtgc 4920
gggcctcttc gctattacgc cagcccaagc taccatgata agtaagtaat attaaggtac 4980
gggaggtact tggagcggcc gcaataaaat atctttattt tcattacatc tgtgtgttgg 5040
ttttttgtgt gaatcgatag tactaacata cgctctccat caaaacaaaa cgaaacaaaa 5100
caaactagca aaataggctg tccccagtgc aagtgcaggt gccagaacat ttctctatcg 5160
ata 5163

<210> 55
<211> 5111
<212> DNA
<213> Artificial Sequence

<220>
<223> Description of Artificial Sequence:
      pGHRH1-29Yala1522CMV construct

<400> 55
gctgtgcctt ctagttgcca gccatctgtt gtttgcccct cccccgtgcc ttccttgacc 60
ctggaaggtg ccactcccac tgtcctttcc taataaaatg aggaaattgc atcgcattgt 120
ctgagtaggt gtcattctat tctggggggt ggggtggggc aggacagcaa gggggaggat 180
tgggaagaca atagcaggca tgctggggat gcggtgggct ctatgggtac ccaggtgctg 240
aagaattgac ccggttcctc ctgggccaga aagaagcagg cacatcccct tctctgtgac 300
acaccctgtc cacgcccctg gttcttagtt ccagccccac tcataggaca ctcatagctc 360
aggagggctc cgccttcaat cccacccgct aaagtacttg gagcggtctc tccctccctc 420
atcagcccac caaaccaaac ctagcctcca agagtgggaa gaaattaaag caagataggc 480
tattaagtgc agagggagag aaaatgcctc caacatgtga ggaagtaatg agagaaatca 540
tagaatttct tccgcttcct cgctcactga ctcgctgcgc tcggtcgttc ggctgcggcg 600
agcggtatca gctcactcaa aggcggtaat acggttatcc acagaatcag gggataacgc 660
aggaaagaac atgtgagcaa aaggccagca aaaggccagg aaccgtaaaa aggccgcgtt 720
gctggcgttt ttccataggc tccgcccccc tgacgagcat cacaaaaatc gacgctcaag 780
tcagaggtgg cgaaacccga caggactata aagataccag gcgtttcccc ctggaagctc 840
cctcgtgcgc tctcctgttc cgaccctgcc gcttaccgga tacctgtccg cctttctccc 900
ttcgggaagc gtggcgcttt ctcatagctc acgctgtagg tatctcagtt cggtgtaggt 960
cgttcgctcc aagctgggct gtgtgcacga accccccgtt cagcccgacc gctgcgcctt 1020
atccggtaac tatcgtcttg agtccaaccc ggtaagacac gacttatcgc cactggcagc 1080
agccactggt aacaggatta gcagagcgag gtatgtaggc ggtgctacag agttcttgaa 1140
gtggtggcct aactacggct acactagaag aacagtattt ggtatctgcg ctctgctgaa 1200
gccagttacc ttcggaaaaa gagttggtag ctcttgatcc ggcaaacaaa ccaccgctgg 1260
tagcggtggt ttttttgttt gcaagcagca gattacgcgc agaaaaaaag gatctcaaga 1320
agatcctttg atcttttcta cggggtctga cgctcagtgg aacgaaaact cacgttaagg 1380
gattttggtc atgagattat caaaaaggat cttcacctag atccttttaa attaaaaatg 1440
aagttttaaa tcaatctaaa gtatatatga gtaaacttgg tctgacagtt accaatgctt 1500
aatcagtgag gcacctatct cagcgatctg tctatttcgt tcatccatag ttgcctgact 1560
cggggggggg gggcgctgag gtctgcctcg tgaagaaggt gttgctgact cataccaggc 1620
ctgaatcgcc ccatcatcca gccagaaagt gagggagcca cggttgatga gagctttgtt 1680
gtaggtggac cagttggtga ttttgaactt ttgctttgcc acggaacggt ctgcgttgtc 1740
gggaagatgc gtgatctgat ccttcaactc agcaaaagtt cgatttattc aacaaagccg 1800

ccgtcccgtc aagtcagcgt aatgctctgc cagtgttaca accaattaac caattctgat 1860
tagaaaaact catcgagcat caaatgaaac tgcaatttat tcatatcagg attatcaata 1920
ccatattttt gaaaaagccg tttctgtaat gaaggagaaa actcaccgag gcagttccat 1980
aggatggcaa gatcctggta tcggtctgcg attccgactc gtccaacatc aatacaacct 2040
attaatttcc cctcgtcaaa aataaggtta tcaagtgaga aatcaccatg agtgacgact 2100
gaatccggtg agaatggcaa aagcttatgc atttctttcc agacttgttc aacaggccag 2160
ccattacgct cgtcatcaaa atcactcgca tcaaccaaac cgttattcat tcgtgattgc 2220
gcctgagcga gacgaaatac gcgatcgctg ttaaaaggac aattacaaac aggaatcgaa 2280
tgcaaccggc gcaggaacac tgccagcgca tcaacaatat tttcacctga atcaggatat 2340
tcttctaata cctggaatgc tgttttcccg gggatcgcag tggtgagtaa ccatgcatca 2400
tcaggagtac ggataaaatg cttgatggtc ggaagaggca taaattccgt cagccagttt 2460
agtctgacca tctcatctgt aacatcattg gcaacgctac ctttgccatg tttcagaaac 2520
aactctggcg catcgggctt cccatacaat cgatagattg tcgcacctga ttgcccgaca 2580
ttatcgcgag cccatttata cccatataaa tcagcatcca tgttggaatt taatcgcggc 2640
ctcgagcaag acgtttcccg ttgaatatgg ctcataacac cccttgtatt actgtttatg 2700
taagcagaca gttttattgt tcatgatgat atatttttat cttgtgcaat gtaacatcag 2760
agattttgag acacaacgtg gctttccccc cccccccatt attgaagcat ttatcagggt 2820
tattgtctca tgagcggata catatttgaa tgtatttaga aaaataaaca aataggggtt 2880
ccgcgcacat ttccccgaaa agtgccacct gacgtctaag aaaccattat tatcatgaca 2940
ttaacctata aaaataggcg tatcacgagg ccctttcgtc ctcgcgcgtt tcggtgatga 3000
cggtgaaaac ctctgacaca tgcagctccc ggagacggtc acagcttgtc tgtaagcgga 3060
tgccgggagc agacaagccc gtcagggcgc gtcagcgggt gttggcgggt gtcggggctg 3120
gcttaactat gcggcatcag agcagattgt actgagagtg caccatatgc ggtgtgaaat 3180
accgcacaga tgcgtaagga gaaaataccg catcagattg gctattggcc attgcatacg 3240
ttgtatccat atcataatat gtacatttat attggctcat gtccaacatt accgccatgt 3300
tgacattgat tattgactag ttattaatag taatcaatta cggggtcatt agttcatagc 3360
ccatatatgg agttccgcgt tacataactt acggtaaatg gcccgcctgg ctgaccgccc 3420
aacgaccccc gcccattgac gtcaataatg acgtatgttc ccatagtaac gccaataggg 3480
actttccatt gacgtcaatg ggtggagtat ttacggtaaa ctgcccactt ggcagtacat 3540
caagtgtatc atatgccaag tacgccccct attgacgtca atgacggtaa atggcccgcc 3600
tggcattatg cccagtacat gaccttatgg gactttccta cttggcagta catctacgta 3660
ttagtcatcg ctattaccat ggtgatgcgg ttttggcagt acatcaatgg gcgtggatag 3720
cggtttgact cacggggatt tccaagtctc caccccattg acgtcaatgg gagtttgttt 3780
tggcaccaaa atcaacggga ctttccaaaa tgtcgtaaca actccgcccc attgacgcaa 3840
atgggcggta ggcgtgtacg gtgggaggtc tatataagca gagctcgttt agtgaaccgt 3900
cagatcgcct ggagacgcca tccacgctgt tttgacctcc atagaagaca ccgggaccga 3960
tccagcctcc gcggccggga acggtgcatt ggaacgcgga ttccccgtgc caagagtgac 4020
gtaagtaccg cctatagact ctataggcac acccctttgg ctcttatgca tgctatactg 4080
tttttggctt ggggcctata cacccccgct tccttatgct ataggtgatg gtatagctta 4140
gcctataggt gtgggttatt gaccattatt gaccactccc ctattggtga cgatactttc 4200
cattactaat ccataacatg gctctttgcc acaactatct ctattggcta tatgccaata 4260
ctctgtcctt cagagactga cacggactct gtatttttac aggatggggt cccatttatt 4320
atttacaaat tcacatatac aacaacgccg tcccccgtgc ccgcagtttt tattaaacat 4380
agcgtgggat ctccacgcga atctcgggta cgtgttccgg acatgggctc ttctccggta 4440
gcggcggagc ttccacatcc gagccctggt cccatgcctc cagcggctca tggtcgctcg 4500
gcagctcctt gctcctaaca gtggaggcca gacttaggca cagcacaatg cccaccacca 4560
ccagtgtgcc gcacaaggcc gtggcggtag ggtatgtgtc tgaaaatgag cgtggagatt 4620
gggctcgcac ggctgacgca gatggaagac ttaaggcagc ggcagaagaa gatgcaggca 4680

gctgagttgt tgtattctga taagagtcag aggtaactcc cgttgcggtg ctgttaacgg 4740
tggagggcag tgtagtctga gcagtactcg ttgctgccgc gcgcgccacc agacataata 4800
gctgacagac taacagactg ttcctttcca tgggtctttt ctgcagtcac cgtcgtcgac 4860
acgtgtgatc agatatcgcg gccgctctag accaggcgcc tggatccgcc accatgccac 4920
tctgggtgtt cttctttgtg atcctcaccc tcagcaacag ctcccactgc tccccacctc 4980
cccctttgac cctcaggatg cggcggtatt atgcagatgc catcttcacc aacagctacc 5040
ggaaggtgct ggcccagctg tccgcccgca aggccctcca ggacatcatg agcaggtaga 5100
gatccagatc t 5111

<210> 56
<211> 3327
<212> DNA
<213> Artificial Sequence

<220>
<223> Description of Artificial Sequence:
      pGHRH1-29Yala1522SK construct

<400> 56
ggtaccgagc tcttacgcgt gctagcccgg gctcgagatc tgcgatctaa gtaagcttgc 60
caccatgcca ctctgggtgt tcttctttgt gatcctcacc ctcagcaaca gctcccactg 120
ctccccacct ccccctttga ccctcaggat gcggcggtat tatgcagatg ccatcttcac 180
caacagctac cggaaggtgc tggcccagct gtccgcccgc aaggccctcc aggacatcat 240
gagcaggtag tctagagtcg gggcggccgg ccgcttcgag cagacatgat aagatacatt 300
gatgagtttg gacaaaccac aactagaatg cagtgaaaaa aatgctttat ttgtgaaatt 360
tgtgatgcta ttgctttatt tgtaaccatt ataagctgca ataaacaagt taacaacaac 420
aattgcattc attttatgtt tcaggttcag ggggaggtgt gggaggtttt ttaaagcaag 480
taaaacctct acaaatgtgg taaaatcgat aaggatccgt cgaccgatgc ccttgagagc 540
cttcaaccca gtcagctcct tccggtgggc gcggggcatg actatcgtcg ccgcacttat 600
gactgtcttc tttatcatgc aactcgtagg acaggtgccg gcagcgctct tccgcttcct 660
cgctcactga ctcgctgcgc tcggtcgttc ggctgcggcg agcggtatca gctcactcaa 720
aggcggtaat acggttatcc acagaatcag gggataacgc aggaaagaac atgtgagcaa 780
aaggccagca aaaggccagg aaccgtaaaa aggccgcgtt gctggcgttt ttccataggc 840
tccgcccccc tgacgagcat cacaaaaatc gacgctcaag tcagaggtgg cgaaacccga 900
caggactata aagataccag gcgtttcccc ctggaagctc cctcgtgcgc tctcctgttc 960
cgaccctgcc gcttaccgga tacctgtccg cctttctccc ttcgggaagc gtggcgcttt 1020
ctcaatgctc acgctgtagg tatctcagtt cggtgtaggt cgttcgctcc aagctgggct 1080
gtgtgcacga accccccgtt cagcccgacc gctgcgcctt atccggtaac tatcgtcttg 1140
agtccaaccc ggtaagacac gacttatcgc cactggcagc agccactggt aacaggatta 1200
gcagagcgag gtatgtaggc ggtgctacag agttcttgaa gtggtggcct aactacggct 1260
acactagaag gacagtattt ggtatctgcg ctctgctgaa gccagttacc ttcggaaaaa 1320
gagttggtag ctcttgatcc ggcaaacaaa ccaccgctgg tagcggtggt ttttttgttt 1380
gcaagcagca gattacgcgc agaaaaaaag gatctcaaga agatcctttg atcttttcta 1440
cggggtctga cgctcagtgg aacgaaaact cacgttaagg gattttggtc atgagattat 1500
caaaaaggat cttcacctag atccttttaa attaaaaatg aagttttaaa tcaatctaaa 1560
gtatatatga gtaaacttgg tctgacagtt accaatgctt aatcagtgag gcacctatct 1620
cagcgatctg tctatttcgt tcatccatag ttgcctgact ccccgtcgtg tagataacta 1680

cgatacggga gggcttacca tctggcccca gtgctgcaat gataccgcga gacccacgct 1740
caccggctcc agatttatca gcaataaacc agccagccgg aagggccgag cgcagaagtg 1800
gtcctgcaac tttatccgcc tccatccagt ctattaattg ttgccgggaa gctagagtaa 1860
gtagttcgcc agttaatagt ttgcgcaacg ttgttgccat tgctacaggc atcgtggtgt 1920
cacgctcgtc gtttggtatg gcttcattca gctccggttc ccaacgatca aggcgagtta 1980
catgatcccc catgttgtgc aaaaaagcgg ttagctcctt cggtcctccg atcgttgtca 2040
gaagtaagtt ggccgcagtg ttatcactca tggttatggc agcactgcat aattctctta 2100
ctgtcatgcc atccgtaaga tgcttttctg tgactggtga gtactcaacc aagtcattct 2160
gagaatagtg tatgcggcga ccgagttgct cttgcccggc gtcaatacgg gataataccg 2220
cgccacatag cagaacttta aaagtgctca tcattggaaa acgttcttcg gggcgaaaac 2280
tctcaaggat cttaccgctg ttgagatcca gttcgatgta acccactcgt gcacccaact 2340
gatcttcagc atcttttact ttcaccagcg tttctgggtg agcaaaaaca ggaaggcaaa 2400
atgccgcaaa aaagggaata agggcgacac ggaaatgttg aatactcata ctcttccttt 2460
ttcaatatta ttgaagcatt tatcagggtt attgtctcat gagcggatac atatttgaat 2520
gtatttagaa aaataaacaa ataggggttc cgcgcacatt tccccgaaaa gtgccacctg 2580
acgcgccctg tagcggcgca ttaagcgcgg cgggtgtggt ggttacgcgc agcgtgaccg 2640
ctacacttgc cagcgcccta gcgcccgctc ctttcgcttt cttcccttcc tttctcgcca 2700
cgttcgccgg ctttccccgt caagctctaa atcgggggct ccctttaggg ttccgattta 2760
gtgctttacg gcacctcgac cccaaaaaac ttgattaggg tgatggttca cgtagtgggc 2820
catcgccctg atagacggtt tttcgccctt tgacgttgga gtccacgttc tttaatagtg 2880
gactcttgtt ccaaactgga acaacactca accctatctc ggtctattct tttgatttat 2940
aagggatttt gccgatttcg gcctattggt taaaaaatga gctgatttaa caaaaattta 3000
acgcgaattt taacaaaata ttaacgttta caatttccca ttcgccattc aggctgcgca 3060
actgttggga agggcgatcg gtgcgggcct cttcgctatt acgccagccc aagctaccat 3120
gataagtaag taatattaag gtacgggagg tacttggagc ggccgcaata aaatatcttt 3180
attttcatta catctgtgtg ttggtttttt gtgtgaatcg atagtactaa catacgctct 3240
ccatcaaaac aaaacgaaac aaaacaaact agcaaaatag gctgtcccca gtgcaagtgc 3300
aggtgccaga acatttctct atcgata 3327

<210> 57
<211> 4469
<212> DNA
<213> Artificial Sequence

<220>
<223> Description of Artificial Sequence:
      pGHRH1-29Yala1522SK construct

<400> 57
bbsggtacca tcgctgggga gctgggggag ggtcgccttt cctgccctac ccaggactcc 60
gggtgcgacc gctcctctat ctctccagcc caccaccact ccaccacttg gacacgtctc 120
cctcctccct ggagtcgctc tagagggttt gggggtctga gtaaagaacc cgaagtaggg 180
atacagtgtg gcggcacctt ccagaggccc cgggcgcagg gtagaccggg gcggggcggc 240
ccgcggacag gtgcagcccc aggcgcaggc gcactcgcgc ctcccggcgc aggcggtgaa 300
cctcgcccca ccccagcccc tccggggggc agctgggccg ggtcgggagg ggcccaccag 360
cccgggagac actccatata cggccaggcc cgctttacct gggctccggc caggccgctc 420
cttctttggt cagcacaggg gacccgggcg ggggcccagg ccgctaaccc gccgggggag 480

ggggctccag tgcccaacac ccaaatatgg ctcgagaagg ggagcgacat tccagtgagg 540
cggctcgggg ggagaacccg cgggctatat aaaacctgag cgtggggacc agcggccacc 600
gcagcggaca gcgccgagag aagcctcgct tccctcccgc ggcgaccagg gccccagccg 660
gagagcagca ggtgtagcca ccaagcttgc caccatgcca ctctgggtgt tcttctttgt 720
gatcctcacc ctcagcaaca gctcccactg ctccccacct ccccctttga ccctcaggat 780
gcggcggtat tatgcagatg ccatcttcac caacagctac cggaaggtgc tggcccagct 840
gtccgcccgc aaggccctcc aggacatcat gagcaggtag tctagagtcg gggcggccgg 900
ccgcttcgag cagacatgat aagatacatt gatgagtttg gacaaaccac aactagaatg 960
cagtgaaaaa aatgctttat ttgtgaaatt tgtgatgcta ttgctttatt tgtaaccatt 1020
ataagctgca ataaacaagt taacaacaac aattgcattc attttatgtt tcaggttcag 1080
ggggaggtgt gggaggtttt ttaaagcaag taaaacctct acaaatgtgg taaaatcgat 1140
aaggatccgt cgaccgatgc ccttgagagc cttcaaccca gtcagctcct tccggtgggc 1200
gcggggcatg actatcgtcg ccgcacttat gactgtcttc tttatcatgc aactcgtagg 1260
acaggtgccg gcagcgctct tccgcttcct cgctcactga ctcgctgcgc tcggtcgttc 1320
ggctgcggcg agcggtatca gctcactcaa aggcggtaat acggttatcc acagaatcag 1380
gggataacgc aggaaagaac atgtgagcaa aaggccagca aaaggccagg aaccgtaaaa 1440
aggccgcgtt gctggcgttt ttccataggc tccgcccccc tgacgagcat cacaaaaatc 1500
gacgctcaag tcagaggtgg cgaaacccga caggactata aagataccag gcgtttcccc 1560
ctggaagctc cctcgtgcgc tctcctgttc cgaccctgcc gcttaccgga tacctgtccg 1620
cctttctccc ttcgggaagc gtggcgcttt ctcaatgctc acgctgtagg tatctcagtt 1680
cggtgtaggt cgttcgctcc aagctgggct gtgtgcacga accccccgtt cagcccgacc 1740
gctgcgcctt atccggtaac tatcgtcttg agtccaaccc ggtaagacac gacttatcgc 1800
cactggcagc agccactggt aacaggatta gcagagcgag gtatgtaggc ggtgctacag 1860
agttcttgaa gtggtggcct aactacggct acactagaag gacagtattt ggtatctgcg 1920
ctctgctgaa gccagttacc ttcggaaaaa gagttggtag ctcttgatcc ggcaaacaaa 1980
ccaccgctgg tagcggtggt ttttttgttt gcaagcagca gattacgcgc agaaaaaaag 2040
gatctcaaga agatcctttg atcttttcta cggggtctga cgctcagtgg aacgaaaact 2100
cacgttaagg gattttggtc atgagattat caaaaaggat cttcacctag atccttttaa 2160
attaaaaatg aagttttaaa tcaatctaaa gtatatatga gtaaacttgg tctgacagtt 2220
accaatgctt aatcagtgag gcacctatct cagcgatctg tctatttcgt tcatccatag 2280
ttgcctgact ccccgtcgtg tagataacta cgatacggga gggcttacca tctggcccca 2340
gtgctgcaat gataccgcga gacccacgct caccggctcc agatttatca gcaataaacc 2400
agccagccgg aagggccgag cgcagaagtg gtcctgcaac tttatccgcc tccatccagt 2460
ctattaattg ttgccgggaa gctagagtaa gtagttcgcc agttaatagt ttgcgcaacg 2520
ttgttgccat tgctacaggc atcgtggtgt cacgctcgtc gtttggtatg gcttcattca 2580
gctccggttc ccaacgatca aggcgagtta catgatcccc catgttgtgc aaaaaagcgg 2640
ttagctcctt cggtcctccg atcgttgtca gaagtaagtt ggccgcagtg ttatcactca 2700
tggttatggc agcactgcat aattctctta ctgtcatgcc atccgtaaga tgcttttctg 2760
tgactggtga gtactcaacc aagtcattct gagaatagtg tatgcggcga ccgagttgct 2820
cttgcccggc gtcaatacgg gataataccg cgccacatag cagaacttta aaagtgctca 2880
tcattggaaa acgttcttcg gggcgaaaac tctcaaggat cttaccgctg ttgagatcca 2940
gttcgatgta acccactcgt gcacccaact gatcttcagc atcttttact ttcaccagcg 3000
tttctgggtg agcaaaaaca ggaaggcaaa atgccgcaaa aaagggaata agggcgacac 3060
ggaaatgttg aatactcata ctcttccttt ttcaatatta ttgaagcatt tatcagggtt 3120
attgtctcat gagcggatac atatttgaat gtatttagaa aaataaacaa ataggggttc 3180
cgcgcacatt tccccgaaaa gtgccacctg acgcgccctg tagcggcgca ttaagcgcgg 3240
cgggtgtggt ggttacgcgc agcgtgaccg ctacacttgc cagcgcccta gcgcccgctc 3300
ctttcgcttt cttcccttcc tttctcgcca cgttcgccgg ctttccccgt caagctctaa 3360

atcgggggct ccctttaggg ttccgattta gtgctttacg gcacctcgac cccaaaaaac 3420
ttgattaggg tgatggttca cgtagtgggc catcgccctg atagacggtt tttcgccctt 3480
tgacgttgga gtccacgttc tttaatagtg gactcttgtt ccaaactgga acaacactca 3540
accctatctc ggtctattct tttgatttat aagggatttt gccgatttcg gcctattggt 3600
taaaaaatga gctgatttaa caaaaattta acgcgaattt taacaaaata ttaacgttta 3660
caatttccca ttcgccattc aggctgcgca actgttggga agggcgatcg gtgcgggcct 3720
cttcgctatt acgccagccc aagctaccat gataagtaag taatattaag gtacgggagg 3780
tacttggagc ggccgcaata aaatatcttt attttcatta catctgtgtg ttggtttttt 3840
gtgtgaatcg atagtactaa catacgctct ccatcaaaac aaaacgaaac aaaacaaact 3900
agcaaaatag gctgtcccca gtgcaagtgc aggtgccaga acatttctct atcgatarh* 3960
rhtrh*rhtt vbbtvbnrma mhadatarag rahntantta rghrshrshs thstttk*kv 4020
ak*akvwara bmwhrsbcmh stctttkvkv akvwakvwav wabmwbmwbc mnybcmnycd 4080
nycdnydrga dvsnasysda taatntndtg tmsnwrmans ymbaracrrn whrbrbggta 4140
ccatcgctgg ggagctgggg gaggggtcgc cttcctgccc tacccaggac tccgggrgad 4200
vsnrgadvsn hggtaccatc gctggggagc tgggggaggg gtcgccttcc tgccctaccc 4260
aggactccgg gcgtargadv sngggargad vsnggmcrst wrdswsdhrn cggtaccatc 4320
gctggggagc tgggggaggg gtcgccttcc tgccctaccc aggactccgg gttdgdanbc 4380
dcdc*rtntr ysstabwrdd cmntsmmary nrmatndcmn tsmmarynrm atncmbbcts 4440
smcrstwrdd cmntmswrdd cwrddcmnt 4469

<210> 58
<211> 5283
<212> DNA
<213> Artificial Sequence

<220>
<223> Description of Artificial Sequence:
      pGHRH1-29Yala1522SK2014 construct

<400> 58
ggtaccgcta taggagagaa aagagctgca ctgagcaccc tccttcccct ttaaatgtca 60
acagattagg agtcagtgaa tgacagcaca cctcttgcta ccttagagac caaaatttaa 120
gctactcccc ttaagctata gctagagtgc acctgccagt gtctttagtc cccactgatg 180
gaacaggacc caaggtattg aagatggaac atagttattc attcatcctc taatttaaaa 240
agctggatat gctgtacagc agaaattgac ggaacaatgt aaatcaacta taacagaaga 300
aataaaaacc tgggggaaaa gaagctgact atgaaacccc aggagctttc tacatgggcc 360
tggactcacc aaactcttta ttttgtaatg gacttctgac atttttagga agggctgtcc 420
tgatgtgggc tatagaagag ggtttcacat gcttcttcaa gaggacccac actgtcccag 480
ttgctgagtc ccaccaccag atgctagtgg cagctatttg gggaacactt aggcactaca 540
aaaaaatgag tgattccatt ctggctcaca ccatatccct gatgtacccc ttaaagcatg 600
tcactgagtt catcacagaa aattgtttcc cctgtgcctt ccacaacaag gttagagctg 660
tccttggggc caggggaagg gggcagggag tgagaagcac caactggata acctcctctg 720
acccccactc caccttacca taagtagatc caaatccttc tagaaaatta ggaaggcata 780
tccccatata tcagcgatat aaatagaact gcttcagcgc tctggtagac ggtgactctc 840
caaggtggac tgggaggcag cctggccttg gctgggcatc gtcctctaaa tagaaagatg 900
aacttgttca gcctttccag aaggaaaact gctgcccagc ctacagtgca acgtccttgt 960
cttccatctg gaggaagcac gggtgacata tcatctagta agggcacctc tctgtttcca 1020

cctccaggtc gaggggtgtg acccttactt ctcagcctca agggagggac actcaacccc 1080
ccaaaaagac atgagggcgc tcagctcggc ccaccgcacc ccggaccgga gccgtcaccc 1140
cccgaaattc actcccttca caagccccca agcgcgttct ctggtgcgga ctgctccggg 1200
gccctggctt tgtgcccagc gttgtcagag ccaccgccct gagcctgtcc ccgggagccc 1260
cgcgcctcct cccaccgctc cgctctcgcg ccccgcggcc agttgtctgc cccgagacag 1320
ctgcgcgccc tcccgctgcc ggtggccctc tccggtgggg gtggggaccg acagggtcag 1380
ccctccggat ccggggcgct ccgggtagcg gggagaagtg atcgctgggg agctggggga 1440
ggggtcgcct tcctgcccta cccaggactc cgggtgcgac cgctcctcta tctctccagc 1500
ccaccaccac tccaccactt ggacacgtct ccctcctccc tggagtcgct ctagagggtt 1560
tgggggtctg agtaaagaac ccgaagtagg gatacagtgt ggcggcacct tccagaggcc 1620
ccgggcgcag ggtagaccgg ggcggggcgg cccgcggaca ggtgcagccc caggcgcagg 1680
cgcactcgcg cctcccggcg caggcggtga acctcgcccc accccagccc ctccgggggg 1740
cagctgggcc gggtcgggag gggcccacca gcccgggaga cactccatat acggccaggc 1800
ccgctttacc tgggctccgg ccaggccgct ccttctttgg tcagcacagg ggacccgggc 1860
gggggcccag gccgctaacc cgccggggga gggggctcca gtgcccaaca cccaaatatg 1920
gctcgagaag gggagcgaca ttccagtgag gcggctcggg gggagaaccc gcgggctata 1980
taaaacctga gcgtggggac cagcggccaa gcttgccacc atgccactct gggtgttctt 2040
ctttgtgatc ctcaccctca gcaacagctc ccactgctcc ccacctcccc ctttgaccct 2100
caggatgcgg cggtattatg cagatgccat cttcaccaac agctaccgga aggtgctggc 2160
ccagctgtcc gcccgcaagg ccctccagga catcatgagc aggtagtcta gagtcggggc 2220
ggccggccgc ttcgagcaga catgataaga tacattgatg agtttggaca aaccacaact 2280
agaatgcagt gaaaaaaatg ctttatttgt gaaatttgtg atgctattgc tttatttgta 2340
accattataa gctgcaataa acaagttaac aacaacaatt gcattcattt tatgtttcag 2400
gttcaggggg aggtgtggga ggttttttaa agcaagtaaa acctctacaa atgtggtaaa 2460
atcgataagg atccgtcgac cgatgccctt gagagccttc aacccagtca gctccttccg 2520
gtgggcgcgg ggcatgacta tcgtcgccgc acttatgact gtcttcttta tcatgcaact 2580
cgtaggacag gtgccggcag cgctcttccg cttcctcgct cactgactcg ctgcgctcgg 2640
tcgttcggct gcggcgagcg gtatcagctc actcaaaggc ggtaatacgg ttatccacag 2700
aatcagggga taacgcagga aagaacatgt gagcaaaagg ccagcaaaag gccaggaacc 2760
gtaaaaaggc cgcgttgctg gcgtttttcc ataggctccg cccccctgac gagcatcaca 2820
aaaatcgacg ctcaagtcag aggtggcgaa acccgacagg actataaaga taccaggcgt 2880
ttccccctgg aagctccctc gtgcgctctc ctgttccgac cctgccgctt accggatacc 2940
tgtccgcctt tctcccttcg ggaagcgtgg cgctttctca atgctcacgc tgtaggtatc 3000
tcagttcggt gtaggtcgtt cgctccaagc tgggctgtgt gcacgaaccc cccgttcagc 3060
ccgaccgctg cgccttatcc ggtaactatc gtcttgagtc caacccggta agacacgact 3120
tatcgccact ggcagcagcc actggtaaca ggattagcag agcgaggtat gtaggcggtg 3180
ctacagagtt cttgaagtgg tggcctaact acggctacac tagaaggaca gtatttggta 3240
tctgcgctct gctgaagcca gttaccttcg gaaaaagagt tggtagctct tgatccggca 3300
aacaaaccac cgctggtagc ggtggttttt ttgtttgcaa gcagcagatt acgcgcagaa 3360
aaaaaggatc tcaagaagat cctttgatct tttctacggg gtctgacgct cagtggaacg 3420
aaaactcacg ttaagggatt ttggtcatga gattatcaaa aaggatcttc acctagatcc 3480
ttttaaatta aaaatgaagt tttaaatcaa tctaaagtat atatgagtaa acttggtctg 3540
acagttacca atgcttaatc agtgaggcac ctatctcagc gatctgtcta tttcgttcat 3600
ccatagttgc ctgactcccc gtcgtgtaga taactacgat acgggagggc ttaccatctg 3660
gccccagtgc tgcaatgata ccgcgagacc cacgctcacc ggctccagat ttatcagcaa 3720
taaaccagcc agccggaagg gccgagcgca gaagtggtcc tgcaacttta tccgcctcca 3780
tccagtctat taattgttgc cgggaagcta gagtaagtag ttcgccagtt aatagtttgc 3840
gcaacgttgt tgccattgct acaggcatcg tggtgtcacg ctcgtcgttt ggtatggctt 3900

cattcagctc cggttcccaa cgatcaaggc gagttacatg atcccccatg ttgtgcaaaa 3960
aagcggttag ctccttcggt cctccgatcg ttgtcagaag taagttggcc gcagtgttat 4020
cactcatggt tatggcagca ctgcataatt ctcttactgt catgccatcc gtaagatgct 4080
tttctgtgac tggtgagtac tcaaccaagt cattctgaga atagtgtatg cggcgaccga 4140
gttgctcttg cccggcgtca atacgggata ataccgcgcc acatagcaga actttaaaag 4200
tgctcatcat tggaaaacgt tcttcggggc gaaaactctc aaggatctta ccgctgttga 4260
gatccagttc gatgtaaccc actcgtgcac ccaactgatc ttcagcatct tttactttca 4320
ccagcgtttc tgggtgagca aaaacaggaa ggcaaaatgc cgcaaaaaag ggaataaggg 4380
cgacacggaa atgttgaata ctcatactct tcctttttca atattattga agcatttatc 4440
agggttattg tctcatgagc ggatacatat ttgaatgtat ttagaaaaat aaacaaatag 4500
gggttccgcg cacatttccc cgaaaagtgc cacctgacgc gccctgtagc ggcgcattaa 4560
gcgcggcggg tgtggtggtt acgcgcagcg tgaccgctac acttgccagc gccctagcgc 4620
ccgctccttt cgctttcttc ccttcctttc tcgccacgtt cgccggcttt ccccgtcaag 4680
ctctaaatcg ggggctccct ttagggttcc gatttagtgc tttacggcac ctcgacccca 4740
aaaaacttga ttagggtgat ggttcacgta gtgggccatc gccctgatag acggtttttc 4800
gccctttgac gttggagtcc acgttcttta atagtggact cttgttccaa actggaacaa 4860
cactcaaccc tatctcggtc tattcttttg atttataagg gattttgccg atttcggcct 4920
attggttaaa aaatgagctg atttaacaaa aatttaacgc gaattttaac aaaatattaa 4980
cgtttacaat ttcccattcg ccattcaggc tgcgcaactg ttgggaaggg cgatcggtgc 5040
gggcctcttc gctattacgc cagcccaagc taccatgata agtaagtaat attaaggtac 5100
gggaggtact tggagcggcc gcaataaaat atctttattt tcattacatc tgtgtgttgg 5160
ttttttgtgt gaatcgatag tactaacata cgctctccat caaaacaaaa cgaaacaaaa 5220
caaactagca aaataggctg tccccagtgc aagtgcaggt gccagaacat ttctctatcg 5280
ata 5283

<210> 59
<211> 5188
<212> DNA
<213> Artificial Sequence

<220>
<223> Description of Artificial Sequence:
pGHRH1-44YWTCMV construct

<400> 59
gctgtgcctt ctagttgcca gccatctgtt gtttgcccct cccccgtgcc ttccttgacc 60
ctggaaggtg ccactcccac tgtcctttcc taataaaatg aggaaattgc atcgcattgt 120
ctgagtaggt gtcattctat tctggggggt ggggtggggc aggacagcaa gggggaggat 180
tgggaagaca atagcaggca tgctggggat gcggtgggct ctatgggtac ccaggtgctg 240
aagaattgac ccggttcctc ctgggccaga aagaagcagg cacatcccct tctctgtgac 300
acaccctgtc cacgcccctg gttcttagtt ccagccccac tcataggaca ctcatagctc 360
aggagggctc cgccttcaat cccacccgct aaagtacttg gagcggtctc tccctccctc 420
atcagcccac caaaccaaac ctagcctcca agagtgggaa gaaattaaag caagataggc 480
tattaagtgc agagggagag aaaatgcctc caacatgtga ggaagtaatg agagaaatca 540
tagaatttct tccgcttcct cgctcactga ctcgctgcgc tcggtcgttc ggctgcggcg 600
agcggtatca gctcactcaa aggcggtaat acggttatcc acagaatcag gggataacgc 660
aggaaagaac atgtgagcaa aaggccagca aaaggccagg aaccgtaaaa aggccgcgtt 720

gctggcgttt ttccataggc tccgcccccc tgacgagcat cacaaaaatc gacgctcaag 780
tcagaggtgg cgaaacccga caggactata aagataccag gcgtttcccc ctggaagctc 840
cctcgtgcgc tctcctgttc cgaccctgcc gcttaccgga tacctgtccg cctttctccc 900
ttcgggaagc gtggcgcttt ctcatagctc acgctgtagg tatctcagtt cggtgtaggt 960
cgttcgctcc aagctgggct gtgtgcacga accccccgtt cagcccgacc gctgcgcctt 1020
atccggtaac tatcgtcttg agtccaaccc ggtaagacac gacttatcgc cactggcagc 1080
agccactggt aacaggatta gcagagcgag gtatgtaggc ggtgctacag agttcttgaa 1140
gtggtggcct aactacggct acactagaag aacagtattt ggtatctgcg ctctgctgaa 1200
gccagttacc ttcggaaaaa gagttggtag ctcttgatcc ggcaaacaaa ccaccgctgg 1260
tagcggtggt ttttttgttt gcaagcagca gattacgcgc agaaaaaaag gatctcaaga 1320
agatcctttg atcttttcta cggggtctga cgctcagtgg aacgaaaact cacgttaagg 1380
gattttggtc atgagattat caaaaaggat cttcacctag atccttttaa attaaaaatg 1440
aagttttaaa tcaatctaaa gtatatatga gtaaacttgg tctgacagtt accaatgctt 1500
aatcagtgag gcacctatct cagcgatctg tctatttcgt tcatccatag ttgcctgact 1560
cggggggggg gggcgctgag gtctgcctcg tgaagaaggt gttgctgact cataccaggc 1620
ctgaatcgcc ccatcatcca gccagaaagt gagggagcca cggttgatga gagctttgtt 1680
gtaggtggac cagttggtga ttttgaactt ttgctttgcc acggaacggt ctgcgttgtc 1740
gggaagatgc gtgatctgat ccttcaactc agcaaaagtt cgatttattc aacaaagccg 1800
ccgtcccgtc aagtcagcgt aatgctctgc cagtgttaca accaattaac caattctgat 1860
tagaaaaact catcgagcat caaatgaaac tgcaatttat tcatatcagg attatcaata 1920
ccatattttt gaaaaagccg tttctgtaat gaaggagaaa actcaccgag gcagttccat 1980
aggatggcaa gatcctggta tcggtctgcg attccgactc gtccaacatc aatacaacct 2040
attaatttcc cctcgtcaaa aataaggtta tcaagtgaga aatcaccatg agtgacgact 2100
gaatccggtg agaatggcaa aagcttatgc atttctttcc agacttgttc aacaggccag 2160
ccattacgct cgtcatcaaa atcactcgca tcaaccaaac cgttattcat tcgtgattgc 2220
gcctgagcga gacgaaatac gcgatcgctg ttaaaaggac aattacaaac aggaatcgaa 2280
tgcaaccggc gcaggaacac tgccagcgca tcaacaatat tttcacctga atcaggatat 2340
tcttctaata cctggaatgc tgttttcccg gggatcgcag tggtgagtaa ccatgcatca 2400
tcaggagtac ggataaaatg cttgatggtc ggaagaggca taaattccgt cagccagttt 2460
agtctgacca tctcatctgt aacatcattg gcaacgctac ctttgccatg tttcagaaac 2520
aactctggcg catcgggctt cccatacaat cgatagattg tcgcacctga ttgcccgaca 2580
ttatcgcgag cccatttata cccatataaa tcagcatcca tgttggaatt taatcgcggc 2640
ctcgagcaag acgtttcccg ttgaatatgg ctcataacac cccttgtatt actgtttatg 2700
taagcagaca gttttattgt tcatgatgat atatttttat cttgtgcaat gtaacatcag 2760
agattttgag acacaacgtg gctttccccc cccccccatt attgaagcat ttatcagggt 2820
tattgtctca tgagcggata catatttgaa tgtatttaga aaaataaaca aataggggtt 2880
ccgcgcacat ttccccgaaa agtgccacct gacgtctaag aaaccattat tatcatgaca 2940
ttaacctata aaaataggcg tatcacgagg ccctttcgtc ctcgcgcgtt tcggtgatga 3000
cggtgaaaac ctctgacaca tgcagctccc ggagacggtc acagcttgtc tgtaagcgga 3060
tgccgggagc agacaagccc gtcagggcgc gtcagcgggt gttggcgggt gtcggggctg 3120
gcttaactat gcggcatcag agcagattgt actgagagtg caccatatgc ggtgtgaaat 3180
accgcacaga tgcgtaagga gaaaataccg catcagattg gctattggcc attgcatacg 3240
ttgtatccat atcataatat gtacatttat attggctcat gtccaacatt accgccatgt 3300
tgacattgat tattgactag ttattaatag taatcaatta cggggtcatt agttcatagc 3360
ccatatatgg agttccgcgt tacataactt acggtaaatg gcccgcctgg ctgaccgccc 3420
aacgaccccc gcccattgac gtcaataatg acgtatgttc ccatagtaac gccaataggg 3480
actttccatt gacgtcaatg ggtggagtat ttacggtaaa ctgcccactt ggcagtacat 3540
caagtgtatc atatgccaag tacgccccct attgacgtca atgacggtaa atggcccgcc 3600

tggcattatg cccagtacat gaccttatgg gactttccta cttggcagta catctacgta 3660
ttagtcatcg ctattaccat ggtgatgcgg ttttggcagt acatcaatgg gcgtggatag 3720
cggtttgact cacggggatt tccaagtctc caccccattg acgtcaatgg gagtttgttt 3780
tggcaccaaa atcaacggga ctttccaaaa tgtcgtaaca actccgcccc attgacgcaa 3840
atgggcggta ggcgtgtacg gtgggaggtc tatataagca gagctcgttt agtgaaccgt 3900
cagatcgcct ggagacgcca tccacgctgt tttgacctcc atagaagaca ccgggaccga 3960
tccagcctcc gcggccggga acggtgcatt ggaacgcgga ttccccgtgc caagagtgac 4020
gtaagtaccg cctatagact ctataggcac acccctttgg ctcttatgca tgctatactg 4080
tttttggctt ggggcctata cacccccgct tccttatgct ataggtgatg gtatagctta 4140
gcctataggt gtgggttatt gaccattatt gaccactccc ctattggtga cgatactttc 4200
cattactaat ccataacatg gctctttgcc acaactatct ctattggcta tatgccaata 4260
ctctgtcctt cagagactga cacggactct gtatttttac aggatggggt cccatttatt 4320
atttacaaat tcacatatac aacaacgccg tcccccgtgc ccgcagtttt tattaaacat 4380
agcgtgggat ctccacgcga atctcgggta cgtgttccgg acatgggctc ttctccggta 4440
gcggcggagc ttccacatcc gagccctggt cccatgcctc cagcggctca tggtcgctcg 4500
gcagctcctt gctcctaaca gtggaggcca gacttaggca cagcacaatg cccaccacca 4560
ccagtgtgcc gcacaaggcc gtggcggtag ggtatgtgtc tgaaaatgag cgtggagatt 4620
gggctcgcac ggctgacgca gatggaagac ttaaggcagc ggcagaagaa gatgcaggca 4680
gctgagttgt tgtattctga taagagtcag aggtaactcc cgttgcggtg ctgttaacgg 4740
tggagggcag tgtagtctga gcagtactcg ttgctgccgc gcgcgccacc agacataata 4800
gctgacagac taacagactg ttcctttcca tgggtctttt ctgcagtcac cgtcgtcgac 4860
acgtgtgatc agatatcgcg gccgctctag accaggcgcc tggatccgcc accatgccac 4920
tctgggtgtt cttctttgtg atcctcaccc tcagcaacag ctcccactgc tccccacctc 4980
cccctttgac cctcaggatg cggcggtatt atgcagatgc catcttcacc aacagctacc 5040
ggaaggtgct gggccagctg tccgcccgca agctgctcca ggacatcatg agcaggcagc 5100
agggagagag aaaccaagag caaggagcaa gggtgcggct ttgaagatct tagtagtagt 5160
aggcggccgc tctagaggat ccagatct 5188

<210> 60
<211> 5254
<212> DNA
<213> Artificial Sequence

<220>
<223> Description of Artificial Sequence:
      pGHRH1-44WTGHpep construct

<400> 60
gctgtgcctt ctagttgcca gccatctgtt gtttgcccct cccccgtgcc ttccttgacc 60
ctggaaggtg ccactcccac tgtcctttcc taataaaatg aggaaattgc atcgcattgt 120
ctgagtaggt gtcattctat tctggggggt ggggtggggc aggacagcaa gggggaggat 180
tgggaagaca atagcaggca tgctggggat gcggtgggct ctatgggtac ccaggtgctg 240
aagaattgac ccggttcctc ctgggccaga aagaagcagg cacatcccct tctctgtgac 300
acaccctgtc cacgcccctg gttcttagtt ccagccccac tcataggaca ctcatagctc 360
aggagggctc cgccttcaat cccacccgct aaagtacttg gagcggtctc tccctccctc 420
atcagcccac caaaccaaac ctagcctcca agagtgggaa gaaattaaag caagataggc 480
tattaagtgc agagggagag aaaatgcctc caacatgtga ggaagtaatg agagaaatca 540

tagaatttct tccgcttcct cgctcactga ctcgctgcgc tcggtcgttc ggctgcggcg 600
agcggtatca gctcactcaa aggcggtaat acggttatcc acagaatcag gggataacgc 660
aggaaagaac atgtgagcaa aaggccagca aaaggccagg aaccgtaaaa aggccgcgtt 720
gctggcgttt ttccataggc tccgcccccc tgacgagcat cacaaaaatc gacgctcaag 780
tcagaggtgg cgaaacccga caggactata aagataccag gcgtttcccc ctggaagctc 840
cctcgtgcgc tctcctgttc cgaccctgcc gcttaccgga tacctgtccg cctttctccc 900
ttcgggaagc gtggcgcttt ctcatagctc acgctgtagg tatctcagtt cggtgtaggt 960
cgttcgctcc aagctgggct gtgtgcacga accccccgtt cagcccgacc gctgcgcctt 1020
atccggtaac tatcgtcttg agtccaaccc ggtaagacac gacttatcgc cactggcagc 1080
agccactggt aacaggatta gcagagcgag gtatgtaggc ggtgctacag agttcttgaa 1140
gtggtggcct aactacggct acactagaag aacagtattt ggtatctgcg ctctgctgaa 1200
gccagttacc ttcggaaaaa gagttggtag ctcttgatcc ggcaaacaaa ccaccgctgg 1260
tagcggtggt ttttttgttt gcaagcagca gattacgcgc agaaaaaaag gatctcaaga 1320
agatcctttg atcttttcta cggggtctga cgctcagtgg aacgaaaact cacgttaagg 1380
gattttggtc atgagattat caaaaaggat cttcacctag atccttttaa attaaaaatg 1440
aagttttaaa tcaatctaaa gtatatatga gtaaacttgg tctgacagtt accaatgctt 1500
aatcagtgag gcacctatct cagcgatctg tctatttcgt tcatccatag ttgcctgact 1560
cggggggggg gggcgctgag gtctgcctcg tgaagaaggt gttgctgact cataccaggc 1620
ctgaatcgcc ccatcatcca gccagaaagt gagggagcca cggttgatga gagctttgtt 1680
gtaggtggac cagttggtga ttttgaactt ttgctttgcc acggaacggt ctgcgttgtc 1740
gggaagatgc gtgatctgat ccttcaactc agcaaaagtt cgatttattc aacaaagccg 1800
ccgtcccgtc aagtcagcgt aatgctctgc cagtgttaca accaattaac caattctgat 1860
tagaaaaact catcgagcat caaatgaaac tgcaatttat tcatatcagg attatcaata 1920
ccatattttt gaaaaagccg tttctgtaat gaaggagaaa actcaccgag gcagttccat 1980
aggatggcaa gatcctggta tcggtctgcg attccgactc gtccaacatc aatacaacct 2040
attaatttcc cctcgtcaaa aataaggtta tcaagtgaga aatcaccatg agtgacgact 2100
gaatccggtg agaatggcaa aagcttatgc atttctttcc agacttgttc aacaggccag 2160
ccattacgct cgtcatcaaa atcactcgca tcaaccaaac cgttattcat tcgtgattgc 2220
gcctgagcga gacgaaatac gcgatcgctg ttaaaaggac aattacaaac aggaatcgaa 2280
tgcaaccggc gcaggaacac tgccagcgca tcaacaatat tttcacctga atcaggatat 2340
tcttctaata cctggaatgc tgttttcccg gggatcgcag tggtgagtaa ccatgcatca 2400
tcaggagtac ggataaaatg cttgatggtc ggaagaggca taaattccgt cagccagttt 2460
agtctgacca tctcatctgt aacatcattg gcaacgctac ctttgccatg tttcagaaac 2520
aactctggcg catcgggctt cccatacaat cgatagattg tcgcacctga ttgcccgaca 2580
ttatcgcgag cccatttata cccatataaa tcagcatcca tgttggaatt taatcgcggc 2640
ctcgagcaag acgtttcccg ttgaatatgg ctcataacac cccttgtatt actgtttatg 2700
taagcagaca gttttattgt tcatgatgat atatttttat cttgtgcaat gtaacatcag 2760
agattttgag acacaacgtg gctttccccc cccccccatt attgaagcat ttatcagggt 2820
tattgtctca tgagcggata catatttgaa tgtatttaga aaaataaaca aataggggtt 2880
ccgcgcacat ttccccgaaa agtgccacct gacgtctaag aaaccattat tatcatgaca 2940
ttaacctata aaaataggcg tatcacgagg ccctttcgtc ctcgcgcgtt tcggtgatga 3000
cggtgaaaac ctctgacaca tgcagctccc ggagacggtc acagcttgtc tgtaagcgga 3060
tgccgggagc agacaagccc gtcagggcgc gtcagcgggt gttggcgggt gtcggggctg 3120
gcttaactat gcggcatcag agcagattgt actgagagtg caccatatgc ggtgtgaaat 3180
accgcacaga tgcgtaagga gaaaataccg catcagattg gctattggcc attgcatacg 3240
ttgtatccat atcataatat gtacatttat attggctcat gtccaacatt accgccatgt 3300
tgacattgat tattgactag ttattaatag taatcaatta cggggtcatt agttcatagc 3360
ccatatatgg agttccgcgt tacataactt acggtaaatg gcccgcctgg ctgaccgccc 3420

aacgaccccc gcccattgac gtcaataatg acgtatgttc ccatagtaac gccaataggg 3480
actttccatt gacgtcaatg ggtggagtat ttacggtaaa ctgcccactt ggcagtacat 3540
caagtgtatc atatgccaag tacgccccct attgacgtca atgacggtaa atggcccgcc 3600
tggcattatg cccagtacat gaccttatgg gactttccta cttggcagta catctacgta 3660
ttagtcatcg ctattaccat ggtgatgcgg ttttggcagt acatcaatgg gcgtggatag 3720
cggtttgact cacggggatt tccaagtctc caccccattg acgtcaatgg gagtttgttt 3780
tggcaccaaa atcaacggga ctttccaaaa tgtcgtaaca actccgcccc attgacgcaa 3840
atgggcggta ggcgtgtacg gtgggaggtc tatataagca gagctcgttt agtgaaccgt 3900
cagatcgcct ggagacgcca tccacgctgt tttgacctcc atagaagaca ccgggaccga 3960
tccagcctcc gcggccggga acggtgcatt ggaacgcgga ttccccgtgc caagagtgac 4020
gtaagtaccg cctatagact ctataggcac acccctttgg ctcttatgca tgctatactg 4080
tttttggctt ggggcctata cacccccgct tccttatgct ataggtgatg gtatagctta 4140
gcctataggt gtgggttatt gaccattatt gaccactccc ctattggtga cgatactttc 4200
cattactaat ccataacatg gctctttgcc acaactatct ctattggcta tatgccaata 4260
ctctgtcctt cagagactga cacggactct gtatttttac aggatggggt cccatttatt 4320
atttacaaat tcacatatac aacaacgccg tcccccgtgc ccgcagtttt tattaaacat 4380
agcgtgggat ctccacgcga atctcgggta cgtgttccgg acatgggctc ttctccggta 4440
gcggcggagc ttccacatcc gagccctggt cccatgcctc cagcggctca tggtcgctcg 4500
gcagctcctt gctcctaaca gtggaggcca gacttaggca cagcacaatg cccaccacca 4560
ccagtgtgcc gcacaaggcc gtggcggtag ggtatgtgtc tgaaaatgag cgtggagatt 4620
gggctcgcac ggctgacgca gatggaagac ttaaggcagc ggcagaagaa gatgcaggca 4680
gctgagttgt tgtattctga taagagtcag aggtaactcc cgttgcggtg ctgttaacgg 4740
tggagggcag tgtagtctga gcagtactcg ttgctgccgc gcgcgccacc agacataata 4800
gctgacagac taacagactg ttcctttcca tgggtctttt ctgcagtcac cgtcgtcgac 4860
acgtgtgatc agatatcgcg gccgctctag accaggcgcc tggatccgcc accatgccac 4920
tctgggtgtt cttctttgtg atcctcaccc tcagcaacag ctcccactgc tccccacctc 4980
cccctttgac cctcaggatg cggcggtatt atgcagatgc catcttcacc aacagctacc 5040
ggaaggtgct gggccagctg tccgcccgca agctgctcca ggacatcatg agcaggcagc 5100
agggagagag aaaccaagag caaggagcaa gggtgcggct tgggcggaaa gtagaaacgt 5160
ttctgcgtat tgtacagtgt cgtagcgtag aagggagctg tgggttttga agatcttagt 5220
agtagtaggc ggccgctcta gaggatccag atct 5254

<210> 61
<211> 39
<212> DNA
<213> Artificial Sequence

<220>
<223> Description of Artificial Sequence: Primer

<400> 61
agatctgcca ccatgccact ctgggtgttc ttctttgtg 39

<210> 62
<211> 36
<212> DNA

<213> Artificial Sequence

<220>
<223> Description of Artificial Sequence: Primer

<400> 62
ggatccaagc cgcacccttg ctccttgctc ttggtt 36

<210> 63
<211> 492
<212> DNA
<213> Artificial Sequence

<220>
<223> Description of Artificial Sequence: Primer

<400> 63
bbttttttst ggatccaagg ccgagacgta cctgcgggtc atgaagtgtc gccgcttcgt 60
ggaaagcagc tgtgccttcc cnrmamhada taragrahnt anttacrgad vsnasysdat 120
aatntndtgt msnwrmansy mbaracrrnw hrbrbybgga tccaaggccg agacgtacct 180
gcgggtcatg aagtgtcgcc gcttcgtgga aagcagcrga dvsnrgadvs nhhcggatcc 240
aaggccgaga cgtacctgcg ggtcatgaag tgtcgccgct tcgtggaaag cagcgatrga 300
dvsnccgarg advsnccmcr stwrdssydh rnccggatcc aaggccgaga cgtacctgcg 360
ggtcatgaag tgtcgccgct tcgtggaaag cagcttdgda nbcdcdcrtn trybsstabw 420
rddcmntsmm arynrmatnd cmntsmmary nrmatncmbb ctssmcrstw rddcmntmsw 480
rddcwrddcm nt 492

<210> 64
<211> 69
<212> DNA
<213> Artificial Sequence

<220>
<223> Description of Artificial Sequence: Primer

<400> 64
ggatccgaag gcacagctgc tttccacgaa gcggcgacac ttcatgaccc gcaggtacgt 60
ctcggcctt 69

<210> 65
<211> 102
<212> DNA
<213> Artificial Sequence

<220>

<223> Description of Artificial Sequence:  Primer

<400> 65
agatcttcaa agccgcaccc ttgctccttg ctcttggttt ctctctccct gctgcctgct 60
catgatgtcc tggagcagct tgcgggcgga cagctggccc ag 102

<210> 66
<211> 21
<212> DNA
<213> Artificial Sequence

<220>
<223> Description of Artificial Sequence:  Primer

<400> 66
ccgcggcatc ctgagggtca a 21

<210> 67
<211> 21
<212> DNA
<213> Artificial Sequence

<220>
<223> Description of Artificial Sequence:  Primer

<400> 67
tatgcagatg ccatcttcaa c 21

## Claims

1. A method for the treating growth hormone related disorders characterized by growth hormone deficiencies in an animal, comprising supplying the animal with a polynucleotide sequence that encodes growth hormone releasing hormone or modified growth hormone releasing hormone.

2. A method for improving the growth and performance of an animal, comprising supplying the animal with a polynucleotide sequence that encodes growth hormone releasing hormone or modified growth hormone releasing hormone.

3. The method of Claim 1 or 2 wherein a polynucleotide sequence encoding growth hormone releasing horomone or modified growth hormone releasing hormone is contained in pharmaceutically acceptable carrier and is administered to an animal.

4. The method of Claim 3 in which the carrier is a DNA vector, a viral vector, a liposome or lipofectin.

5. The method of Claim 4 in which the DNA vector is an expression vector.

6. The expression vector of Claim 5 containing a polynucleotide sequence of growth hormone releasing hormone or modified growth hormone releasing hormone in operative association with a nucleotide regulatory sequence that controls expression of the polynucleotide.

7. The expression vector of Claim 6, wherein said regulatory element is selected from the group consisting of the cytomegalovirus hCMV immediate early gene, the early or late promoters of SV40 adenovirus, and the swine alpha-skeletal actin promoter.

8. The method of Claim 2 in which the animal is a cat, dog, cow, pig, horse or chicken.

9. A method for the treating growth hormone related disorders characterized by growth hormone deficiencies in an animal, comprising supplying the animal with a polynucleotide sequence that encodes growth hormone or modified growth hormone.

10. A method for improving the growth and performance of an animal, comprising supplying the animal with a polynucleotide sequence that encodes growth hormone or modified growth hormone.

11. The method of Claim 9 or 10 wherein a polynucleotide sequence encoding growth hormone or modified growth hormone is contained in pharmaceutically acceptable carrier and is administered to an animal.

12. The method of Claim 11 in which the carrier is a DNA vector, a viral vector, a liposome or lipofectin.

13. The method of Claim 12 in which the DNA vector is an expression vector.

14. The expression vector of Claim 13 containing a polynucleotide sequence of growth hormone or modified growth hormone in operative association with a nucleotide regulatory sequence that controls expression of the polynucleotide.

15. The expression vector of Claim 14, wherein said regulatory element is selected from the group consisting of the cytomegalovirus hCMV immediate early gene, the early or late promoters of SV40 adenovirus, and the swine alpha-skeletal actin promoter.

16. The method of Claim 15 in which the animal is a dog, cat, cow, pig, horse or chicken.

17. A growth hormone releasing hormone (GHRH) variant comprising the addition of one amino acid to the amino terminus of a 29 amino acid amino terminal fragment of GHRH, in a pharmaceutical formulation suitable for delivery to a human or livestock.

18. The growth hormone releasing hormone of Claim 17 wherein the amino acid is a hydrophobic residue or tyrosine.

19. A growth hormone releasing hormone variant comprising the addition of two or three amino acids to the amino terminus, of a 29 amino acid amino terminal framgnt of GHRH wherein the second amino acid is not proline or alanine in a pharmaceutical formulation suitable for delivery to a human or livestock.

20. A growth hormone releasing hormone variant of Claim 19 comprising the addition of more than three amino acids to the amino terminus of a 29 amino acid amino terminal fragment of GHRH, wherein the addition does not interfere with the functional activity of growth hormone releasing hormone.

21. The growth hormone releasing hormone variant of Claim 17, 19 or 20, further comprising a substitution of glycine with alanine at residue 15.

22. The growth hormone releasing hormone variant of Claim 17, 19 or 20, further comprising a substitution of leucine with alanine at residue 22.

23. The growth hormone releasing hormone variant of Claim 17, 19 or 20, further comprising substitutions of glycine with alanine at residue 15 and leucine with alanine at residue 22.

24. The growth hormone releasing hormone variant of Claim 17, 19, or 20, further comprising the addition of glycine and arginine at the carboxy-terminus.

25. The growth hormone releasing hormone variant of Claim 18, 19 or 20 in which the amino acids are naturally occurring.

26. A polynucleotide sequence encoding the growth hormone releasing hormone variant of Claim 17, 19 or 20.

27. A nucleotide vector containing the polynucleotide sequence of Claim 26.

28. An expression vector containing the polynucleotide sequence of Claim 26 in operative association with a nucleotide regulatory sequence that controls expression of the polynucleotide sequence in a host cell.

29. The expression vector of Claim 28, wherein said regulatory element is selected from the group consisting of the cytomegalovirus hCMV immediate early gene, the early or late promoters of SV40 adenovirus, and the swine alpha-skeletal actin promoter.

30. A genetically engineered host cell that contains the polynucleotide sequence of Claim 26.

31. A genetically engineered host cell that contains the polynucleotide sequence of Claim 26 in operative association with a nucleotide regulatory sequence that controls expression of the polynucleotide sequence in the host cell.

32. A method for the treating growth hormone related disorders characterized by growth hormone deficiencies in an animal, comprising supplying the animal with a polynucleotide sequence that encodes the growth hormone releasing hormone variant of Claim 17, 19 or 20.

33. A method for improving the growth and performance of an animal, comprising supplying the animal with a polynucleotide sequence that encodes the growth hormone releasing hormone variant of Claim 17, 19 or 20.

34. A purified polypeptide of the growth hormone releasing hormone variant of Claim 17, 19 or 20.

35. A method for the treating growth hormone related disorders characterized by growth hormone deficiencies in an animal, comprising supplying the animal with an effective amount of a polypeptide of Claim 34.

36. A method for improving the growth and performance of an animal, comprising supplying the animal with an effective amount of a polypeptide of Claim 34.

37. A pharmaceutical composition for promoting the expression and elevation of growth hormone in an animal, comprising administering to said animal an effective amount of the growth hormone releasing hormone variant of Claim 17, 19 or 20.

38. A pharmaceutical composition for the treatment of growth hormone related disorders characterized by growth hormone deficiencies in an animal, comprising administering to said animal an effective amount of the growth hormone releasing hormone variant of Claim 17, 19 or 20.

39. A pharmaceutical composition for the improvement of growth and performance of an animal, comprising administering to said animal an effective amount of a growth hormone releasing hormone variant of Claim 17, 19 or 20.
